# FASCICULE DE BREVET EUROPEEN

(11) **EP 2 521 727 B1**
(45) Date de publication et mention de la délivrance du brevet: **19.03.2014**
(21) Numéro de dépôt: 11704261.4
(22) Date de dépôt: 07.01.2011
(51) Int. Cl.: C07D 471/04, A61K 31/437, A61K 31/455, A61K 31/538, A61P 25/16, A61P 25/28, A61P 25/14

(54) **NOUVEAUX DÉRIVÉS PYRROLOPYRIDINE BENZOÏQUES ET LEUR UTILISATION POUR LE TRAITEMENT DE LA MALADIE DE PARKINSON**
NEUE BENZOSÄURE-PYRROLOPYRIDIN-DERIVATE UND DEREN VERWENDUND FÜR DIE BEHANDLUNG DER PARKINSON-KRANKHEIT
NEW BENZOIC PYRROLOPYRIDINE DERIVATIVES AND THEIR USE FOR THE TREATMENT OF PARKINSON'S DISEASE

(30) Priorité: 08.01.2010 FR 1050113
(43) Date de publication de la demande: 14.11.2012
(73) Titulaire: INVENTIVA, 21121 Daix (FR)
(72) Inventeur: AMAUDRUT, Jérôme, F-21000 Dijon (FR); BOUBIA, Benaissa, F-21850 Saint Apollinaire (FR); GUILLIER, Fabrice, F-21310 Belleneuve (FR); POUPARDIN-OLIVIER, Olivia, F-21490 Varois et Chaignot (FR)
(74) Mandataire: Nevant, Marc
(86) Numéro de dépôt international: PCT/FR2011/050023
(87) Numéro de publication internationale: WO 2011/083278

(56) Documents cités:
- EP-A1- 1 086 950
- WO-A1-2004/072050
- WO-A1-2007/026104
- ERIC DESARBRE ET AL: "Synthesis of 2-substituted-1H-pyrrolo[2,3-b]pyridines: preparation of 7-azaolivacine analogue and 7-azaindolopyridopyrimidine derivatives", TETRAHEDRON, DOI:10.1016/S0040-4020(97)00107-5, vol. 53, no. 10, 1997, pages 3637-3648, XP004105443,
- CELINE DUBOIS ET AL: "Identification of a potent agonist of the orphan nuclear receptor Nurr1", CHEMMEDCHEM, DOI:10.1002/CMDC.200600078, vol. 1, 2006, pages 955-958, XP007907069,

## Description

La présente invention concerne de nouveaux composés de type pyrrolopyridine, préférentiellement des dérivés de type pyrrolopyridine benzoïque, ainsi que leur procédé de préparation et leur utilisation en tant que principe actif de médicaments, notamment destinés au traitement et/ou à la prévention de maladies impliquant les récepteurs nucléaires NURR-1. Plus spécifiquement, cette invention concerne l'utilisation de ces composés pour la fabrication d'un médicament pour le traitement et/ou la prévention des maladies neurodégénératives et en particulier de la maladie de Parkinson.

### Art antérieur

Les maladies neurodégénératives sont définies comme des maladies caractérisées par un dysfonctionnement progressif du système nerveux. Elles sont souvent associées à une atrophie des structures du système nerveux central ou périphérique touché. Elles incluent, entre autres, des maladies telles que la maladie d'Alzheimer, la maladie de Creutzfeldt-Jakob, la maladie de Huntington, la maladie de Parkinson, les maladies lysosomales, la paralysie supranucléaire progressive, la sclérose en plaques et la sclérose latérale amyotrophique. Parmi ces maladies neurodégénératives, la maladie de Parkinson est une affection qui touche environ quatre millions de personnes dans le monde. Bien qu'elle affecte des individus de tout âge, elle est plus commune chez les personnes âgées (avec 2 % de la population des personnes de plus de 65 ans touchées par cette maladie). Elle est caractérisée par une dégénérescence des neurones dopaminergiques de la substantia nigra. Ces types de neurones synthétisent la dopamine et l'utilisent comme neurotransmetteurs.

On a pu établir une relation entre le déficit de la dopamine et les troubles nerveux. La dopamine exerce un rôle central dans le contrôle des mouvements volontaires, les fonctions cognitives et le développement de comportements associés aux émotions.

La stratégie thérapeutique actuelle pour le traitement de la maladie de Parkinson repose sur l'atténuation des symptômes en suppléant la déficience en dopamine par l'administration d'un précurseur métabolique tel que la L-DOPA.

Or, aujourd'hui, l'augmentation de la fréquence de cette pathologie a rendu nécessaire le développement de nouveaux agents thérapeutiques, exerçant un rôle bénéfique dans la survie et la différenciation neuronale.

Ce développement a conduit à identifier des composés capables d'activer les récepteurs nucléaires impliqués dans la pathogénèse de la maladie de Parkinson.

Fortement exprimé dans le cerveau, le facteur de transcription NURR-1, membre de la superfamille des récepteurs nucléaires orphelins, a été identifié comme ayant un rôle essentiel dans le développement et le maintien des neurones dopaminergiques du mésencéphale (Zetterstrom et al. 1997, Science 276(5310):248-50).

Le récepteur nucléaire NURR-1 intervient dans le maintien du phénotype dopaminergique via la régulation des gènes spécifiques des neurones dopaminergiques (DA). Il favorise aussi la survie des neurones DA en les protégeant des agressions toxiques. Le récepteur nucléaire NURR-1 sert donc de facteur de transcription spécifique des neurones dopaminergiques pour lequel les activités pourraient être régulées en modulant la neurotransmission dopaminergique dans la maladie de Parkinson.

Ce récepteur se lie à l'ADN sous forme de monomères, d'homodimères ou d'hétérodimères avec RXR (Retinoid X Receptor) un récepteur nucléaire qui est l'hétéropartenaire de nombreux autres membres de la famille des récepteurs nucléaires. RXR intervient dans de nombreux processus physiologiques comme le métabolisme des lipides et du glucose, le développement et la différenciation. NURR-1 interagit ainsi avec les isoformes α et γ de RXR. RXRα est exprimé de façon ubiquitaire alors que l'expression de RXRγ se concentre principalement dans le cerveau et notamment dans le striatum, l'hypothalamus et l'hypophyse.

Les complexes formés NURR-1/RXRα et NURR-1/RXRγ sont capables de réguler la transcription en réponse à un ligand de RXR. RXR module donc positivement le potentiel d'activation de la transcription de NURR-1.

L'identification de composés capables d'induire l'activité des complexes NURR-1/RXRα et NURR-1/RXRγ devrait en conséquence permettre de disposer de nouvelles voies pour traiter la maladie de Parkinson.

On connaît par le document WO2003/015780 des composés hétérocycliques actifs pour le traitement de la maladie de Parkinson. Des composés hétérocycliques ayant une affinité pour les récepteurs PPAR sont décrits dans le document WO2005/009958.

Par ailleurs, les documents FR 2 903 105, FR 2 903 106 et FR 2 903 107 décrivent des composés activateurs du récepteur NURR-1, tandis que l'utilisation de composés hétérocycliques modulateurs de l'activité des récepteurs de la famille des NGFI-B (dont NURR-1 est un membre) est décrite dans le document WO2005/047268.

Des composés hétérocycliques présentés comme des inhibiteurs du facteur Xa sont décrits dans le document WO 98/25611. D'autres composés hétérocycliques présentés comme des modulateurs du récepteur 5-HT6 sont décrits dans les documents WO2008/101 et WO2010/002802.

Les dérivés de 1H-pyrrolo[2,3-b]pyridine suivants sont décrits dans le document : Tetrahedron, Vol. 53, N.10, pp. 3637-3648, 1997:
- l'acide 2-[[1-(phénylsulfonyl)-1H-pyrrolo[2,3-b]pyridin-2-yl]carbonyl]-benzoïque ;
- le N,N-diéthyl-4-[hydroxy-[1-(phénylsulfonyl)-1H-pyrrolo[2,3-b]pyridin-2-yl]méthyl]-2-méthoxy-3-pyridinecarboxamide ;
- le N,N-diéthyl-2-méthoxy-4-[[1-(phénylsulfonyl)-1H-pyrrolo[2,3-b]pyridin-2-yl]carbonyl]-3-pyridinecarboxamide ;
- le N,N-diéthyl-4-[1-hydroxy-1-[1-(phénylsulfonyl)-1H-pyrrolo[2,3-b]pyridin-2-yl]éthyl]-2-méthoxy-3-pyridinecarboxamide.
Ces composés sont des intermédiaires de synthèse de composés polyazatétracycliques.

### Objet de l'invention

Selon un premier aspect, la présente invention concerne de nouveaux composés de type pyrrolopyridine qui sont des agonistes NURR-1/RXRα et NURR-1/RXRγ, capables d'inhiber la dégénérescence des neurones observée dans la maladie de Parkinson, choisis parmi :
(i) les composés de formule (I) : 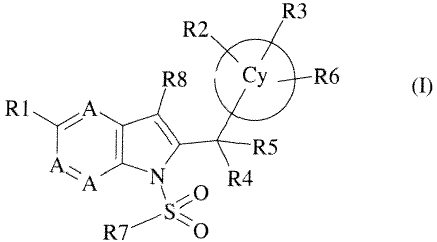 dans laquelle :
   l'un des groupes A représente un atome d'azote et les autres groupes A représentent un atome de carbone ;
   Cy représente un phényle ou un noyau hétéroaromatique à 5 ou 6 chaînons ;
   R1 représente un atome d'hydrogène, un atome d'halogène, un groupe (C₁-C₄)alkyle éventuellement totalement ou partiellement halogéné, ou un groupe (C₁-C₄)alcoxy ;
   R2 et R3 représentent chacun, indépendamment l'un de l'autre, un atome d'hydrogène, un atome d'halogène, un groupe (C₁-C₄)alkyle, un groupe hydroxy, ou un groupe (C₁-C₄)alcoxy ;
   R4 et R5 représentent chacun, indépendamment l'un de l'autre, un atome d'hydrogène, un atome d'halogène, un groupe (C₁-C₄)alkyle, ou un groupe hydroxy;
   ou R4 et R5 forment ensemble avec l'atome de carbone auquel ils sont rattachés un groupe éthylène (C=CH₂) ou un groupe carbonyle (C=O) ;
   R6 représente un groupe -COOR9 ou un groupe bioisostère d'acide carboxylique, de préférence un groupe -COOR9;
   R7 représente un phényle éventuellement substitué par un groupe (C₁-C₄)alkyle, ou un noyau hétéroaromatique ayant de 6 à 10 chaînons éventuellement substitué par un groupe (C₁-C₄)alkyle ;
   R8 représente un atome d'hydrogène, un groupe (C₁-C₄)alkyle ou un atome d'halogène ;
   R9 représente un atome d'hydrogène ou un groupe (C₁-C₄)alkyle ; et
(ii) les sels pharmaceutiquement acceptables desdits composés de formule (I).

Selon un deuxième aspect, l'invention concerne les composés précités pour leur utilisation en tant que substances thérapeutiquement actives, notamment dans le traitement et/ou la prévention des maladies neurodégénératives, en particulier la maladie de Parkinson, ainsi que les compositions pharmaceutiques les contenant.

Selon un troisième aspect, l'invention concerne l'utilisation d'au moins un composé de formule (I) ou l'un de ses sels pharmaceutiquement acceptable en tant que principe actif pour la préparation d'un médicament destiné au traitement des maladies dans lesquelles le récepteur NURR-1 est impliqué, notamment des neuro-dégénérescences, comme en particulier la maladie de Parkinson.

Selon un quatrième aspect, l'invention concerne une méthode de prévention et/ou de traitement des maladies dans lesquelles le récepteur NURR-1 est impliqué, notamment les maladies neurodégénératives, et plus particulièrement la maladie de Parkinson, qui consiste à administrer à un patient en ayant besoin une quantité thérapeutiquement efficace d'un composé de formule (I) ou d'un sel pharmaceutiquement acceptable dudit composé, ou bien d'une composition pharmaceutique contenant un tel composé.

### Description détaillée

On entend par "groupe alkyle", une chaîne hydrocarbonée saturée qui peut être linéaire ou ramifiée. Par exemple et sans limitation, un groupe alkyle ayant de 1 à 6 atomes de carbone peut être un groupe méthyle, éthyle, propyle, butyle, pentyle, hexyle, 1-méthyléthyle, 1-méthylpropyle, 2-méthylpropyle, 1,1-diméthyléthyle, 1-méthylbutyle, 1,1-diméthylpropyle, 1-méthylpentyle, ou 1,1-diméthylbutyle.

On entend par "halogène", un atome de brome, de fluor ou de chlore.

On entend par "groupe alkyle partiellement ou totalement halogéné" un groupe alkyle tel que défini ci-dessus dans lequel un ou plusieurs atomes d'hydrogène est (sont) remplacé(s) par un (des) atome(s) d'halogène. A titre d'exemple, on peut citer les groupes difluorométhyle ou trifluorométhyle.

On entend par "groupe alcoxy" un groupe OR dans lequel R est un groupe alkyle tel que défini ci-dessus. A titre d'exemple de groupe alcoxy ayant de 1 à 4 atomes de carbone, on peut citer les groupes méthoxy, éthoxy, propoxy, butoxy, 1-méthyléthoxy, 1,1-diméthyléthoxy, 1-méthylpropoxy, ou 2-méthylpropoxy.

On entend par "noyau hétéroaromatique à 5 ou 6 chaînons" un monocycle aromatique comprenant de 1 à 3 hétéroatomes, de préférence 1 ou 2 hétéroatomes, choisis parmi l'azote, l'oxygène et le soufre. A titre d'exemple, on peut citer les groupes pyrrolyle, pyrazolyle, imidazolyle, oxazolyle, isoxazolyle, triazolyle, oxadiazolyle, furanyle, thiényle thiazolyle, isothiazolyle, thiadiazolyle, pyridyle, pyridazinyle, pyrimidinyle, pyrazinyle ou triazinyle.

On entend par "noyau hétéroaromatique ayant de 6 à 10 chaînons" un groupe monocyclique ou bicyclique insaturé ou partiellement insaturé comprenant de 1 à 4 hétéroatomes, de préférence de 1 à 3 hétéroatomes, et de préférence encore 1 ou 2 hétéroatomes, choisis parmi l'azote, l'oxygène et le soufre, ledit groupe étant éventuellement substitué par un (C₁-C₄)alkyle. A titre d'exemple, on peut citer les groupes pyridyle, pyridazinyle, pyrimidinyle, pyrazinyle, triazinyle, 1,2,3,4-isoquinolinyle, quinolinyle, 1,2,3,4-tétrahydroquinolinyle, benzimidazolyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle ou benzoxazolyle.

On entend par "groupe bioisostère d'acide carboxylique", un groupe présentant des similitudes chimiques et physiques et produisant des propriétés biologiques largement semblables à un groupe carboxylique comme décrit dans Lipinski, Annual Reports in Medicinal Chemistry, 1986,21, p. 283 "Bioisosterism In Drug Design"; Graham, Theochem, 1995,343, p.105-109 "Theoretical Studies Applied To Drug Design: ab initio Electronic Distributions In Bioisosteres".

A titre d'exemple de groupe bioisostère d'acide carboxylique, on peut citer les groupes acyl-hydrazines optionnellement substitués, acyl-hydrazine carboxylates optionnellement substitués, alkyl et aryl sulfonylcarbamoyles optionnellement substitués, carboxamides, sulfonamides optionnellement substitués, oxadiazolones, phosphonates optionnellement substitués, isothiazoles optionnellement substitués, isoxazoles optionnellement substitués, isoxazolones optionnellement substitués, tétrazoles, thiazolidine-diones optionnellement substitués, thioxothiazolidinones optionnellement substitués. Le groupe bioisostère d'acide carboxylique est choisi parmi les groupes -SO₂NHR₁₀, -CONHNHCOOR11, -CONR12R13 ou -CONHSO₂R14, où R10, R11, R12, R13 et R14 représentent chacun indépendamment l'un de l'autre un atome d'hydrogène ou un groupe (C₁-C₄)alkyle, et de préférence parmi les groupes -CONR12R13 ou -CONHSO₂R14. Le groupe bioisostère d'acide carboxylique peut également être choisi parmi les groupes 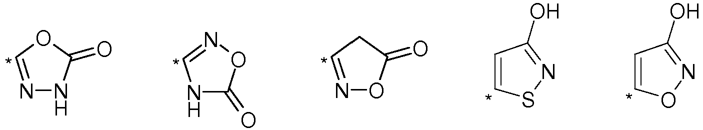 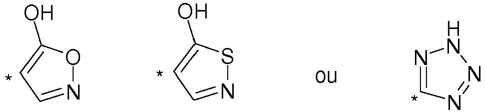 dans lesquels le symbole * désigne le point d'attachement au noyau Cy.

Les composés de formule (I) dans laquelle les substituants R4 et R5 sont différents présentent un centre asymétrique. Pour ces composés, l'invention couvre aussi bien le composé racémique que chacun des isomères optiques considérés séparément.

Les composés de formule (I) dans laquelle R6 représente un groupe COOH sont des acides carboxyliques qui peuvent être utilisés sous la forme d'acides libres ou sous la forme de sels, lesdits sels étant obtenus par combinaison de l'acide avec une base minérale ou organique non toxique, de préférence pharmaceutiquement acceptable. Parmi les bases minérales, on peut utiliser par exemple les hydroxydes de sodium, de potassium, de magnésium ou de calcium. Parmi les bases organiques, on peut utiliser par exemple les amines, les aminoalcools, des acides aminés basiques tels que la lysine ou l'arginine ou encore des composés porteurs d'une fonction ammonium quaternaire tels que par exemple la bétaïne ou la choline. Les sels des acides de formule (I) avec une base minérale ou organique peuvent être obtenus de façon classique, en utilisant les méthodes bien connues de l'homme de métier, par exemple en mélangeant des quantités stoechiométriques de l'acide de formule (I) dans laquelle R6 = COOH et de la base dans un solvant, tel que par exemple l'eau ou un mélange hydroalcoolique, et en lyophilisant ensuite la solution obtenue.

Selon un mode de réalisation de l'invention, cette dernière concerne les composés de formule (I) à l'exception des composés suivants :
- l'acide 2-[[1-(phénylsulfonyl)-1H-pyrrolo[2,3-b]pyridin-2-yl]carbonyl]-benzoïque ;
- le N,N-diéthyl-4-[hydroxy[1-(phénylsulfonyl)-1H-pyrrolo[2,3-b]pyridin-2-yl]méthyl]-2-méthoxy-3-pyridinecarboxamide ;
- le N,N-diéthyl-2-méthoxy-4-[[1-(phénylsulfonyl)-1H-pyrrolo[2,3-b]pyridin-2-yl]carbonyl]-3-pyridinecarboxamide ;
- le N,N-diéthyl-4-[1-hydroxy-1-[1-(phénylsulfonyl)-1H-pyrrolo[2,3-b]pyridin-2-yl]éthyl]-2-méthoxy-3-pyridinecarboxamide ;
- Une famille préférée de composés selon l'invention répond à la formule (I) dans laquelle R1 représente un atome d'halogène ou un groupe (C₁-C₄)alkyle éventuellement totalement ou partiellement halogéné et/ou R8 représente un atome d'hydrogène.

Une autre famille préférée de composés selon l'invention répond à la formule (I) dans laquelle :
Cy représente un phényle ou un noyau hétéroaromatique à 5 ou 6 chaînons ;
R1 représente un atome d'halogène ou un groupe (C₁-C₄)alkyle éventuellement totalement ou partiellement halogéné ;
R2 et R3 représentent chacun, indépendamment l'un de l'autre, un atome d'hydrogène ou un atome d'halogène ;
R4 et R5 représentent chacun, indépendamment l'un de l'autre, un atome d'hydrogène, un atome d'halogène ou un groupe hydroxy,
ou R4 et R5 forment ensemble avec l'atome de carbone auxquels ils sont rattachés un groupe carbonyle (C=O) ;
R6 représente un groupe -COOR9;
R7 représente un phényle éventuellement substitué par un groupe (C₁-C₄)alkyle ;
R8 représente un atome d'hydrogène ;
R9 représente un atome d'hydrogène ou un groupe (C₁-C₄)alkyle.

Parmi cette famille, on préfère les composés de formule (I) dans laquelle R4 et R5 représentent chacun, indépendamment l'un de l'autre, un atome d'hydrogène ou un groupe hydroxy.

Parmi les composés décrits ci-dessus, on préfère ceux qui répondent à au moins l'une des conditions suivantes :
Cy représente un phényle, thiényle, thiazolyle, furanyle ou pyridyle, de préférence un phényle ou un thiényle ;
R2 et R3 représentent chacun un atome d'hydrogène :
R4 et R5 représentent chacun un atome d'hydrogène.

A titre de composés tout particulièrement préférés, on peut citer :
l'acide 5-[[1-[[3-(1,1-diméthyléthyl)phényl]sulfonyl]-5-(trifluorométhyl)-1H-pyrrolo[3,2-b]pyridin-2-yl]méthyl]-thiophène-2-carboxylique,
l'acide 3-[[1-[[3-(1,1-diméthyléthyl)phényl]sulfonyl]-5-(trifluorométhyl)-1H-pyrrolo[2,3-c]pyridin-2-yl]méthyl]-2-benzoïque,
l'acide 2-chloro-4-[[1-[[3-(1,1-diméthyléthyl)phényl]sulfonyl]-5-(trifluorométhyl)-1H-pyrrolo[2,3-c]pyridin-2-yl]méthyl]-benzoïque,
l'acide 5-[[1-[[3-(1,1-diméthyléthyl)phényl]sulfonyl]-5-(trifluorométhyl)-1H-pyrrolo[2,3-c]pyridin-2-yl]méthyl]-furane-3-carboxylique,
l'acide 5-[[1-[[3-(1,1-diméthyléthyl)phényl]sulfonyl]-5-(trifluorométhyl)-1H-pyrrolo[2,3-c]pyridin-2-yl]méthyl]-pyridine-3-carboxylique,
l'acide 4-[[1-[[3-(1,1-diméthyléthyl)phényl]sulfonyl]-5-(tritluorométhyl)-1H-pyrrolo[2,3-c]pyridin-2-yl]méthyl]-thiophène-2-carboxylique,
l'acide 5-[[1-[[3-(1,1-diméthyléthyl)phényl]sulfonyl]-5-(trifluorométhyl)-1H-pyrrolo[2,3-c]pyridin-2-yl]méthyl]-2-fluoro-benzoïque,
l'acide 2-[[1-[[3-(1,1-diméthyléthyl)phényl]sulfonyl]-5-(trifluorométhyl)-1H-pyrrolo[3,2-b]pyridin-2-yl]méthyl]-thiazole-4-carboxylique,
le 4-[1-(4-(1-méthyléthyl)phénylsulfonyl)-5-trifluorométhyl-1H-pyrrolo[2,3-b]pyridin-2-ylméthyl]-benzoate de méthyle,
l'acide 4-[[1-[[3-(1-méthyléthyl)phényl]sulfonyl]-5-(trifluorométhyl)-1H-pyrrolo[2,3-b]pyridin-2-yl]méthyl]-benzoïque,
le 4-[[1-[(3,4-dihydro-4-méthyl-2H-1,4-benzoxazin-6-yl)sulfonyl]-5-(trifluorométhyl)-1H-pyrrolo[2,3-c]pyridin-2-yl]méthyl]-benzoate de méthyle,
l'acide 4-[[1-[(3,4-dihydro-4-méthyl-2H-1,4-benzoxazin-6-yl)sulfonyl]-5-(trifluorométhyl)-1H-pyrrolo[2,3-c]pyridin-2-yl]méthyl]-benzoïque,
le 4-[1-(4-(1-méthyléthyl)phénylsulfonyl)-5-trifluorométhyl-1H-pyrrolo[2,3-c]pyridin-2-ylméthyl]-benzoate de méthyle,
l'acide 4-[[1-[[4-(1-méthyléthyl)phényl]sulfonyl]-5-(trifluorométhyl)-1H-pyrrolo[2,3-c)pyridin-2-yl]méthyl)-benzoïque,
le 4-[1-(4-(1-méthyléthyl)phénylsulfonyl)-5-trifluorométhyl-1H-pyrrolo(3,2-b]pyridin-2-ylméthyl]-benzoate de méthyle,
l'acide 4-[1-(4-(1-méthyléthyl)phénylsulfonyl)-5-trifluorométhyl-1H-pyrrolo(3,2-b]pyridin-2-ylméthyl]-benzoïque,
le 4-[[1-[(3,4-dihydro-4-méthyl-2H-1,4-benzoxazin-6-yl)sulfonyl]-5-(trifluorométhyl)-1H-pyrrolo[2,3-b]pyridin-2-yl]méthyl]-benzoate de méthyle,
l'acide 4-[[1-[(3,4-dihydro-4-méthyl-2H-1,4-benzoxazin-6-yl)sulfonyl]-5-(trifluorométhyl)-1 H-pyrrolo[2,3-b]pyridin-2-yl]méthyl]-benzoïque,
le 4-[1-(4-(1-méthyléthyl)phénylsulfonyl)-5-chloro-1H-pyrrolo[2,3-c]pyridin-2-ylméthyl]-benzoate de méthyle,
l'acide 4-[[5-chloro-1-[[4-(1-méthyléthyl)phényl]sulfonyl]-1H-pyrrolo[2,3-c]pyridin-2-yl]méthyl)-benzoïque,
le 4-[1-(3-(1,1-diméthyléthyl)phénylsulfonyl)-5-trifluorométhyl-1H-pyrrolo[3,2-b)pyridin-2-ylméthyl]-benzoate de méthyle,
l'acide 4-[[1-[[3-(1,1-diméthyléthyl)phényl]sulfonyl]-5-(triftuorométhyl)-1H-pyrolo[3,2-b]pyridin-2-yl]méthyl]-benzoïque,
le 4-{Hydroxy-[1-(3-(1-méthyléthyl)phénylsulfonyl)-5-trifluorométhyl-1H-pyrrolo[2,3-b)pyridin-2-yl]-méthyl}-benzoate de méthyle,
le 4-[1-(3-(1-méthyléthyl)phénylsulfonyl)-5-trifluorométhyl-1H-pyrrolo[2,3-b]pyridin-2-ylméthyl]-benzoate de méthyle,
l'acide 4-[[1-[[3-(1-méthyléthyl)phényl]sulfonyl)-5-(trifluorométhyl)-1H-pyrrolo[2,3-b]pyridin-2-yl]méthyl]-benzoïque,
le 4-{Hydroxy-[1-(3-(1,1-diméthyléthyl)phénylsulfonyl)-5-trifluorométhyl-1H-pyrrolo[2,3-b]pyridin-2-yl]-méthyl}-benzoate de méthyle,
le 4-[1-(3-(1,1-diméthyléthyl)phénylsulfonyl)-5-trifluorométhyl-1H-pyrrolo[2,3-b]pyridin-2-ylméthyl]-benzoate de méthyle,
l'acide 4-[[1-[[3-(1,1-diméthyléthyl)phényl]sulfonyl]-5-(trifluorométhyl)-1H-pyrrolo[2,3-b]pyridin-2-yl] méthyl]-benzoïque,
le 4-{Hydroxy-[5-chloro-1-(4-(1-méthyléthyl)phénylsulfonyl)-1H-pyrrolo[2,3-b]pyridin-2-yl]-méthyl}-benzoate de méthyle,
le 4-[5-Chloro-1-(4-(1-méthyléthyl)phénylsulfonyl)-1H-pyrrolo[2,3-b]pyridin-2-ylméthyl]-benzoate de méthyle,
l'acide 4-[[5-chloro-1-[[4-(1-méthyléthyl)phényl]sulfonyl]- 1 H-pyrrolo-[2,3-b]pyridin-2-yl] méthyl]-benzoïque,
l'acide 5- {hydroxy-[1-(3-(1,-diméthyléthyl)phénylsulfonyl)-5-trifluorométhyl-1H-pyrrolo[3,2-b]pyridin-2-yl]-méthyl }-thiophène-2-carboxylique,
le 5-[[1-[[3-(1,1-diméthyléthyl)phényl]sulfonyl]-5-(trifluorométhyl)-1H-pyrrolo[3,2-b]pyridin-2-yl]-fluorométhyl]-N,N-diéthyl-2-thiophénecarboxamide,
l'acide 5-{1-(3-(1,1-diméthyléthyl)phénylsulfonyl)-5-trifluorométhyl-1H-pyrrolo[3,2-b)pyridine-2-carbonyl}-thiophène-2-carboxylique,
l'acide 4-[[5-chloro-1-[[4-(1-méthyléthyl)phényl]sulfonyl]-1H-pyrrolo-[2,3-b]pyridin-2-yl]méthyl]-benzoïque, sel de sodium,
l'acide 4-[[5-chloro-1-[[4-(1-méthyléthyl)phényl]sulfonyl]-1H-pyrrolo-[2,3-b]pyridin-2-yl)méthyl]-benzoïque, sel de pipérazine,
l'acide 4-[[1-[[3-(1,1-diméthyléthyl)phényl]sulfonyl]-3-méthyl-5-(trifluorométhyl)-1H-pyrolo[3,2-b]pyridin-2-yl]méthyl]-benzoïque,
l'acide 5-[[1-[[4-(1,1-méthyléthyl)phényl]sulfonyl]-5-chloro-1H-pyrrolo[2,3-b]pyridin-2-yl)méthyl]-thiophène-2-carboxylique,
le N-{4-[1-(3-tert-butyl-benzènesulfonyl)-5-trifluorométhyl-1H-pyrrolo[3,2-b]pyridin-2-ylméthyl]-benzoyl}-méthanesulfonamide.

Les synthèses décrites ci-après, y compris dans les préparations et les exemples, illustrent des méthodes de préparation des composés de formule (I). Dans ces synthèses, les substituants A, Cy, R1, R2, R3, R4, R5, R6, R7 et R8 ont la signification indiquée ci-dessus pour les composés de formule (I), sauf indication contraire.

Selon un premier mode de réalisation, les composés de formule (I) dans lesquels R4 représente H, OH ou (C₁-C₄)alkyle et R5 représente H peuvent être préparés comme décrit dans le schéma 1.

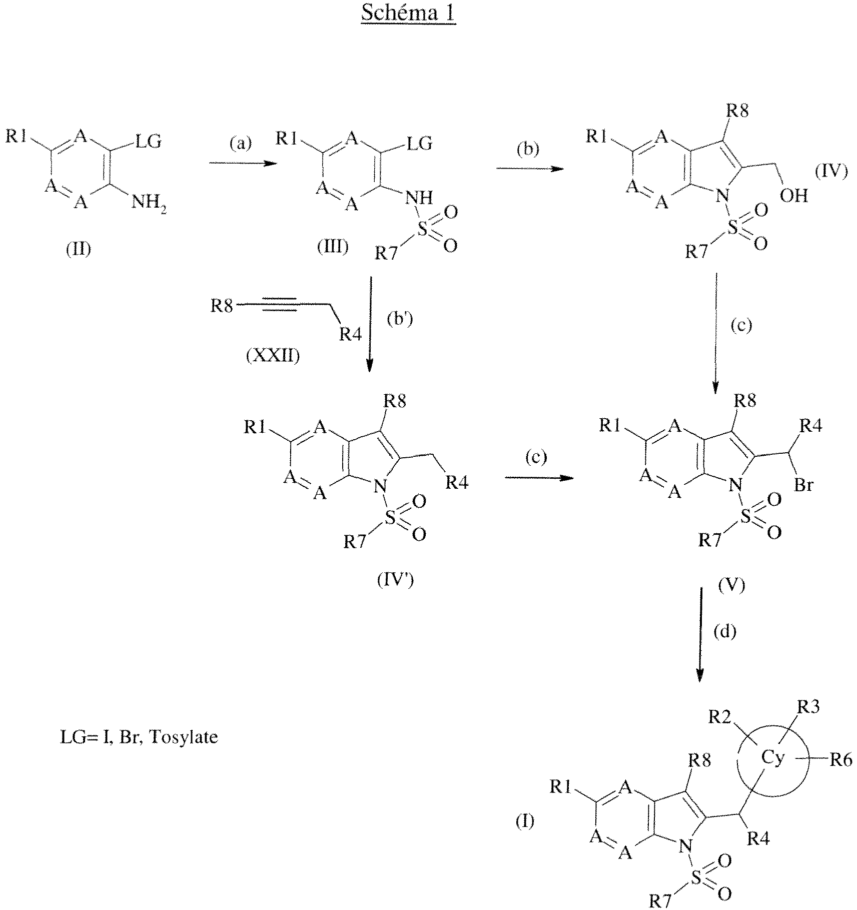

### Etape (a)

On fait réagir une amine de formule (II), dans lequel LG est de préférence l'iode, avec un chlorure de sulfonyle R7SO₂Cl en présence d'une base convenable, comme par exemple la pyridine, à température ambiante pendant environ 2 à 24 heures. On fait ensuite réagir le milieu réactionnel ainsi obtenu avec une base convenable, comme par exemple la potasse, dans un solvant convenable, comme par exemple le dioxane, à une température comprise entre la température ambiante et la température de reflux du solvant, pendant une durée d'environ 1 à 6 heures. On obtient ainsi le composé de formule (III).

### Etape (b)

On fait réagir le composé de formule (III) avec du propyne-2 ol-1 en présence d'iodure de cuivre et d'un catalyseur à base de palladium, comme par exemple le chlorure de bis(triphénylphosphine)palladium(II), dans un solvant convenable, comme par exemple le N,N-diméthylformamide (DMF). On ajoute ensuite une base convenable, comme par exemple la diéthylamine ou la triéthylamine, et on chauffe le mélange réactionnel pendant une durée d'environ 1 à 6 heures à une température comprise entre la température ambiante et la température de reflux du solvant. Selon une variante, le chauffage peut être effectué au four micro-ondes pendant une durée d'environ 5 à 30 minutes. On obtient ainsi le composé de formule (IV) dans laquelle R8 représente un atome d'hydrogène.

### Etape (b')

On fait réagir le composé de formule (III) avec un alcyne de formule (XXII) en présence d'un catalyseur à base de palladium, comme par exemple l'acétate de palladium, et de chlorure de lithium dans un solvant convenable, comme par exemple le N,N-diméthylformamide (DMF). On ajoute ensuite une base convenable, comme par exemple le carbonate de potassium et on chauffe le mélange réactionnel pendant une durée d'environ 1 à 24 heures à une température comprise entre la température ambiante et la température de reflux du solvant. Selon une variante, le chauffage peut être effectué au four micro-ondes pendant une durée d'environ 5 à 30 minutes. On obtient ainsi le composé de formule (IV') dans laquelle R8 représente un groupe (C₁-C₄)alkyle.

### Etape (c)

On fait réagir le composé de formule (IV) ou (IV') avec une source de brome, comme par exemple le tribromure de phosphore, dans un solvant convenable, comme par exemple le dichlorométhane (DCM), à 0°C ou à température ambiante pendant une durée d'environ 1 heure à 4 jours, ou le N-bromosuccinimide (NBS) en présence d'azobisisobutyronitrile (AIBN) dans un solvant convenable comme par exemple le tétrachlorure de carbone au reflux pendant 24 heures. On obtient ainsi le composé de formule (V).

### Etape (d)

On fait réagir le composé de formule (V) en solution dans un solvant convenable, comme par exemple un mélange éthanol/dioxane, avec un composé de formule (HO)₂B-Cy(R2,R3)-R6 en présence d'un catalyseur à base de palladium, comme par exemple le complexe Pd(dppf)Cl₂, CH₂Cl₂, et d'une base convenable, comme par exemple le carbonate de potassium, et on chauffe le mélange réactionnel pendant une durée d'environ 1 à 6 heures à une température comprise entre la température ambiante et la température de reflux du solvant. Selon une variante, le chauffage peut être effectué au four micro-ondes pendant une durée d'environ 5 à 30 minutes. On obtient ainsi le composé de formule (I). Si nécessaire (lorsque R6 = COOR9 et R9 = (C₁-C₄)alkyle), on hydrolyse la fonction ester du composé de formule (I), par exemple par action d'une base minérale telle que l'hydroxyde de lithium, selon des modes opératoires bien connus de l'homme du métier, pour obtenir un composé de formule (I) dans laquelle R6 = COOH.

Selon un second mode de réalisation, les composés de formule (I) dans lesquels R4 et R5 représentent H peuvent être préparés comme décrit dans le schéma 2.

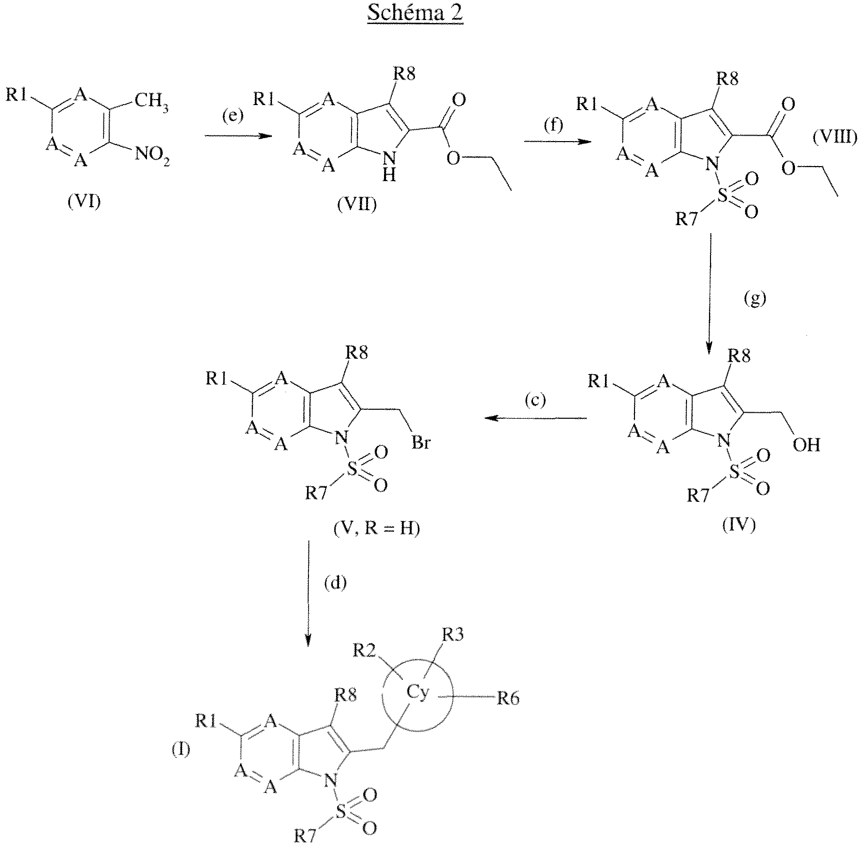

### Etape (e)

On fait réagir le composé de formule (VI) avec de l'oxalate de diéthyle en présence d'une base convenable, comme par exemple le 1,8-diazabicyclo[5.4.0]undéc-7-ène (DBU), à température ambiante, pendant une durée d'environ 1 à 6 heures. Si nécessaire, on fait réagir le composé obtenu avec un agent halogénant comme par exemple le 1-chlorométhyl-4-fluoro-1,4-diazoniabicyclo[2.2.2]octane bis(tétrafluoroborate), dans un solvant convenable, comme par exemple l'acétonitrile, à une température comprise entre 0 et 50°C, pendant une durée d'environ 1 à 96 heures. On obtient ainsi le composé de formule (VII).

### Etape (f)

On traite le composé de formule (VII) par une base convenable, comme par exemple l'hydrure de sodium, dans un solvant convenable, comme par exemple le tétrahydrofurane (THF, à température ambiante pendant une durée d'environ 1 à 6 heures, puis on fait réagir un chlorure de sulfonyle R7SO₂Cl à température ambiante pendant une durée d'environ 2 à 24 heures. On obtient ainsi le composé de formule (VIII).

### Etape (g)

On traite le composé de formule (VIII) avec un agent réducteur convenable, comme par exemple DIBAL-H ou LiAlH₄, dans un solvant convenable, comme par exemple le toluène, à une température comprise entre environ -78°C et la température ambiante, pendant une durée d'environ 1 à 24 heures. On obtient ainsi le composé de formule (IV).

### Etape (c)

Cette étape est identique à l'étape (c) décrite pour le schéma 1, et conduit à l'obtention du composé de formule (V).

### Etape (d)

Cette étape est identique à l'étape (d) décrite pour le schéma 1, et conduit à l'obtention du composé de formule (I). Si nécessaire (lorsque R6 = COOR9 et R9 = (C₁-C₄)alkyle), on hydrolyse la fonction ester du composé de formule (I), par exemple par action d'une base minérale telle que l'hydroxyde de lithium, selon des modes opératoires bien connus de l'homme du métier, pour obtenir un composé de formule (I) dans laquelle R6 = COOH.

Selon un troisième mode de réalisation, les composés de formule (I) dans lesquels R2, R3, R4, R5 et R8 représentent H, Cy représente un thiazolyle et R6 représente COOH peuvent être préparés comme décrit dans le schéma 3.

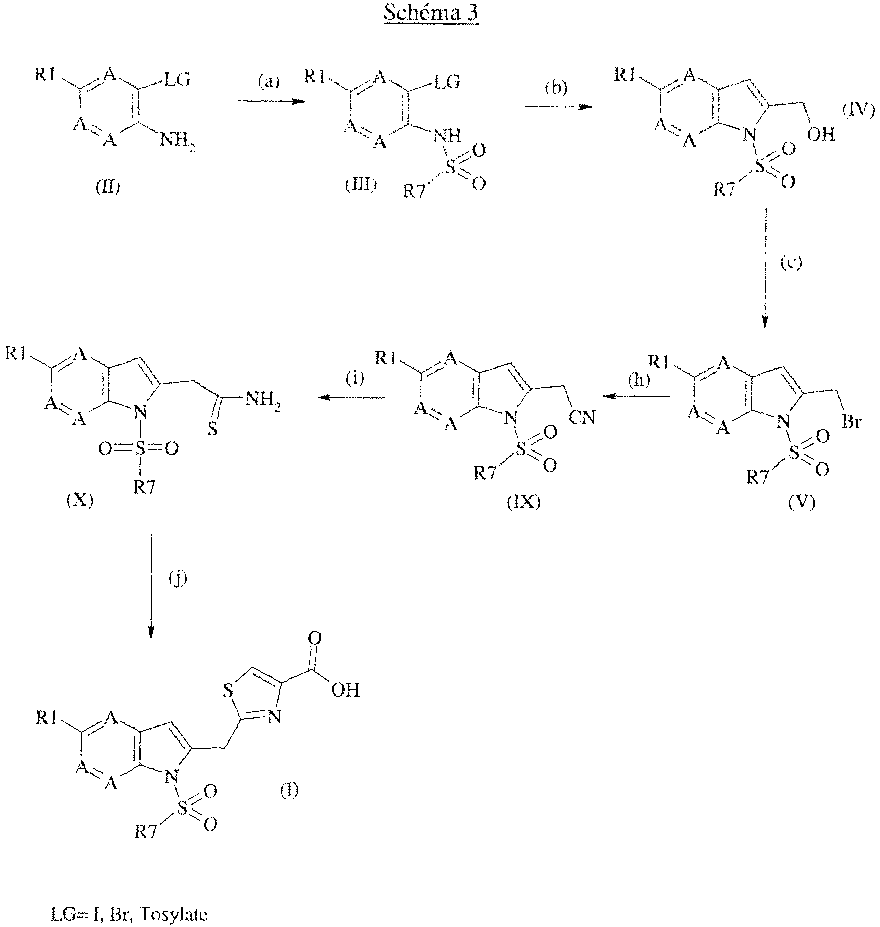

### Etape (a)

Cette étape est identique à l'étape (a) décrite pour le schéma 1, et conduit à l'obtention du composé de formule (III).

### Etape (b)

Cette étape est identique à l'étape (b) décrite pour le schéma 1, et conduit à l'obtention du composé de formule (IV).

### Etape (c)

Cette étape est identique à l'étape (c) décrite pour le schéma 1, et conduit à l'obtention du composé de formule (V).

### Etape (h)

On fait réagir le composé de formule (V) avec du cyanure de potassium dans un solvant convenable, comme par exemple le DCM, en présence d'un catalyseur de transfert de phase, comme par exemple le bromure de tétrabutylammonium, à température ambiante pendant une durée d'environ 1 à 4 jours. On obtient ainsi le composé de formule (IX).

### Etape (i)

On fait réagir le composé de formule (IX) en solution dans un solvant convenable, comme par exemple un mélange THF/eau, avec du dithiophosphate de diéthyle à une température d'environ 80 à 120 °C pendant une durée d'environ 1 à 6 heures. On obtient ainsi le composé de formule (X).

### Etape (j)

On fait réagir le composé de formule (X) avec de l'acide bromopyruvique dans un solvant convenable, comme par exemple l'éthanol, à température ambiante pendant une durée d'environ 12 à 36 heures. On obtient ainsi le composé de formule (I).

Selon un quatrième mode de réalisation, les composés de formule (I) dans lesquels R4 et R8 représentent H et Cy représente un phényle peuvent être préparés comme décrit dans le schéma 4.

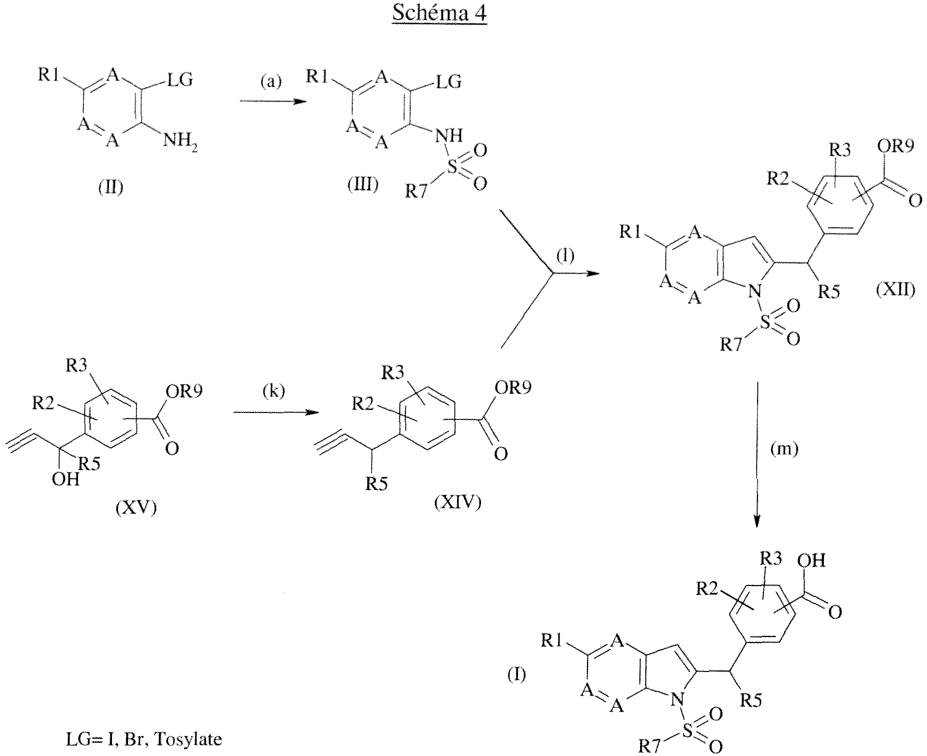

### Etape (a)

Cette étape est identique à l'étape (a) décrite pour le schéma 1, et conduit à l'obtention du composé de formule (III).

### Etape (k)

On réduit le dérivé acétylénique de formule (XV) en présence d'un trialkylsilane, comme par exemple le triéthylsilane, et d'une quantité catalytique optionnelle d'un acide, comme par exemple l'acide trifluoroacétique, dans un solvant convenable, comme par exemple le DCM, à température ambiante pendant une durée d'environ 4 à 8 jours. On obtient ainsi le composé de formule (XIV).

### Etape (l)

On fait réagir les composés de formule (III) et (XIV) en présence d'iodure de cuivre et d'un catalyseur à base de palladium, comme par exemple le chlorure de bis(triphénylphosphine)palladium(II), dans un solvant convenable, comme par exemple le N,N-diméthylformamide (DMF). On ajoute ensuite une base convenable, comme par exemple la diéthylamine ou la triéthylamine, et on chauffe le mélange réactionnel pendant une durée d'environ 1 à 6 heures à une température comprise entre la température ambiante et la température de reflux du solvant. Selon une variante, le chauffage peut être effectué au four micro-ondes pendant une durée d'environ 5 à 30 minutes. On obtient ainsi le composé de formule (XII).

### Etape (m)

Lorsque R9 est différent de H, on hydrolyse la fonction ester du composé de formule (XII) selon des modes opératoires bien connus de l'homme du métier, par exemple par action d'une base minérale telle que l'hydroxyde de lithium, dans un solvant convenable comme le THF ou un mélange THF/eau, à une température comprise entre la température ambiante et la température de reflux du solvant. On obtient ainsi le composé de formule (I).

Selon un cinquième mode de réalisation, les composés de formule (I) dans lesquels R8 représente H peuvent être préparés comme décrit dans le schéma 5.

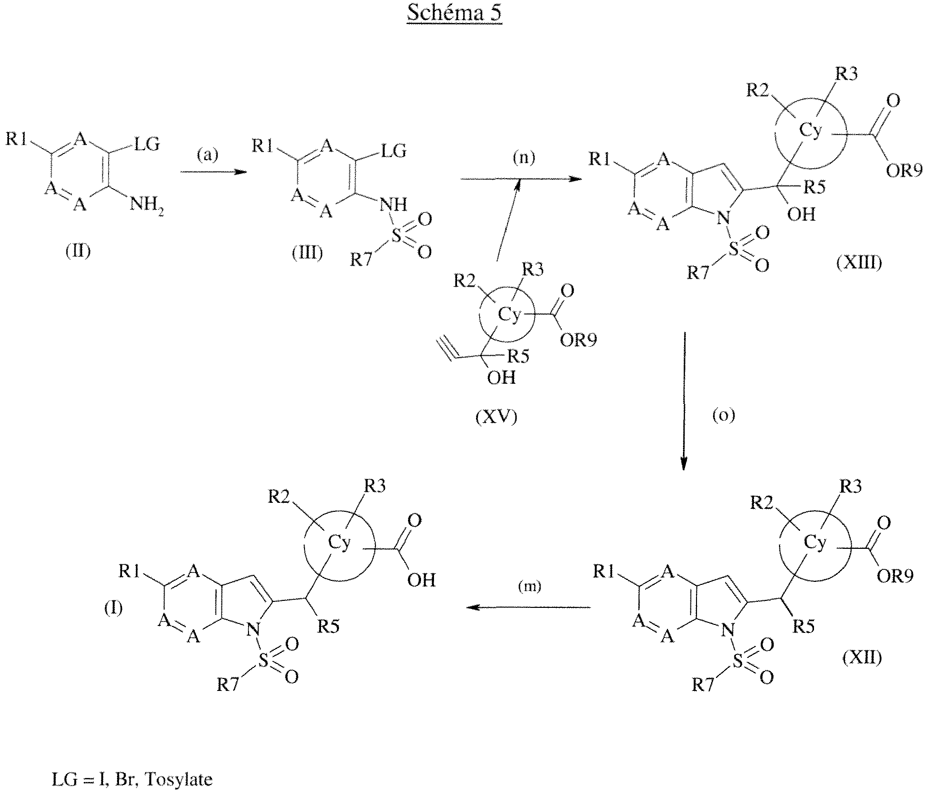

### Etape (a)

Cette étape est identique à l'étape (a) décrite pour le schéma 1, et conduit à l'obtention du composé de formule (III).

### Etape (n)

On fait réagir les composés de formule (III) et (XV) en présence d'iodure de cuivre et d'un catalyseur à base de palladium, comme par exemple le chlorure de bis(triphénylphosphine)palladium(II), dans un solvant convenable, comme par exemple le DMF. On ajoute ensuite une base convenable, comme par exemple la diéthylamine ou la triéthylamine, et on chauffe le mélange réactionnel pendant une durée d'environ 1 à 6 heures à une température comprise entre la température ambiante et la température de reflux du solvant. Selon une variante, le chauffage peut être effectué au four micro-ondes pendant une durée d'environ 5 à 30 minutes. On obtient ainsi le composé de formule (XIII).

### Etape (o)

On réduit le composé de formule (XIII) en présence d'un trialkylsilane, comme par exemple le triéthylsilane, de diéthyléthérate de tritluorure de bore et d'une quantité catalytique optionnelle d'un acide, comme par exemple l'acide tritluoroacétique, dans un solvant convenable, comme par exemple le DCM, à température ambiante pendant une durée d'environ 6 à 18 heures. On obtient ainsi le composé de formule (XII).

### Etape (m)

Cette étape est identique à l'étape (m) décrite pour le schéma 4, et conduit à l'obtention du composé de formule (I).

Selon un sixième mode de réalisation, les composés de formule (I) dans lesquels R4 et R5 forment ensemble avec l'atome de carbone auquel ils sont rattachés un groupe éthylène (C=CH₂), R6 représente COOH et R8 représente H peuvent être préparés comme décrit dans le schéma 6.

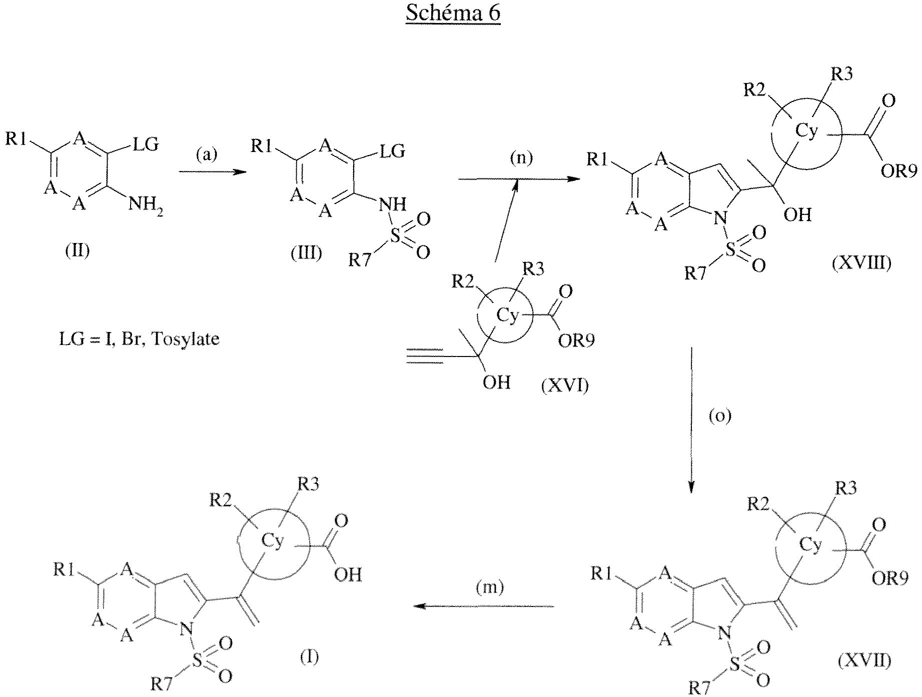

### Etape (a)

Cette étape est identique à l'étape (a) décrite pour le schéma 1, et conduit à l'obtention du composé de formule (III).

### Etape (n)

Cette étape est identique à l'étape (n) décrite pour le schéma 5, et conduit à l'obtention du composé de formule (XVIII).

### Etape (o)

Cette étape est identique à l'étape (o) décrite pour le schéma 5, et conduit à l'obtention du composé de formule (XVII).

### Etape (m)

Cette étape est identique à l'étape (m) décrite pour le schéma 4, et conduit à l'obtention du composé de formule (I).

Selon un septième mode de réalisation, les composés de formule (I) dans lesquels R4 et R5 forment ensemble avec l'atome de carbone auquel ils sont rattachés un groupe carbonyle et R8 représente H peuvent être préparés comme décrit dans le schéma 7.

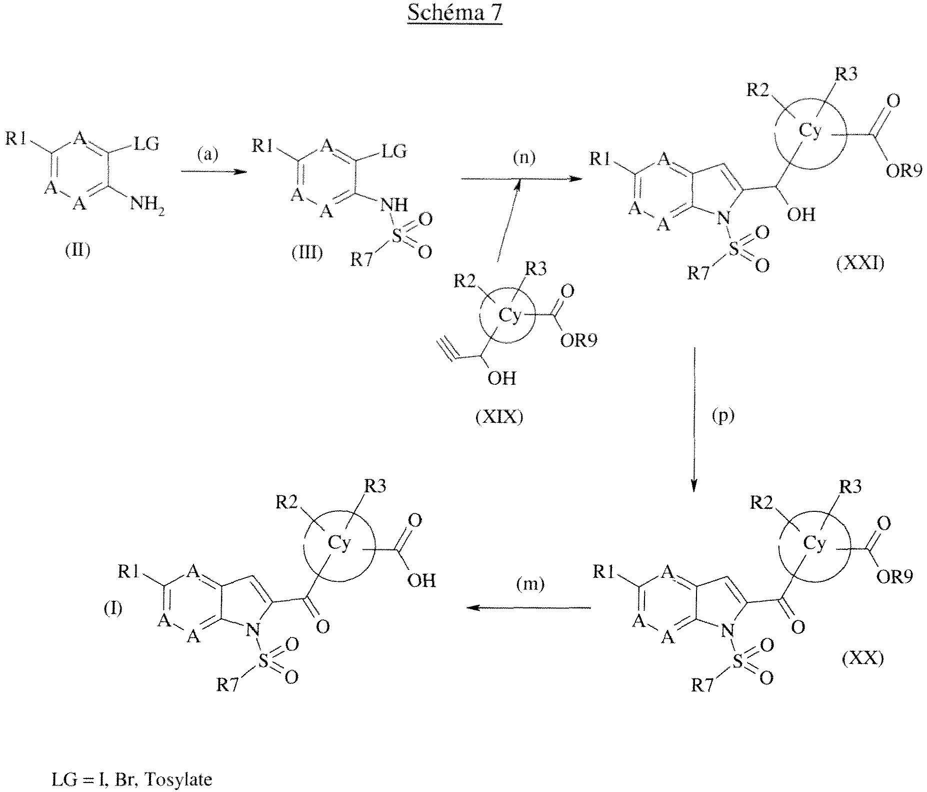

### Etape (a)

Cette étape est identique à l'étape (a) décrite pour le schéma 1, et conduit à l'obtention du composé de formule (III).

### Etape (n)

Cette étape est identique à l'étape (n) décrite pour le schéma 5, et conduit à l'obtention du composé de formule (XXI).

### Etape (p)

On traite le composé de formule (XXI) avec un agent oxydant convenable, comme par exemple le dichromate de pyridinium, dans un solvant convenable, comme par exemple le DCM, à température ambiante pendant une durée d'environ 6 à 18 heures. On obtient ainsi le composé de formule (XX).

### Etape (m)

Cette étape est identique à l'étape (m) décrite pour le schéma 4, et conduit à l'obtention du composé de formule (I).

Selon un huitième mode de réalisation, les composés de formule (I) dans lesquels R4 représente un atome d'halogène et R5 représente un atome d'hydrogène peuvent être préparés comme décrit dans le schéma 8.

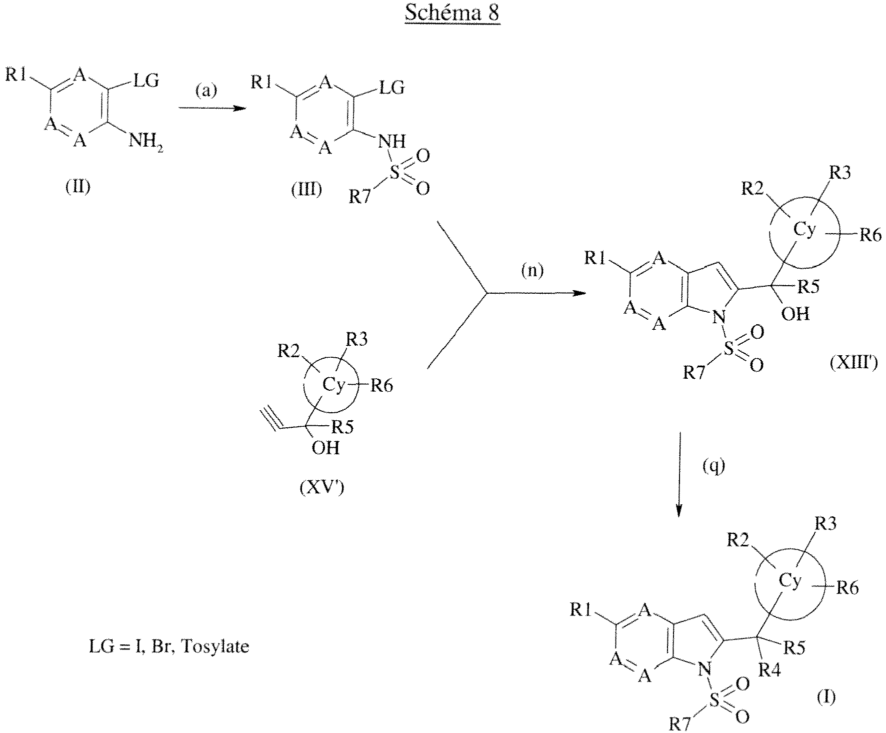

### Etape (a)

Cette étape est identique à l'étape (a) décrite pour le schéma 1, et conduit à l'obtention du composé de formule (III).

### Etape (n)

Cette étape est identique à l'étape (n) décrite pour le schéma 5, et conduit à l'obtention du composé de formule (XIII').

### Etape (q)

On fait réagir le composé de formule (XIII') avec un agent halogénant, comme par exemple le 1-chlorométhyl-4-fluoro-1,4-diazoniabicyclo[2.2.2]octane bis(tétrafluoroborate) à température ambiante pendant environ 30 minutes à 2 heures, pour obtenir le composé de formule I.

De manière générale, la fonction acide carboxylique des composés de formule (I) dans laquelle R6 représente COOH peut être avantageusement remplacée par un groupe bioisostère d'acide carboxylique selon des méthodes bien connues par l'homme du métier telles que les méthodes décrites ci-après.

Les composés de formule (I) selon l'invention, dans laquelle R6 représente un groupe bioisostère acylhydrazine, acyl-hydrazine carboxylate ou oxadiazolone peuvent être préparés selon un procédé consistant à :
a) faire réagir le composé de formule (I) dans laquelle R6 représente COOH sur un carbazate en présence d'un agent de couplage tel que notamment le couple de réactifs 1-(3-diméthylaminopropyl)-3-éthyl-carbodiimide (EDCI) / 1-hydroxy-7-azabenzotriazole (HOAT), dans un solvant organique tel que en particulier du toluène à température ambiante et pendant 2 à 24 heures pour conduire à un composé acylhydrazine carboxylate de formule (I) dans laquelle R6 représente CONHNHCOOR11 et R11 représente un atome d'hydrogène ou un groupe alkyle ayant 1 à 4 atomes de carbone ;
b) si nécessaire, déprotéger le composé de formule (I) précité selon un mode opératoire bien connu de l'homme du métier comme par exemple en traitant ledit composé de formule (I) par un acide tel que l'acide trifluoroacétique dans un solvant tel que en particulier le dichlorométhane, pour obtenir une acyl-hydrazine ;
c) si nécessaire, cycliser l'acyl-hydrazine en présence d'un agent de condensation tel que le carbonyldiimidazole (CDI) dans un solvant organique tel que le dichlorométhane, à température ambiante et pendant 2 à 15 heures pour obtenir l'oxadiazolone de formule (I) dans laquelle R6 représente :

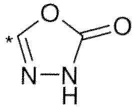

Les composés de formule (I) selon l'invention, dans laquelle R6 représente un groupe bioisostère carboxamide peuvent être préparés selon un procédé consistant à faire réagir le composé de formule (I) dans laquelle R6 représente COOH sur une amine en présence d'un agent de couplage tel que notamment le couple de réactifs 1-(3-diméthylaminopropyl)-3-éthyl-carbodiimide (EDCI) / 1-hydroxy-7-azabenzotriazole (HOAT), dans un solvant organique tel que en particulier du dichlorométhane à température ambiante et pendant 2 à 24 heures pour conduire à un composé carboxamide de formule (I) dans laquelle R6 représente CONR12R13 et R12,R13 représentent indépendamment un atome d'hydrogène ou un groupe alkyle ayant 1 à 4 atomes de carbone.

Les composés de formule (I) selon l'invention, dans laquelle R6 représente un groupe bioisostère sulfonylcarbamoyl ou un groupe dérivé peuvent être préparés selon un procédé consistant à coupler le composé de formule 1 dans laquelle R6 représente COOH avec un sulfonamide en présence d'un agent de couplage tel que en particulier le couple de réactifs 1-(3-diméthylaminopropyl)-3-éthyl-carbodiimide chlorhydrate / 4-diméthylaminopyridine (EDCI/DMAP) dans un solvant organique tel que le dichlorométhane à température ambiante pendant 12 à 24 heures.

Les composés de formules (I) selon l'invention, dans laquelle R6 représente un groupe bioisostère isoxazole ou un groupe dérivé tel qu'un groupe isoxazolone peuvent être préparés selon un procédé consistant à :
a) activer la fonction acide du composé de formule (I) dans laquelle R6 représente COOH à l'aide du carbonyldiimidazole (CDI) et le faire réagir sur le sel de magnésium du monomalonate d'éthyle;
b) cycliser en présence d'hydroxylamine et en milieu basique à température ambiante pendant 2 à 4 jours pour obtenir le composé de formule I dans laquelle R6 représente :

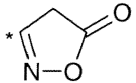

Les composés de formule R7SO₂Cl, (HO)₂B-Cy(R2,R3)-R6, (II), (VI), (XV), (XV'), (XVI), (XIX) et (XXII) sont disponibles dans le commerce ou peuvent être préparés selon des modes opératoires bien connus de l'homme du métier. La présente invention concerne les procédés de préparation décrits ci-dessus, ainsi que les intermédiaires mis en oeuvre dans ces procédés. En particulier, l'invention a pour objet les composés de formule (IV), (IV'), (V), (VII), (VIII), (IX), (X), (XII), (XIII), (XIII'), (XVII), (XVIII), (XX), (XXI), ainsi que les sels éventuels de ces composés.

Les exemples suivants de préparation de composés selon la formule (I) permettront de mieux comprendre l'invention.

Dans ces exemples, qui ne sont pas limitatifs de la portée de l'invention, on désigne par «préparation» les exemples décrivant la synthèse de composés intermédiaires et par «exemples» ceux décrivant la synthèse de composés de formule (I) selon l'invention.

Les abréviations suivantes ont été utilisées :
- CuI: iodure de cuivre
- DAST: Trifluorure de diéthylaminosulfure
- DIBAL-H: Hydrure de diisobutylaluminium
- DBU: 1,8-diazabicyclo[5.4.0]undéc-7-ène
- DCM: dichlorométhane
- DMF: N,N-diméthylformamide
- DMSO: diméthylsulfoxyde
- eq: équivalent
- h: heure
- HCl: Acide chlorhydrique
- min: minutes
- mM: millimole
- TA: température ambiante
- TEMPO: 2,2,6,6-Tétraméthylpipéridine-1-oxyl
- TFA: acide trifluoroacétique
- THF: tétrahydrofurane

Les points de fusion (F) ont été mesurés à l'aide d'un appareil automatique, (Optimelt) et les valeurs spectrales de Résonance Magnétique Nucléaire ont été caractérisées par le déplacement chimique (δ) calculé par rapport au TMS (tétraméthylsilane), par le nombre de protons associés au signal et par la forme du signal (s pour singulet, d pour doublet, t pour triplet, q pour quadruplet, m pour multiplet, dd pour doublet de doublet). La fréquence de travail (en MégaHerz) et le solvant utilisés sont indiqués pour chaque composé.

La température ambiante est de 20°C ± 5°C.

### Préparation 1: 3-(1,1-diméthyléthyl)-N-(2-iodo-6-trifluorométhyl-pyridin-3-yl)-benzènesulfonamide

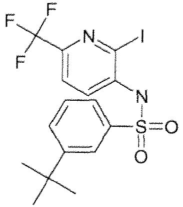

On a ajouté 9,07 g (38,96 mM) de chlorure de 3-tert-butyl benzènesulfonyle à une solution de 6,60 g (22,92 mM) de 2-Iodo-6-trifluorométhyl-pyridin-3-ylamine dans 10,0 mL de pyridine. Ce mélange réactionnel a été agité à TA pendant une nuit. Le milieu a été dilué dans l'eau puis extrait à l'acétate d'éthyle. La phase organique a été lavée avec une solution d'acide chlorhydrique 1N puis séchée sur sulfate de magnésium, filtrée puis concentrée sous pression réduite. 14,40 g du résidu d'évaporation ont été solubilisés dans 50,0 mL de 1,4-dioxane, et 56,4 mL (169,28 mM) d'hydroxyde de potassium 3M ont été ajoutés, puis le mélange réactionnel a été agité sous reflux pendant 1 heure. Le milieu a été concentré sous pression réduite. Le résidu a été solubilisé dans l'eau, et la solution a été acidifiée avec de l'acide chlorhydrique concentré puis extraite au DCM 2 fois. La phase organique a été séchée sur sulfate de magnésium, filtrée puis concentrée sous pression réduite. Le résidu a été lavé avec de l'éther de pétrole puis filtré sur Büchner. Le produit visé en titre a été obtenu sous la forme d'un solide blanc (8,80 g, rendement = 86%). F = 125°C.

### Préparation 2: [1-(3-(1,1-diméthyléthyl)phénylsulfonyl)-5-trifluorométhyl-1H-pyrrolo(3,2-b]pyridin-2-yl]-méthanol

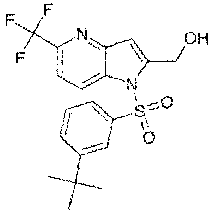

On a préparé une solution de 2,00 g (4,13 mM) de 3-tert-Butyl-N-(2-iodo-6-trifluorométhyl-pyridin-3-yl)-benzènesulfonamide (préparation 1) dans 10,0 mL de DMF. On a additionné 0,04 g (0,21 mM) de Cul, 0,14 g (6,19 mM) de chlorure de bis(triphénylphosphine)palladium(II), et 0,35 g (6,19 mM) de propyne-2 ol-1. On a ajouté à ce mélange 5,0 mL de triéthylamine puis ce mélange réactionnel a été irradié aux micro-ondes 10 min à 120 °C. Le milieu a été concentré sous pression réduite, et le résidu d'évaporation a été purifié par chromatographie sur gel de silice à l'aide d'un éluant cyclohexane / acétate d'éthyle 90/10 puis 80/20 (v/v). Le produit visé en titre a été obtenu sous la forme d'un solide beige (1,29 g; rendement = 76%). F = 118°C.

### Préparation 3: 2-Bromométhyl-1-(3-1,1-diméthyléthyl)phénylsulfonyl)-5-trifluorométhyl-1H-pyrrolo[3,2-b]pyridine

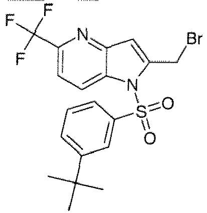

On a préparé une solution de 1,29 g (3,13 mM) de [1-(3-(1,1-diméthyléthyl)phénylsulfonyl)-5-trifluorométhyl-1H-pyrrolo[3,2-b]pyridin-2-yl]-méthanol (préparation 2) dans 25,0 mL de DCM. 1,69 g (6,26 mM) de tribromure de phosphore ont été ajoutés goutte à goutte. Ce mélange réactionnel a été agité 4 jours à TA. On a ajouté 100 mL d'une solution saturée de carbonate de potassium et 100 mL d'eau, puis on a extrait deux fois au DCM (50 mL). Les phases organiques ont été réunies, séchées sur sulfate de magnésium, filtrées et concentrées sous pression réduite. Le produit visé en titre a été obtenu sous la forme d'un solide beige (1,62 g, rendement = 100%). F = 124°C.

### Exemple 1 : Acide 5-[[1-[[3-(1,1diméthyléthyl)phényl]sulfonyl-5-(trifluorométhyl)-1H-pyrrolo[3,2-b]pyridin-2-yl]méthyl]-thiophène-2-carboxylique

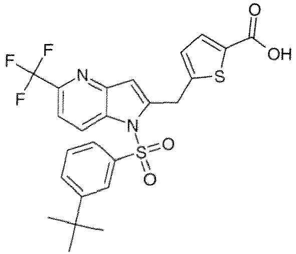

On a préparé une solution de 230 mg (0,48 mM) de 2-Bromométhyl-1-(3-tert-butyl-benzènesulfony)-5trifluorométhyl-1H-pyrolo[3,2-b]pyridine (préparation 3) dans 4,0 ml d'éthanol et 1,0 mL de 1,4-dioxane. On a ajouté à cette solution 99.8 mg (0,58 mM) d'acide 5-(Dihydroxyboryl)-2-thiophènecarboxylique, 39,5 mg (0,58 mM) de complexe Pd(dppf)Cl₂, DCM et 80 mg (0,58 mM) de carbonate de potassium. Ce mélange réactionnel a été irradié aux micro-ondes 20 min à 120°C. Le milieu a été dilué dans de l'eau puis extrait à l'acétate d'éthyle. La phase organique a été lavée avec une solution d'eau saturée en sel, puis séchée sur sulfate de magnésium, filtrée et concentrée sous pression réduite. Le résidu d'évaporation a été purifié par chromatographie liquide avec détection par UV (LC-UV) (sur colonne SunFire® C18) en éluant par un mélange eau / méthanol / 0.1% TFA. Les fractions contenant le produit attendu ont été réunies et concentrées à sec sous pression réduite. Le produit visé en titre a été obtenu sous la forme d'une huile orange (25 mg, rendement = 10%).
¹H RMN (400 MHz, DMSO) δ = 13,00 (s, 1H), 8,68 (d, 1H), 7,85 (d, 1H), 7,75 (d, 1H), 7,67-7,70 (m, 2H), 7,55 (d, 1H), 7,48 (t, 1H), 7,00 (d, 2H), 4,77 (s, 2H), 1,18 (s, 9H).

### Préparation 4 : 4-Méthyl-5-nitro-2-trifluorométhyl-pyridine

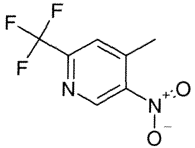

On a mis en suspension 25,0 g (115,2 mM) de 2-bromo-4-méthyl-5-nitropyridine, 44,3 g (230 mM) de fluorosulfonyldifluoroacétate de méthyle et 17,6 g (92,2 mM) de Cul dans 250 mL de DMF. Ce mélange réactionnel a été agité à 120 °C pendant 48h. Le milieu a été refroidi puis dilué avec 1000 mL d'une solution saturée en chlorure d'ammonium et 100 mL d'hydroxyde d'ammonium, puis on a agité jusqu'à homogénéisation. Le produit a été extrait trois fois avec de l'acétate d'éthyle. Le résidu a été purifié par chromatographie sur gel de silice à l'aide d'un éluant cyclohexane / acétate d'éthyle 95/5 puis 90/10 (v/v). Les fractions contenant le produit attendu ont été réunies et concentrées à sec sous pression réduite. Le produit visé en titre a été obtenu sous la forme d'une huile brune (8,0 g, rendement = 34%).
¹H RMN (300 MHz, DMSO) δ = 9,29 (s, 1H), 8,21 (s, 1H), 2,68 (s, 3H).

### Préparation 5: 5-Trifluorométhyl-1H-pyrrolo[2,3-c]pyridine-2-carboxylate d'éthyle

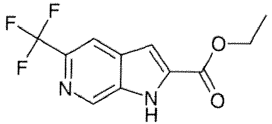

On a préparé une solution de 6,0 g (23,29 mM) de 4-Méthyl-5-nitro-2-trifluorométhyl-pyridine (préparation 4) dans 14,8 mL (109,45 mM) d'oxalate de diéthyle. On a ajouté 8,65 g (56,8 mM) de DBU et le mélange a été agité 4h à TA. Le milieu a été concentré sous pression réduite, puis le résidu a été solubilisé dans 120 mL d'acide acétique. Ce mélange a été porté à 60°C et 2,60 g (46,6 mM) de fer ont été ajoutés. Le milieu a été chauffé à 70°C une nuit. Le milieu a été dilué dans l'eau et le précipité formé a été filtré et lavé trois fois à l'eau. Le solide a été solubilisé dans l'acétate d'éthyle, et la solution a été filtrée. Le filtrat obtenu a été concentré sous pression réduite. Le produit visé en titre a été obtenu sous la forme d'un solide marron (5,70 g, rendement = 95%). F = 142°C.

### Préparation 6: 1-(3-(1,1-diméthyléthyl)phénylsulfonyl)-5-trifluorométhyl-1H-pyrrolo[2,3-c]pyridine-2-carboxylate d'éthyle

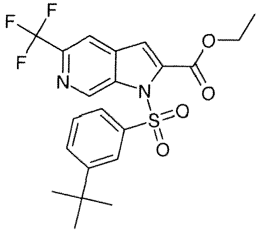

Sous Argon, on a mis en suspension 1,55 g (38,7 mM) d'hydrure de sodium à 60% dans l'huile dans 20,0 mL de THF. On a préparé une solution de 5,0 g (19,36 mM) de 5-Trifluorométhyl-1H-pyrrolo[2,3-c]pyridine-2-carboxylate d'éthyle (préparation 5) dans 20,0 mL de THF puis on a ajouté lentement cette solution au mélange réactionnel. Le milieu a été agité 1h à TA puis on a ajouté 6,76 g (29,0 mM) de chlorure de 3-tert-butyl benzènesulfonyle et on a agité ce mélange réactionnel à TA une nuit. Le milieu a été dilué dans l'eau puis extrait à l'acétate d'éthyle. La phase organique a été lavée par une solution d'eau saturée en sel, séchée sur sulfate de magnésium, filtrée et concentrée sous pression réduite. Le résidu d'évaporation a été purifié par chromatographie sur gel de silice à l'aide d'un éluant cyclohexane / acétate d'éthyle 90/10 (v/v). Les fractions contenant le produit attendu ont été réunies et concentrées à sec sous pression réduite. Le produit visé en titre a été obtenu sous la forme d'un solide blanc (7,25 g, rendement = 82%). F = 70°C.

### Préparation 7: [1-(3-(1,1-diméthyléthyl)phénylsulfonyl)-5-trifluorométhyl-1H-pyrrolo[2,3-c]pyridin-2-yl]-méthanol

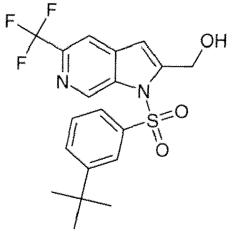

Sous Argon, on a préparé une solution de 7,25 g (15,9 mM) de 1-(3-(1,1-diméthyléthyl)phénylsulfonyl)-5-trifluorométhyl-1H-pyrrolo[2,3-c]pyridine-2-carboxylate d'éthyle (préparation 6) dans 140 mL de toluène. Cette solution a été refroidie à -78°C, puis 40 mL de DIBAL-H 1,0 mol/L dans le toluène ont été ajoutés goutte à goutte. Ce mélange réactionnel a été agité 3h à -70°C. Le milieu a été dilué dans 300 mL d'eau, puis on a ajouté une solution d'hydrogénocarbonate de sodium et de l'acétate d'éthyle. Ce mélange a été agité 2 jours à TA. La phase organique a été séchée sur sulfate de magnésium, filtrée et concentrée sous pression réduite. Le résidu d'évaporation a été purifié par chromatographie sur gel de silice à l'aide d'un gradient cyclohexane / acétate d'éthyle de 90/10 à 70/30 (v/v). Les fractions contenant le produit attendu ont été réunies et concentrées à sec sous pression réduite. Le produit visé en titre a été obtenu sous la forme d'un solide beige (4,08 g, rendement = 62%).
¹H RMN (300 MHz, DMSO) δ = 9,43 (s, 1H), 8,19 (s, 1H), 8,00 (d, 1H), 7,93 (d, 1H), 7,80 (d, 1H), 7,57 (t, 1H), 6,99 (s, 1H), 5,85 (m, 1H), 4,92 (d, 2H), 1,23 (s, 9H)

### Préparation 8: 2-Bromométhyl-1-(3-(1,1-diméthyléthyl)phénylsulfonyl)-5-trifluorométhyl-1H-pyrrolo[2,3-c]pyridine

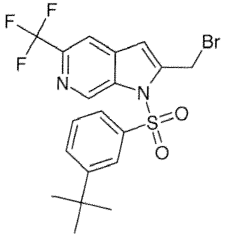

Ce composé a été obtenu sous la forme d'un solide blanc à partir du composé de la préparation 7, en suivant le mode opératoire décrit à la préparation 3 (rendement = 68%). F = 115°C.

### Exemple 2: Acide 3-[[1-[[3-diméthyléthyl)phényl]sulfonyl]-5-(thrifluorométhyl)-1H-pyrrolo[2,3-c]pyridin-2-yl]méthyl]-2-benzoïque

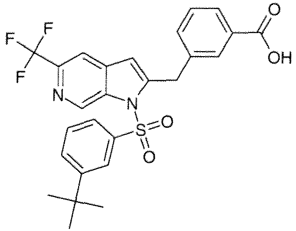

Ce composé a été obtenu sous la forme d'un solide blanc en suivant le mode opératoire décrit à l'exemple 1, à partir du composé de la préparation 8 et d'acide 3-(dihydroxyboryl)-benzoïque (rendement = 36%). F = 196°C.

### Exemple 3 : acide 2-chloro-4-[[1-[[3-(1,1-diméthyléthyl)phényl]sulfonyl]-5-(trifluorométhyl)-1H-pyrrolo[2,3-c]pyridin-2-yl]méthyl]-benzoïque

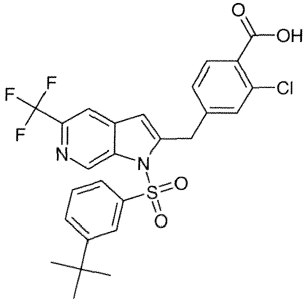

Ce composé a été obtenu sous la forme d'un solide blanc en suivant le mode opératoire décrit à l'exemple 1, à partir du composé de la préparation 8 et d'acide 4-(dihydroxyboryl)-2-chlorobenzoïque (rendement = 21%).
¹H RMN (500 MHz, DMSO) δ = 13,35 (s, 1H), 9,45 (s, 1H), 8,15 (s, 1H), 7,78 (m, 4H), 7,52 (t, 1H), 7,39 (s, 1H), 7,26 (d, 1H), 6,70 (s, 1H), 4,56 (s, 2H), 1,20 (s, 9H).

### Exemple 4: acide 5-[[1-[[3-(1,1-diméthyléthyl)phényl]sulfonyl]-5-(trifluorométhyl)-1H-pyrrolo[2,3-c]pyridin-2-yl]méthyl]-furane-3-carboxylique

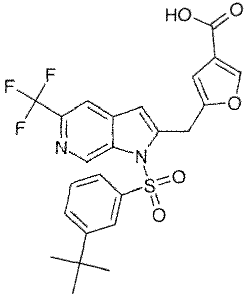

Ce composé a été obtenu sous la forme d'un solide marron en suivant le mode opératoire décrit à l'exemple 1, à partir du composé de la préparation 8 et d'acide 5-(dihydroxyboryl)-3-furanecarboxylique (rendement = 8%).
¹H RMN (500 MHz, DMSO) δ = 12,70 (s, 1H), 9,45 (s, 1H), 8,22 (s, 1H), 8,14 (s, 1H), 7,82 (m, 3H), 7,54 (t, 1H), 6,72 (s, 1H), 6,49 (s, 1H), 4,59 (s, 2H), 1,20 (s, 9H).

### Exemple 5: acide 5-[[1-[[3-(1,1-diméthyléthyl)phényl]sulfonyl]-5-(trifluorométhyl)-1H-pyrrolo[2,3-c]pyridin-2-yl]méthyl]-pyridine-3-carboxyligue

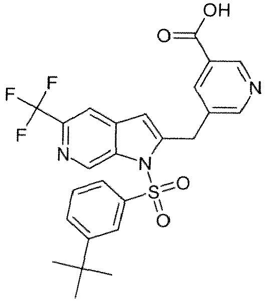

Ce composé a été obtenu sous la forme d'un solide noir-marron en suivant le mode opératoire décrit à l'exemple 1, à partir du composé de la préparation 8 et d'acide 5-(dihydroxyboryl)-3-pyridinecarboxylique (rendement = 7%).
¹H RMN (500 MHz, DMSO) δ = 13,50 (s, 1H), 9,45 (s, 1H), 8,96 (s, 1H), 8,73 (s, 1H), 8,14 (s, 1H), 8,07 (s, 1H), 7,77 (m, 3H), 7,51 (t, 1H), 6,72 (s, 1H), 4,63 (s, 2H), 1,19 (s, 9H).

### Exemple 6: acide 4-[[1-3[[1,1-diméthyléthyl)phényl)sulfonyl]-5-(trifluorométhyl)-1H-pyrrolo[2,3-c]pyridin-2-yl]méthyl]-thiophène-2-carboxylique

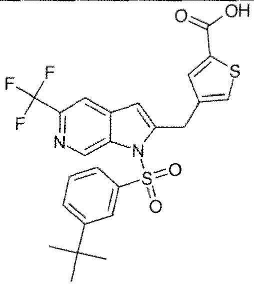

Ce composé a été obtenu sous la forme d'un solide marron en suivant le mode opératoire décrit à l'exemple 1, à partir du composé de la préparation 8 et d'acide 4-(dihydroxyboryl)-2-thiophènecarboxylique (rendement = 11%).
¹H RMN (500 MHz, DMSO) δ = 13,15 (s, 1H), 9,45 (s, 1H), 8,14 (s, 1H), 7,78 (m, 3H), 7,64 (s, 1H), 7,57 (s, 1H), 7,52 (t, 1H), 6,69 (s, 1H), 4,50 (s, 2H), 1,20 (s, 9H).

### Exemple 7: acide 5-[[-[[3-(1,1-diméthyléthyl)phényl]sulfonyl]-5-(trifluorométhyl)-1H-pyrrolo[2,3-c]pyridin-2-yliméthyl]-2-fluoro-benzaïque

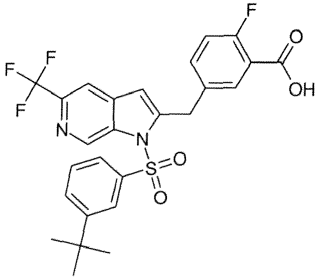

Ce composé a été obtenu sous la forme d'un solide brun en suivant le mode opératoire décrit à l'exemple 1, à partir du composé de la préparation 8 et d'acide 5-(dihydroxyboryl)-2-fluorobenzoïque (rendement = 18%).
¹H RMN (500 MHz, DMSO) δ = 13,25 (s, 1H), 9,40 (s, 1H), 8,13 (s, 1H), 7,77 (m, 3H), 7,69 (m, 1H), 7,51 (m, 2H), 7,27 (t, 1H), 6,62 (s, 1H), 4,53 (s, 2H), 1,20 (s, 9H).

### Préparation 9: [1-(3-(1,1-diméthyléthyl)phénylsulfonyl)-5-trifluorométhyl-1H-pyrrolo[3,2-b]pyridin-2-yl]-acétonitrile

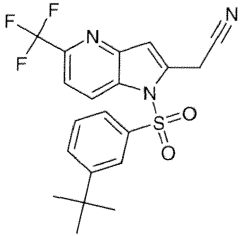

On a préparé une solution de 1,62 g (3,41 mM) de 2-Bromométhyl-1-(3-tert-butyl-benzènesulfonyl)-5-trifluorométhyl-1H-pyrrolo[3,2-b]pyridine (préparation 3) dans 13 ml de DCM. On a ajouté 3,24 mL d'eau, 0,11 g (0,34 mM) de bromure de tétrabutylammonium et 0,33g (5,11 mM) de cyanure de potassium. Ce mélange réactionnel a été agité à TA trois jours. On a ajouté au milieu 100 mL de solution de thiosulfate de sodium à 10 % et extrait par 100 mL de DCM trois fois. Les phases organiques ont été séchées sur sulfate de magnésium et réunies, puis concentrées sous pression réduite. Le résidu d'évaporation a été purifié par chromatographie sur gel de silice à l'aide d'un éluant cyclohexane / acétate d'éthyle 95/5 puis 90/10 (v/v). Les fractions contenant le produit attendu ont été réunies et concentrées à sec sous pression réduite. Le produit visé en titre a été obtenu sous la forme d'une résine jaune (280 mg, rendement = 19%).
¹H RMN (300 MHz, DMSO) δ = 8,67 (d, 1H), 7,92-7,50 (m, 4H), 7,20 (s, 1H), 4,68 (s, 2H), 1.22 (s, 9H).

### Préparation 10: 2-[1-(3-(1,1-diméthyléthyl)phénylsulfonyl)-5-trifluorométhyl-1H-pyrrolo[3,2-b]pyridin-2-yl]-thioacétamide

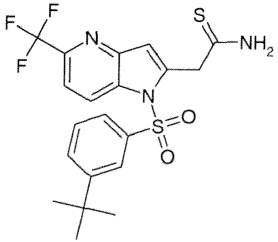

On a préparé une solution de 280 mg (0,66 mM) de [1-(3-(1,1-diméthyléthyl)phénylsulfonyl)-5-trifluorométhyl-1H-pyrrolo[3,2-b]pyridin-2-yl]-acétonitrile (préparation 9) dans 5,6 mL de THF et 11,2 mL d'eau. On a additionné 494 mg (2,66 mM) de dithiophosphate de diéthyle, puis on a agité ce mélange réactionnel une nuit à 100°C. On a ajouté 2000 mg (10,74 mM) de dithiophosphate de diéthyle et on a poursuivi l'agitation à 100°C pendant 4 h. On a dilué le milieu dans 100 mL d'eau puis on a extrait 4 fois par 50 mL de DCM. Les phases organiques ont été séchées sur sulfate de magnésium, réunies et concentrées sous pression réduite. Le résidu d'évaporation a été purifié par chromatographie sur gel de silice à l'aide d'un éluant cyclohexane / acétate d'éthyle 90/10 puis 80/20 et 70/30. Les fractions contenant le produit attendu ont été réunies et concentrées à sec sous pression réduite. Le produit visé en titre a été obtenu sous la forme d'un solide jaune (280,00 mg, rendement = 92%).
¹H RMN (300 MHz, DMSO) δ = 8,62 (d, 1H), 7,76-7,83 (m, 4H), 7,57 (t, 1H), 6,95 (s, 1H), 4,40 (s, 2H), 1,22 (s, 9H).

### Exemple 8: acide 2-[[1-[[3-(1,1-diméthyléthyl)phényl]sulfonyl]-5-(trifluorométhyl)-1H-pyrrolo[3,2-b]pyridin-2-yl]méthyl]-thiazole-4-carboxylique

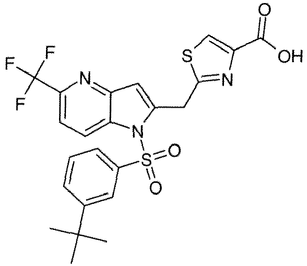

On a préparé une solution de 200 mg (0,44 mM) de 2-[1-(3-(1,1-diméthyléthyl)phénylsulfonyl)-5-trifluorométhyl-1H-pyrrolo[3,2-b]pyridin-2-yl]-thioacétamide (préparation 10) dans 2,0 mL d'éthanol, puis on a ajouté 110 mg (0,66 mM) d'acide bromopyruvique. Ce mélange réactionnel a été agité 24h à TA. On a ajouté au milieu 150 mL d'acétate d'éthyle puis on a lavé trois fois par 50 mL de carbonate de sodium à 10% puis par 50 mL d'acide chlorhydrique 0,2 M. La phase organique a été séchée sur sulfate de magnésium, puis filtrée et concentrée sous pression réduite. Le produit visé en titre a été obtenu sous la forme d'un solide blanc (94 mg, rendement = 41 %). F = 180°C.

### Préparation 11: N-(3-iodo-5-trifluorométhyl-pyridin-2-yl)-4-(1-méthyléthyl)-benzènesulfonamide

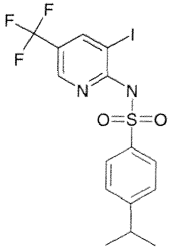

On a obtenu ce composé sous la forme d'un solide jaune, en suivant le mode opératoire décrit à la préparation 1, à partir de 3-iodo-5-trifluorométhyl-pyridin-2-ylamine et de chlorure de 4-isopropyl-benzènesulfonyle (rendement = 52%). F = 124°C.

### Préparation 12 : 4-Prop-2-ynyl-benzoate de méthyle

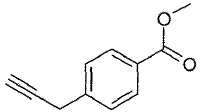

On a préparé une solution de 5,97 g (31,4 mM) de 4-(1-Hydroxy-prop-2-ynyl)-benzoate de méthyle dans 40 mL de DCM. On a ajouté 20 mL (62,9 mM) d'acide trifluoroacétique puis 10,0 mL (62,9 mM) de triéthylsilane. Ce mélange réactionnel a été agité à TA 7 jours. Le milieu a été concentré sous pression réduite. Le résidu d'évaporation a été purifié par chromatographie sur gel de silice à l'aide d'un éluant cyclohexane / acétate d'éthyle 95/5 (v/v). Les fractions contenant le produit attendu ont été réunies et concentrées à sec sous pression réduite. Le produit visé en titre a été obtenu sous la forme d'un solide blanc (3,23 g, rendement = 61%). F = 78°C.

### Exemple 9: 4-[1-(4-(1-méthyléthyl)phénylsulfonyl)-5-trifluorométhyl-1H-pyrrolo[2,3-b]pyridin-2-ylméthyl]-benzoate de méthyle

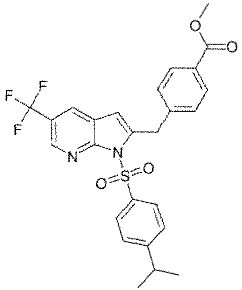

On a obtenu ce composé sous la forme d'un solide beige en suivant le mode opératoire décrit à la préparation 2, à partir des composés des préparations 11 et 12 (rendement = 56%). F = 123°C.

### Exemple 10: Acide 4-[[1-[[3-(1-méthyléthyl)phényl]sulfonyl]-5-(trifluorométhyl)-1H-pyrrolo[2,3-b]pyridin-2-yl]méthyl]-benzoïque

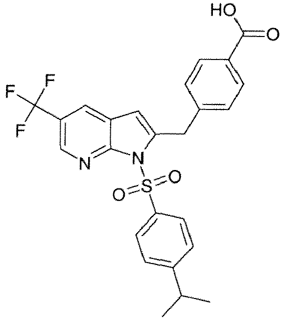

On a préparé une solution de 140 mg (0,27 mM) de 4-[1-(4-(1-méthyléthyl)phénylsulfonyl)-5-trifluorométhyl-1H-pyrrolo[2,3-b]pyridin-2-ylméthyl)-benzoate de méthyle (exemple 9) dans 16,0 mL de THF et 4,0 mL d'eau. On a additionné 13,65 mg (0,33 mM) de lithine, et ce mélange réactionnel a été agité à TA pendant 22 h. Le milieu a été dilué dans l'eau puis acidifié avec de l'acide chlorhydrique concentré, puis extrait au DCM 2 fois. La phase organique a été séchée sur sulfate de magnésium, filtrée puis concentrée sous pression réduite. Le résidu d'évaporation a été purifié par chromatographie liquide avec détection par UV (LC-UV) (sur colonne SunFire® C18) en éluant par un mélange eau / acétonitrile/ 0,1 % TFA. Les fractions contenant le produit attendu ont été réunies et concentrées à sec sous pression réduite. Le produit visé en titre a été obtenu sous la forme d'un solide blanc (113 mg, rendement = 83%). F = 218°C.

### Préparation 13: 4-Iodo-6-trifluorométhyl-pyridin-3-ylamine

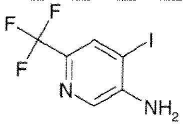

### a) (6-Trifluorométhyl-pyridin-3-yl)-carbamate de tert-butyle

On a préparé une solution de 5,0 g (30,84 mM) de 3-amino-6-(trifluorométhyl)pyridine et de 6,73 g (30,84 mM) de dicarbonate de di-tert-butyle dans 30 mL de 1,4-dioxane. Ce mélange réactionnel a été agité sous reflux pendant 19h. 8,08g (37,01 mM) de dicarbonate de di-tert-butyle ont été ajoutés et le milieu a été agité sous reflux pendant 24 h. 6,73 g (30,84 mM) de dicarbonate de di-tert-butyle ont été ajoutés et le milieu a été agité sous reflux pendant 24 h. Le milieu a été concentré sous pression réduite et le résidu d'évaporation a été purifié par chromatographie sur gel de silice à l'aide d'un éluant cyclohexane / acétate d'éthyle 90/10 (v/v). Les fractions contenant le produit attendu ont été réunies et concentrées à sec sous pression réduite. Le produit protégé a été obtenu sous la forme d'un solide beige (10,9 g, rendement = 100%). F = 117°C.

### b) (4-Iodo-6-trifluorométhyl-pyridin-3-yl)-carbamate de tert-butyle

On a préparé une solution de 10,80 g (41,19 mM) de (6-Trifluorométhyl-pyridin-3-yl)-carbamate de tert-butyle dans 300 mL d'éther diéthylique. On a ajouté 15,5 mL de N,N,N',N' tétraméthyléthylène diamine. Ce mélange réactionnel a été refroidi à -78°C avec un bain acétone-carboglace, puis on a ajouté goutte à goutte en 5 min, 64,3 mL (103 mM) de N-butyllithium 1,60M dans l'hexane. Le milieu a été agité pendant 30 min à -10°C puis refroidi à -78°C. On a ajouté rapidement une solution de 13,59 g (53,5 mM) d'iode dans 60,0 mL de THF. On a laissé la température remonter à TA, et le mélange réactionnel a été agité à TA pendant 16 h. Le milieu a été hydrolysé avec 200 ml d'eau puis avec 400 ml d'une solution saturée de bisulfite de sodium. On a extrait ensuite avec de l'éther. La phase organique a été séchée sur sulfate de magnésium, filtrée et concentrée sous pression réduite. Le résidu d'évaporation a été purifié par chromatographie sur gel de silice à l'aide d'un éluant cyclohexane / acétate d'éthyle 100/0 puis 95/5 (v/v). Les fractions contenant le produit attendu ont été réunies et concentrées à sec sous pression réduite. Le produit iodé a été obtenu sous la forme d'une huile jaune (5,04 g, rendement = 37%).
¹H RMN (250 MHz, DMSO) δ = 9,05 (s large, 1H), 8,62 (s, 1H), 8,36 (s, 1H), 1,40 (s, 9H).

### c) 4-iodo-6-trifluorométhyl-pyridin-3-ylamine

On a préparé une solution de 5,04 g (13 mM) de (4-Iodo-6-trifluorométhyl-pyridin-3-yl)-carbamate de tert-butyle dans 100 mL de DCM. On a ajouté à cette solution 10 mL d'acide trifluoroacétique. Ce mélange réactionnel a été agité à TA pendant 18 h. Le milieu a été dilué dans de l'eau puis extrait au DCM. La phase organique a été séchée sur sulfate de magnésium, filtrée et concentrée sous pression réduite. Le résidu d'évaporation a été purifié par chromatographie sur gel de silice à l'aide d'un éluant cyclohexane / acétate d'éthyle 100/0 puis 95/5 et 90/10 (v/v). Les fractions contenant le produit attendu ont été réunies et concentrées à sec sous pression réduite. Le produit visé en titre a été obtenu sous la forme d'un solide beige (620,00 mg, rendement = 16 %). F = 116°C.

### Préparation 14 : N-(2-Iodo-4-trifluorométhyl-pyridin-3-yl)-(3,4-dihydro-4-méthyl-2H-1,4-benzoxazin-6-yl)-sulfonamide

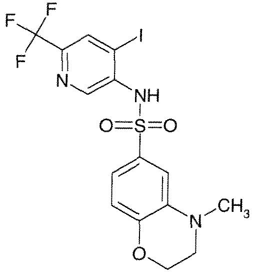

On a obtenu ce composé sous la forme d'une huile jaune en suivant le mode opératoire décrit à la préparation 1, à partir de 4-iodo-6-trifluorométhyl-pyridin-3-ylamine (préparation 13) et de chlorure de 3,4-dihydro-4-méthyl-2H-1,4-benzoxazin-6-yl-sulfonyle (rendement = 68%).
¹H RMN (300 MHz, DMSO) δ = 10,14 (s, 1H), 8,35 (s, 1H), 8,26 (s, 1H), 6,95 (m, 2H), 6,80 (d, 1H), 4,29 (t, 2H), 3,29 (t, 2H), 2,80 (s, 3H).

### Exemple 11 : 4-[[1-[(3,4-dihydro-4-méthyl-2H-1,4-benzoxazin-6-yl)sulfonyl]-5-(trifluorométhyl)-1H-pyrrolo[2,3-c]pyridin-2-yl]méthyl]-benzoate de méthyle

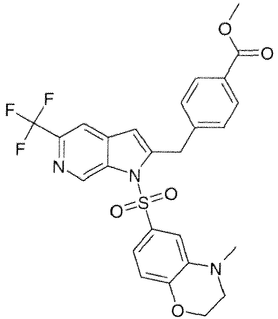

On a obtenu ce composé sous la forme d'un solide blanc en suivant le mode opératoire décrit à la préparation 2, à partir des composés des préparations 12 et 14 (rendement = 25%). F = 207°C.

### Exemple 12 : acide 4-[[1-[(3,4-dihydro-4-méthyl-2H-1,4-benzoxazin-6-yl)sulfonyl]-5-(trifluorométhyl)-1H-pyrrolo(2,3-c]pyridin-2-yl]méthyl]-benzoïque

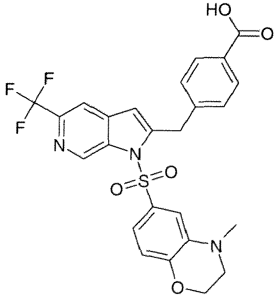

On a obtenu ce composé sous la forme d'un solide blanc en suivant le mode opératoire décrit à l'exemple 10, à partir du composé de l'exemple 11 (rendement = 43%). F = 192°C.

### Préparation 15: N-(4-trifluorométhyl-6-iodo-pyridin-3-yl)-4-(1-méthyléthyl)-benzènesulfonamide

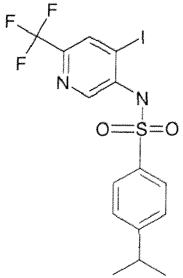

On a obtenu ce composé sous la forme d'un solide beige, en suivant le mode opératoire décrit à la préparation 1, à partir de 4-iodo-6-trifluorométhyl-pyridin-3-ylamine (préparation 13) et de chlorure de 4-(1-méthyléthyl)-benzènesulfonyle (rendement = 95%). F = 156°C.

### Exemple 13: 4-[1-(4-(1-méthyléthyl)phénylsulfonyl)-5-trifluorométhyl-1H-pyrrolo[2,3-c]pyridin-2-ylméthyl]-benzoate de méthyle

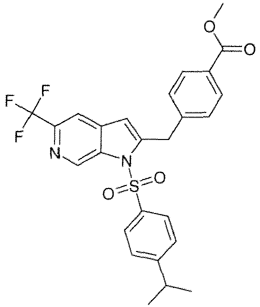

On a obtenu ce composé sous la forme d'un solide beige en suivant le mode opératoire décrit à la préparation 2, à partir des composés des préparations 12 et 15 (rendement = 33%). F = 160°C

### Exemple 14: acide 4-[[1-[[1-(4-(1-méthyléthyl)phényl]sulfonyl]-5-(trifluorométhyl)-1H-pyrrolo[2,3-c]pyridin-2-yl]méthyl]-benzoïque

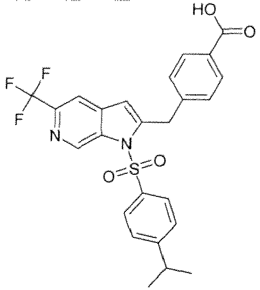

On a obtenu ce composé sous la forme d'un solide blanc en suivant le mode opératoire décrit à l'exemple 10, à partir du composé de l'exemple 13 (rendement = 24%). F= 218°C.

### Préparation 16: N-(2-Iodo-6-trifluorométhyl-pyridin-3-yl)-4-(1-méthyléthyl)-benzènesulfonamide

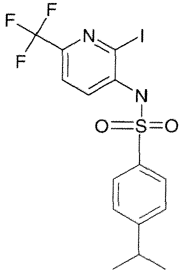

On a obtenu ce composé sous la forme d'un solide jaune pâle, en suivant le mode opératoire décrit à la préparation 1, à partir de 2-iodo-6-trifluorométhyl-pyridin-3-ylamine et de chlorure de 4-isopropyl-benzènesulfonyle (rendement = 98%).
¹H RMN (300 MHz, DMSO) δ = 10,35 (s, 1H), 7,87 (d, 1H), 7,72 (dd, 2H), 7,62 (d, 1H), 7,48 (d, 2H), 2,98 (m, 1H), 1,20 (d, 6H).

### Exemple 15: 4-[1-(4-(1-méthyléthyl)phénylsulfonyl)-5-trifluorométhyl-1H-pyrrolo[3,2-b]pyridin-2-ylméthyl]-benzoate de méthyle

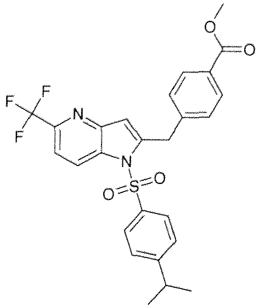

On a obtenu ce composé sous la forme d'un solide beige en suivant le mode opératoire décrit à la préparation 2, à partir des composés des préparations 12 et 16 (rendement = 15%). F = 98,5°C.

### Exemple 16: acide 4-[1-(4-(1-méthyléthyl)phénylsulfonyl)-5-trifluorométhyl-1H-pyrrolo[3,2-b]pyridin-2-ylméthyl]-benzoïque

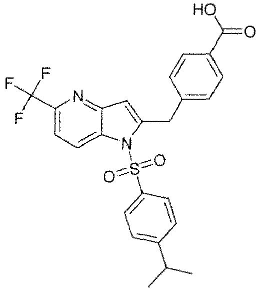

On a obtenu ce composé sous la forme d'un solide blanc en suivant le mode opératoire décrit à l'exemple 10, à partir du composé de l'exemple 15 (rendement = 14%. ¹H RMN (500 MHz, DMSO) δ = 12,89 (s, 1H), 8,66 (d, 1H), 7,83 (d, 3H), 7,72 (d, 2H), 7,36 (d, 2H), 7,30 (d, 2H), 6,84 (s, 1H), 4,58 (s, 2H), 2,91 (m,1H), 1,13 (d, 6H).

### Préparation 17 : N-(3-Iodo-5-trifluorométhyl-pyridin-2-yl)-(3,4-dihydro-4-méthyl-2H-1,4-benzoxazin-6-yl)-sulfonamide

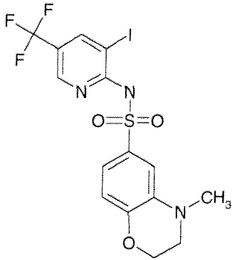

On a obtenu ce composé sous la forme d'une huile marron en suivant le mode opératoire décrit à la préparation 1, à partir de 3-iodo-5-trifluorométhyl-pyridin-2-ylamine et de chlorure de 3,4-dihydro-4-méthyl-2H-1,4-benzoxazin-6-yl-sulfonyle (rendement = 66%).
¹H RMN (300 MHz, DMSO) δ = 10,43 (s, 1H), 8,54 (s, 2H), 7,33 (s, 1H), 7,24 (d, 1H), 6,80 (d, 1H), 4,28 (t, 2H), 3,28 (t, 2H), 2,88 (s, 3H).

### Exemple 17: 4-[[1-[(3,4-dihydro-4-méthyl-2H-1,4-benzoxazin-6-yl)sulfonyl]-5-(trifluorométhyl)-1H-pyrrolo[2,3-b]pyridin-2-yl]méthyl]-benzoate de méthyle

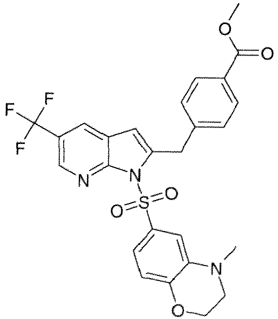

On a obtenu ce composé sous la forme d'un solide orange en suivant le mode opératoire décrit à la préparation 2, à partir des composés des préparations 12 et 17 (rendement = 30%). F = 202°C.

### Exemple 18 : acide 4-[[1-[(3,4-dihydro-4-méthyl-2H-1,4-benzoxazin-6-yl)sulfonyl]-5-(trifluorométhyl)-1H-pyrrolo[2,3-b]pyridin-2-yl]méthyl]-benzoïque

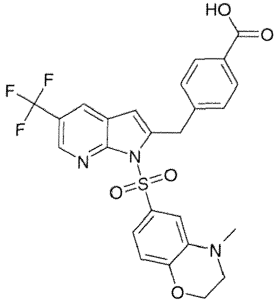

On a obtenu ce composé sous la forme d'un solide blanc en suivant le mode opératoire décrit à l'exemple 10, à partir du composé de l'exemple 17 (rendement = 21 %). F = 234°C.

### Préparation 18: N-(6-Chloro-4-iodo-pyridin-3-yl)-4-(1-méthyléthyl)-benzène-sulfonamide

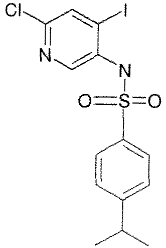

On a obtenu ce composé sous la forme d'un solide orange, en suivant le mode opératoire décrit à la préparation 1, à partir de 6-chloro-4-iodo-pyridin-3-ylamine et de chlorure de 4-isopropyl-benzènesulfonyle (rendement = 97%). F = 160°C.

### Exemple 19: 4-[1-(4-(1-méthyléthyl)phénylsulfonyl)-5-chloro-1H-pyrrolo[2,3-c]pyridin-2-ylméthyl]-benzoate de méthyle

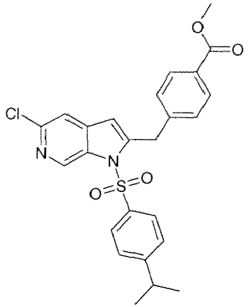

On a obtenu ce composé sous la forme d'un solide marron en suivant le mode opératoire décrit à la préparation 2, à partir des composés des préparations 12 et 18 (rendement = 44%). F = 123°C.

### Exemple 20 : acide 4-[[5-chloro-1-[[4-(1-méthyléthyl)phényl]sulfonyl]-1H-pyrrolo[2,3-c]pyridin-2-yl]méthyl]-benzoïque

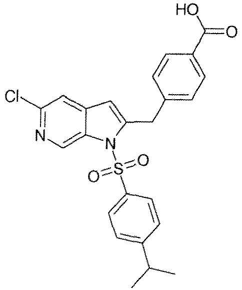

On a obtenu ce composé sous la forme d'un solide beige en suivant le mode opératoire décrit à l'exemple 10, à partir du composé de l'exemple 19 (rendement = 13%). F = 228°C.

### Exemple 21: 4-[1-(3-(1,1-diméthyléthyl)phénylsulfonyl)-5-trifluorométhyl-1H-pyrrolo[3,2-b]pyridin-2-ylméthyl]-benzoate de méthyle

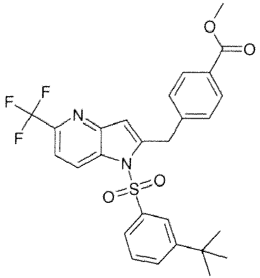

On a obtenu ce composé sous la forme d'une pâte orange en suivant le mode opératoire décrit à la préparation 2, à partir des composés des préparations 1 et 12 (rendement = 4%).
¹H RMN (300 MHz, DMSO) δ = 8,67 (d, 1H), 7,86 (m, 3H), 7,73 (d, 1H), 7,71 (d, 1H), 7,62 (d, 1H), 7,46 (t, 1H), 7,36 (d, 2H), 6,85 (s, 1H), 4,60 (s, 2H), 3,45 (s, 3H), 1,18 (s, 9H).

### Exemple 22 : acide 4-[[1-[[3-(1,1-diméthyléthyl)phényl]sulfonyl]-5-(trifluorométhyl)-1H-pyrrolo(3,2-b]pyridin-2-yl]méthyl]-benzoïque

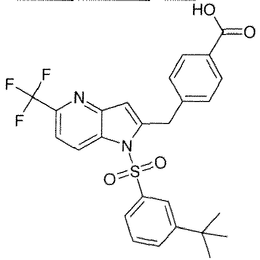

On a obtenu ce composé sous la forme d'un solide blanc en suivant le mode opératoire décrit à l'exemple 10, à partir du composé de l'exemple 21 (rendement = 33%). F = 240°C.

### Préparation 19: N-(3-Iodo-5-trifluorométhyl-pyridin-2-yl)-3-(1-méthyléthyl)-benzènesulfonamide

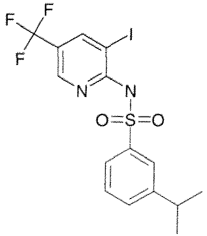

On a obtenu ce composé sous la forme d'une huile jaune en suivant le mode opératoire décrit à la préparation 1, à partir de 3-Iodo-5-trifluorométhyl-pyridin-2-ylamine et de chlorure de 3-isopropyl-benzènesulfonyle (rendement = 84%).
¹H RMN (300 MHz, DMSO) δ = 10,70 (s, 1H), 8,55 (s, 1H), 8,44 (s, 1H), 7,91 (s, 1H), 7,62 (d, 1H), 7,51 (m, 2H), 3,00 (m, 1H), 1,23 (d, 6H).

### Exemple 23 : 4-(Hydroxy-[1-(3-(1-méthyléthyl)phénylsulfonyl)-5-trifluorométhyl-1H-pyrrolo[2,3-b]pyridin-2-yl]-méthyl}-benzoate de méthyle

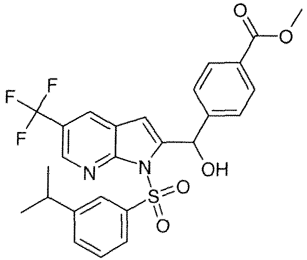

On a obtenu ce composé sous la forme d'une huile jaune en suivant le mode opératoire décrit à la préparation 2, à partir du composé de la préparation 19 et de 4-(1-Hydroxy-prop-2-ynyl)-benzoate de méthyle (rendement = 60%).
¹H RMN (300 MHz, DMSO) δ = 8,72 (s, 1H), 8,49 (s, 1H), 7,98 (d, 2H), 7,79 (d, 1H), 7,54 (m, 4H), 7,43 (t, 1H), 6,86 (s, 1H), 6,57 (m, 2H), 3,87 (s,3H), 2,74 (m, 1H), 1,08 (dd, 6H).

### Exemple 24: 4-[1-(3-(1-méthyléthyl)phénylsulfonyl)-5-trifluorométhyl-1H-pyrrolo[2,3-b]pyridin-2-ylméthyl]-benzoate de méthyle

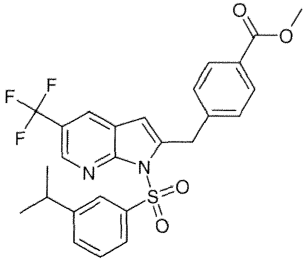

On a préparé une solution de 220 mg (0,41 mM) de 4-{Hydroxy-[1-(3-isopropyl-benzènesulfonyl)-5-trifluorométhyl-1H-pyrrolo[2,3-b]pyridin-2-yl]-méthyl}-benzoate de méthyle (exemple 23) dans 10 mL de DCM. Cette solution a été refroidie à 0°C avec un bain de glace, puis on a ajouté 4,19 mL (33,05 mM) de diéthyléthérate de trifluorure de bore (4,19 ml; 33,05 mM; 80,00 eq,) et 2,0 mL (12,39 mM) de triéthylsilane. Ce mélange réactionnel a été agité à TA toute la nuit. Le milieu a été hydrolysé lentement avec une solution saturée de carbonate de sodium, puis extrait au DCM. La phase organique a été lavée à l'eau puis séchée sur sulfate de magnésium, filtrée et concentrée sous pression réduite. Le résidu d'évaporation a été purifié par chromatographie sur gel de silice à l'aide d'un éluant cyclohexane / acétate d'éthyle 95/5 puis 90/10 (v/v). Les fractions contenant le produit attendu ont été réunies et concentrées à sec sous pression réduite. Le produit obtenu a été lavé avec du cyclohexane puis filtré sur filtre Whatman. Le produit visé en titre a été obtenu sous la forme d'un solide blanc (90 mg, rendement = 42%). F = 110°C.

### Exemple 25 : acide 4-[[1-(3-(1-méthyléthyl)phényl)sulfonyl]-5-(trifluorométhyl)-1H-pyrrolo(2,3-b]pyridin-2-yl]méthyl]-benzöique

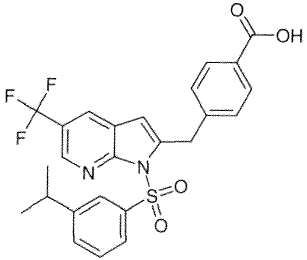

On a obtenu ce composé sous la forme d'un solide blanc en suivant le mode opératoire décrit à l'exemple 10, à partir du composé de l'exemple 24 (rendement = 17%). F = 203°C.

### Préparation 20: N-(3-Iodo-5-trifluorométhyl-pyridin-2-yl)-3-(1,1-diméthyléthyl)-benzènesulfonamide

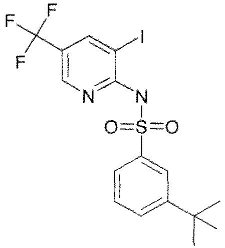

On a obtenu ce composé sous la forme d'un solide beige en suivant le mode opératoire décrit à la préparation 1, à partir de 3-Iodo-5-tritluorométhyl-pyridin-2-ylamine et de chlorure de 3-tert-butyl-benzènesulfonyle (rendement = 72%). F = 135°C.

### Exemple 26 : 4-(Hydroxy-(1-(3-(1,1-diméthyléthyl)phénylsulfonyl)-5-trifluorométhyl-1H-pyrrolo[2,3-b]pyridin-2-yl]-méthyl}-benzoate de méthyle

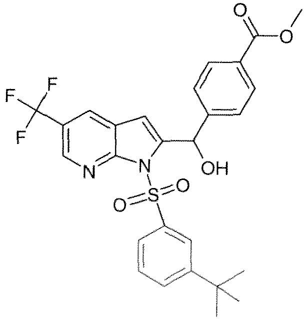

On a obtenu ce composé sous la forme d'une huile jaune en suivant le mode opératoire décrit à la préparation 2, à partir du composé de la préparation 20 et de 4-(1-Hydroxy-prop-2-ynyl)-benzoate de méthyle (rendement = 92%).
¹H RMN (300 MHz, DMSO) δ = 8,73 (d, 1H), 8,47 (d, 1H). 8,12 (d, 1H), 7,97 (d, 2H), 7,65-7,73 (m, 2H), 7,52 (d, 2H), 7,42 (t, 1H), 6,75 (s, 1H), 6,57 (m, 2H), 3,87 (s, 3H), 1.21 (s, 9H).

### Exemple 27: 4-(1-(3-(1,1-diméthyléthyl)phénylsulfonyl)-5-trifluorométhyl-1H-pyrrolo[2,3-b]pyridin-2-ylméthyl]-benzoate de méthyle

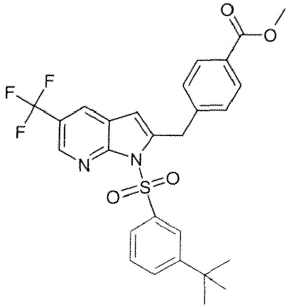

On a obtenu ce composé sous la forme d'une huile jaune en suivant le mode opératoire décrit dans l'exemple 24, à partir du composé de l'exemple 26 (rendement = 92%). Ce composé est utilisé tel quel dans l'exemple suivant.

### Exemple 28 : acide 4-[[1-[[3-(1,1-diméthyléthyl)phényl]sulfonyl]-5-(trifluorométhyl)-1H-pyrrolo[2,3-b]pyridin-2-yl]méthyl]-benzoïque

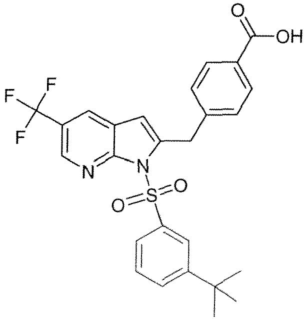

On a obtenu ce composé sous la forme d'un solide blanc en suivant le mode opératoire décrit à l'exemple 10, à partir du composé de l'exemple 27 (rendement = 22%). F = 209°C.

### Préparation 21: N-(5-Chloro-3-iodo-pyridin-2-yl)-4-(1-méthyléthyl)-benzène-sulfonamide

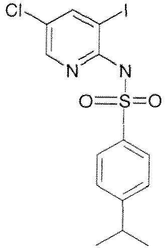

On a obtenu ce composé sous la forme d'un solide orange, en suivant le mode opératoire décrit à la préparation 1, à partir de -5-chloro-3-iodo-pyridin-3-ylamine et de chlorure de 4-isopropyl-benzènesulfonyle (rendement = 15%).
¹H RMN (250 MHz, DMSO) δ = 10,42 (s, 1H), 8,40 (d, 1H), 8,23 (s, 1H), 7,91 (d, 2H), 7,44 (d, 2H), 2,97(m, 1H), 1,21 (d, 6H).

### Exemple 29: 4-{Hydroxy-[5-chloro-1-(4-(1-méthyléthyl)phénylsulfonyl)-1H-pyrrolo[2,3-b]pyridin-2-yl]-méthyl}-benzoate de méthyle

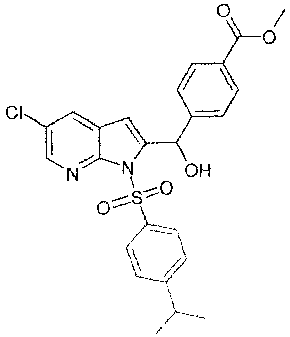

On a obtenu ce composé sous la forme d'un solide jaune en suivant le mode opératoire décrit à la préparation 2, à partir du composé de la préparation 21 et de 4-(1-Hydroxy-prop-2-ynyl)-benzoate de méthyle (rendement = 58%). F = 77°C.

### Exemple 30: 4-[5-Chloro-1-(4-(1-méthyléthyl)phénylsulfonyl)-1H-pyrrolo[2,3-b]pyridin-2-ylméthyl]-benzoate de méthyle

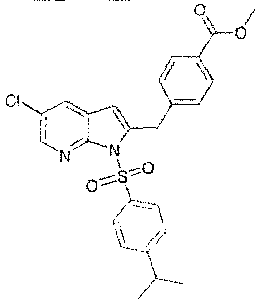

On a obtenu ce composé sous la forme d'un solide beige en suivant le mode opératoire décrit dans l'exemple 24, à partir du composé de l'exemple 29 (rendement = 77%). F = 186°C.

### Exemple 31 : acide 4-[[5-chloro-1-[[4-(1-méthyléthyl)phényl]sulfonyl]-1H-pyrrolo-[2,3-b]pyridin-2-yl]méthyl]-benzoïque

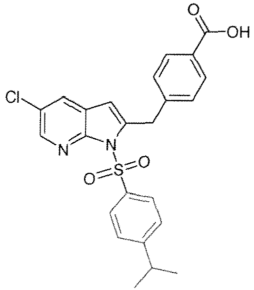

On a obtenu ce composé sous la forme d'un solide blanc en suivant le mode opératoire décrit à l'exemple 10, à partir du composé de l'exemple 30 (rendement = 100%). F = 227°C.

### Préparation 22 : acide 5- (1-hydroxy-prop-2-ynyl)-thiophène-2-carboxylique

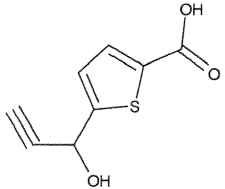

On a préparé une solution de 2,342 g (15,0 mM) d'acide 5-formyl-2-thiophènecarboxylique dans 23,4 mL de THF distillé. On a additionné goutte à goutte 60,0 mL d'une solution de bromure d'éthylmagnésium (0,50 mol/l 30,0 mM). Le milieu a été agité à température ambiante pendant deux heures. Puis il a été versé sur un mélange de 200 mL de glace et 70 mL d'HCl M et extrait par 100 mL de DCM puis 50 mL deux fois. Les phases organiques ont été séchées sur sulfate de magnésium et réunies, puis concentrées sous pression réduite. Le produit visé en titre a été obtenu sous la forme d'un solide beige (2,6 g, rendement = 100%). F= 128°C.

### Exemple 32: acide 5-{Hydroxy-[1-(3-(1,1-diméthyléthyl)phénylsulfonyl)-5-trifluorométhyl-1H-pyrrolo[3,2-b]pyridin-2-yl]-méthyl}-thiophène-2-carboxylique

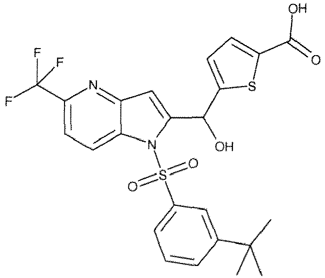

On a obtenu ce composé sous la forme d'un solide blanc en suivant le mode opératoire décrit à la préparation 2, à partir du composé de la préparation 1 et de la préparation 22 (rendement = 5%). F= 100°C

### Exemple 33 : 5-[[1-[[3-(1,1-diméthyléthyl)phényl]sulfonyl]-5-(trifluorométhyl)-1H-pyrrolo[3,2-b]pyridin-2-yl]-fluorométhyl]-N,N-diéthyl-2-thiophénecarboxamide

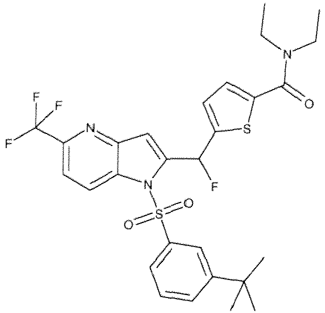

On a préparé une solution de 160 mg (0,30 mM) d'acide 5-{hydroxy-[1-(3-(1,1-diméthyléthyl)phénylsulfonyl)-5-trifluorométhyl-1H-pyrrolo[3,2-b]pyridin-2-yl]-méthyl} - thiophène-2-carboxylique (exemple 32) dans 10mL de DCM. On a ajouté 238,55 µl de (n-Bu)₃N (1,0 mM) puis le milieu a été agité 5 minutes. Le solvant et l'excès de réactif ont été évaporés. Le solide amorphe obtenu a été redissous dans 10 mL de DCM. Cette solution a été refroidie à -78 °C sous atmosphère d'argon. 238,75 µl de DAST (1,78 mM) ont été additionnés au goutte à goutte. Le milieu a été agité une heure à - 78 °C, puis on l'a laissé remonter à température ambiante et on l'a de nouveau agité 1 heure. On a additionné 100 mL de solution de carbonate de sodium, puis 40 mL de solution de HCl à 10 M et on a extrait par 100 mL de DCM puis deux fois par 50 mL. Les phases organiques ont été séchées sur du sulfate de magnésium, réunies et le solvant évaporé. Le résidu d'évaporation a été purifié par chromatographie liquide LC-MS semi-préparative (sur colonne SunFire® C18) en éluant par un mélange eau / acétonitrile. Les fractions contenant le produit attendu ont été réunies et concentrées à sec. Le produit visé en titre a été obtenu sous la forme d'un solide marron (19,85 mg, rendement = 11%).
¹H RMN (500 MHz, DMSO) δ = 8,75 (d, 1H), 7,93 (d, 1H), 7,81 (t, 1H), 7,76 (d, 1H), 7,72 (d, 1H), 7,69 (d, 1H), 7,49 (t, 1H), 7.32 (dd, 2H), 7,30 (d, 1H), 3,46 (s large , 4H), 1,18 (m, 15H).

### Exemple 34 : acide 5-{1-(3-(1,1-diméthyléthyl)phénylsulfonyl)-5-trifluorométhyl-1H-pyrrolo[3,2-b]pyridine-2-carbonyl}-thiophène-2-carboxylique

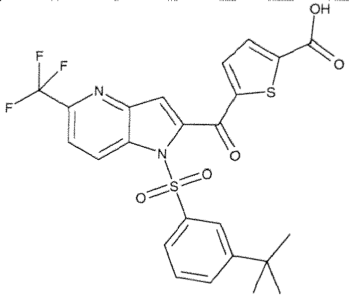

On a préparé une solution de 110,00 mg (0,20 mM) d'acide 5-{Hydroxy-[1-(3-(1,1-diméthyléthyl)phénylsulfonyl)-5-trifluorométhyl-1H-pyrrolo[3,2-b]pyridin-2-yl]-méthyl}-thiophène-2-carboxylique (exemple 32) dans 5 mL d'acétonitrile et de 5 mL d'eau. On a additionné 260,95 mg (1,84 mM) d'hydrogénophosphate de sodium. Le milieu a été agité 15 minutes à température ambiante. On a additionné 110,83 mg de chlorite de sodium (1,22 mM), 0,24 mL (13,0 g/1; 0,04 mM) d'une solution d'hypochlorite de sodium et 3,19 mg de 2,2,6,6-Tétramethylpipéridine-1-oxyl, (0,02 mM). Le milieu a été agité 5 heures à température ambiante. On a additionné 25 mL de thiosulfate à 10 %, 50 ml d'eau et 25 mL d'HCl M puis on a extrait quatre fois par 50 mL de DCM. Les phases organiques ont été séchées sur sulfate de magnésium, réunies et le solvant évaporé. Le résidu d'évaporation a été purifié par chromatographie liquide LC-MS semi-préparative (sur colonne SunFire® C18) en éluant par un mélange eau / acétonitrile. Les fractions contenant le produit attendu ont été réunies et concentrées à sec. Le produit visé en titre a été obtenu sous la forme d'un solide blanc (12 mg, rendement =11%). F=108°C.

### Exemple 35: acide 4-[[5-chloro-1-[[4-(1-méthyléthyl]phényl]sulfonyl]-1H-pyrrolo-[2,3-b]pyridin-2-yl]méthyl]-benzoïque, sel de sodium.

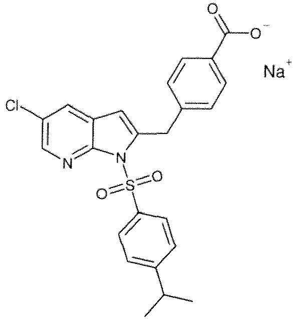

On a préparé une solution de 50 mg (0,11 mM) d'acide 4-[[5-chloro-1-[[4-(1-méthyléthyl)phényl)sulfonyl)-1H-pyrrolo-[2,3-b]pyridin-2-yl]méthyl]-benzoïque (exemple 31) dans 2,5 mL de THF. On a ajouté 0,21 ml d'hydroxyde de sodium (0,50 mol/1 ; 0,11 mM). Le milieu réactionnel a été agité une nuit à température ambiante, puis évaporé sous vide pour obtenir 31 mg de sel de sodium sous la forme d'un solide blanc (rendement = 63 %). F=218°C.
¹H RMN (400 MHz, DMSO) δ = 8,32 (d, 1H), 8,05 (d, 1H), 7,82 (d, 2H), 7,79 (d, 2H), 7,36 (d, 2H), 7,15 (d, 2H), 6,30 (s, 1H), 4,47 (s, 2H), 2,91 (m, 1H), 1,15 (d, 6H).

### Exemple 36: acide 4-[[5-chloro-1-[[4-(1-méthyléthyl)phényl)sulfonyl]- 1H-pyrrolo-[2,3-b]pyridin-2-yl]méthyl]-benzoïque, sel de pipérazine.

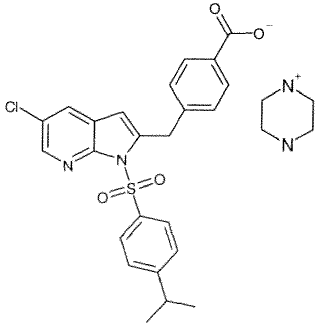

On a préparé une solution de 46,20 mg (0,10 mM) d'acide 4-[[5-chloro-1-[[4-(1-méthyléthyl)phényl]sulfonyl]-1H-pyrrolo-[2,3-b]pyridin-2-yl]méthyl]-benzoïque (exemple 31) dans 4 mL de THF. On a ajouté 8.49 mg de pipérazine (0,10 mM). Le milieu réactionnel a été agité 24 heures à température ambiante, puis évaporé sous vide pour obtenir 40 mg de sel de pipérazine sous la forme d'un solide blanc (rendement = 80 %). F= 105°C.
¹H RMN (400 MHz, DMSO) δ = 8,34 (d, 1H), 8,08 (d, 1H), 7,86 (d, 2H), 7,79 (d, 2H), 7,36 (d, 2H), 7,27 (d, 2H), 6,37 (s, 1H), 4,53 (s, 2H), 2,91 (m, 1H), 2,79 (s, 8H), 1,14 (d, 6H).

### Préparation 23: 1-(3-(1,1-diméthyléthyl)phénylsulfonyl)-2,3-diméthyl-5-trifluorométhyl-1H-pyrrolo(3,2-b]pyridine

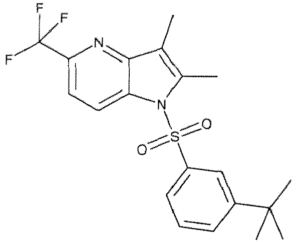

On a préparé une solution de 242,14 mg (0,50 mM) du composé de la préparation 1 dans 10 mL de DMF. On a additionné 11,22 mg (0,04 mM) d'acétate de palladium, 21,20 mg (0,50 mM) de chlorure de lithium anhydre, 345,51 mg (2,50 mM) de carbonate de potassium puis 135,23 mg (2,5 mM) de 2-butyne. Le milieu a été irradié par micro-ondes 30 minutes à 100 ° C. On a additionné 100 mL d'eau. On a extrait par quatre fois 100 ml de DCM puis par quatre fois 100 mL d'acétate d'éthyle. Les phases organiques ont été séchées sur sulfate de magnésium. Le milieu a été concentré sous pression réduite et le résidu d'évaporation a été purifié par chromatographie sur gel de silice à l'aide d'un éluant cyclohexane / acétate d'éthyle 95/5 (v/v). 220 mg de produit visé ont été obtenus sous la forme d'une huile jaune (rendement = 91,5%).
¹H RMN (300 MHz, DMSO) δ = 8,66 (d, 1H), 7,81 (d, 1H), 7,77 (m, 2H), 7,69 (dt, 1H), 7,53 (t, 1H), 2,61 (s, 3H), 2,18 (s, 3H), 1,21 (s, 9H).

### Exemple 37: acide 4-[[1-[[3-(1,1-diméthyléthyl)phényl]sulfonyl]-3-méthyl-5-(trifluorométhyl)-1H-pyrrolo[3,2-b]pyridin-2-yl]méthyl]-benzoïque

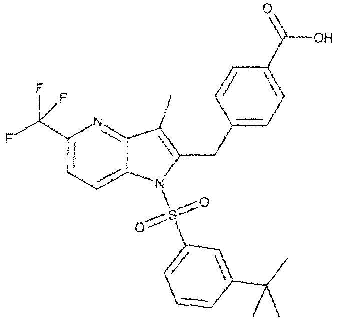

On a préparé une solution de 310,0 mg (0,76 mM) de 1-(3-(1,1-diméthyléthyl)phénylsulfonyl)-2,3diméthyl-5-trifluorométhyl-1H-pyrrolo[3,2-b]pyridine (préparation 23) dans 5 mL de tétrachlorure de carbone. On a additionné 180,0 mg (1,01 mM) de N-bromosuccinimide (NBS) puis le milieu a été porté au reflux. On a alors additionné 12,40 mg (0,08 mM) d'azobisisobutyronitrile (AIBN) puis le milieu a été agité au reflux 24 heures. On a observé un taux de conversion de 50 %. On a de nouveau additionné 200,00 mg (1,12 mM) de NBS puis 24,8 mg (0,16 mM) d'AIBN et on a continué l'agitation au reflux 24 heures. 25 mL de DCM ont été additionnés au milieu et les solvants ont été évaporés. Le résidu d'évaporation a été purifié par chromatographie sur gel de silice à l'aide d'un éluant cyclohexane / acétate d'éthyle 95/5 (v/v) pour obtenir 435 mg de 2-bromométhyl-1-(3-(1,1-diméthyléthyl)phénylsulfonyl)-3méthyl-5-trifluorométhyl-1H-pyrrolo[3,2-b]pyridine sous la forme d'un solide orange (pureté = 58 %).

On a préparé une solution de 200,0 mg (0,41 mM) du composé précédent dans un mélange de 10 mL de diméthyléther (DME) et de 2 mL d'eau. On a ajouté 33,38 mg (0,04 mM) du complexe 1,1'-bis(diphénylphosphino)ferrocène-palladium (II) dichlorure dichlorométhane, 101,73 mg (0,61 mM) d'acide 4-carboryphénylboronique et 399,50 mg (1,23 mM) de carbonate de césium. Le milieu a été agité deux heures au reflux. On a additionné 100 mL d'eau, 10 mL d' HCl M et extrait par quatre fois 100 mL de DCM. Les phases organiques ont été séchées sur sulfate de magnésium et le solvant a été évaporé. Le résidu d'évaporation a été purifié par chromatographie liquide LC-MS semi-préparative (sur colonne Discovery®) en éluant par un mélange eau / acétonitrile/ TFA. Les fractions contenant le produit attendu ont été réunies et concentrées à sec. Le produit visé a été obtenu sous la forme d'un solide beige (20 mg, rendement = 9,6%).

### Préparation 24: [1-(4-(1,1-méthyléthyl)phénylsulfonyl)-5-chloro-1H-pyrrolo[2,3-b]pyridin-2-yl]-méthanol

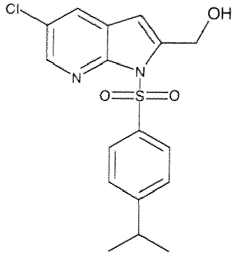

On a obtenu ce composé sous la forme d'un solide beige en suivant le mode opératoire décrit à la préparation 2, à partir du composé de la préparation 21 et du propyne-2 ol-1 (rendement = 78%). F = 140°C.

### Préparation 25: 2-bromométhyl-[1-(4-(1,1-méthyléthyl)phénylsulfonyl)-5-chloro-1H-pyrrolo[2,3-b]pyridine

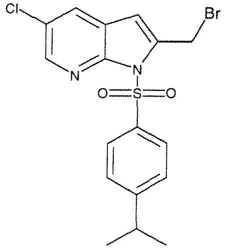

Ce composé a été obtenu sous la forme d'un solide blanc à partir du composé de la préparation 24, en suivant le mode opératoire décrit à la préparation 3 (rendement = 65%). F = 138°C.

### Exemple 38: acide 5-[[1-[[4-(1,1-méthyléthyl)phényl]sulfonyl]-5-chloro-1H-pyrrolo[2,3-b]pyridin-2-yl]méthyl]-thiophène-2-carboxylique

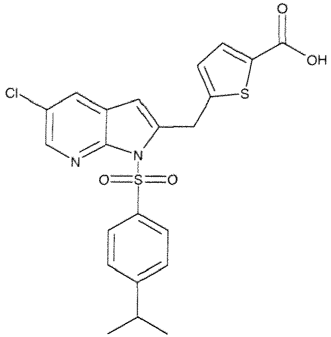

Ce composé a été obtenu sous la forme d'un solide beige en suivant le mode opératoire décrit à l'exemple 1, à partir du composé de la préparation 25 et de l'acide 5-(dihydroxyboryl)-2-thiophènecarboxylique (rendement = 3%). F = 210-243°C.

### Exemple 39 : N-{4-[1-(3-tert-Butyl-benzènesulfonyl)-5-trifluorométhyl-1H-pyrrolo[3,2-b]pyridin-2-ylméthyl]-benzoyl}-méthanesulfonamide

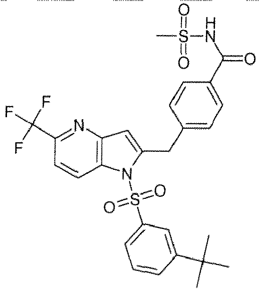

On a préparé une solution de 240 mg (0,46 mM) du composé de l'exemple 22 dans 12 mL de dichlorométhane. On a ajouté 89,07 mg (0,46 mM) de carbodiimide de 1-éthyl-3-(3-diméthylaminopropyl) (EDCI), 56,76 mg (0,46 mM) de 4-diméthylaminopyridine et 88,839 mg (0,93 mM) de méthanesulfonamide. Le milieu réactionnel a été agité 20 heures à température ambiante. Puis il a été concentré sous pression réduite et le résidu d'évaporation a été purifié par chromatographie liquide préparative (LC-MS) en éluant par un mélange H₂O/CH₃CN/ 0,1% TFA. Les fractions contenant le produit attendu ont été réunies et concentrées à sec sous pression réduite pour obtenir le produit désiré sous la forme d'un solide blanc (rendement : 39 %). F = 95°C

### Activité pharmacologique

Les composés de l'invention ont été soumis à des tests biologiques de façon à évaluer leur potentiel à traiter ou prévenir certaines pathologies neurodégénératives.

On a mesuré, par un test *in vitro,* l'aptitude des composés selon l'invention à se comporter en activateur des hétérodimères formés par le récepteur nucléaire NURR-1 et les récepteurs nucléaires RXR.

Un test de transactivation a été utilisé comme test de sélection (screening) primaire. Des cellules Cos-7 ont été co-transfectées avec un plasmide exprimant une chimère du récepteur humain NURR-1-Gal4, un plasmide exprimant le récepteur humain RXR (récepteur RXRα ou RXRγ) et un plasmide rapporteur 5Gal4pGL3-TK-Luc. Les transfections ont été réalisées à l'aide d'un agent chimique (Jet PEI).

Les cellules transfectées ont été distribuées dans des plaques de 384 puits et laissées au repos pendant 24 heures.

Au temps 24 heures le milieu de culture a été changé. Les produits à tester ont été ajoutés (concentration finale comprise entre 10⁻⁴ et 3.10⁻¹⁰ M) dans le milieu de culture. Après une nuit d'incubation, l'expression de luciférase a été mesurée après addition de « SteadyGlo » selon les instructions du fabricant (Promega).

L'acide 4-[[6-méthyl-2-phényl-5-(2-propényl)-4-pyrimidinyl]amino]-benzoïque (nommé XCT0135908, décrit par Wallen-Mackenzie et al. 2003, Genes & Development 17 : 3036-3047) à 2.10⁻⁵ M (agoniste RXR) a été utilisé comme référence.

Les niveaux d'induction ont été calculés par rapport à l'activité basale de chaque hétérodimère. Les résultats ont été exprimés en pourcentage du niveau d'induction par rapport au niveau d'induction obtenu avec la référence (le niveau d'induction de la référence est arbitrairement égal à 100 %).

A titre d'exemple, parmi les composés selon l'invention, on obtient les résultats suivants exprimés en pourcentage par rapport à un composé de référence activateur NURR-1/RXR (XCT0135908):

| **Exemple** | **hNurr1_RXRγFL** | | **hNurr1_RXRαFL** | |
|---|---|---|---|---|
| | EC₅₀ (nM) | Eff (%) | EC₅₀ (nM) | Eff (%) |
| 1 | 73 | 64 | 25 | 67 |
| 2 | 100 | 86 | 52 | 70 |
| 4 | - | - | 35 | 104 |
| 5 | - | - | 2344 | 94 |
| 6 | - | - | 17 | 137 |
| 7 | - | - | 11 | 119 |
| 8 | 1514 | 72 | 1023 | 103 |
| 10 | 576 | 82 | 337 | 68 |
| 12 | 761 | 75 | 310 | 81 |
| 14 | 771 | 54 | 541 | 64 |
| 16 | 1052 | 53 | 575 | 68 |
| 18 | 495 | 69 | 194 | 62 |
| 20 | 1189 | 27 | 731 | 42 |
| 22 | 93 | 68 | 42 | 92 |
| 25 | nc | 52 | nc | 57 |
| 28 | 803 | 51 | 634 | 66 |
| 31 | 1346 | 48 | 713 | 63 |
| 32 | - | - | 288 | 84 |
| 33 | - | - | 1820 | 92 |
| 34 | - | - | 1000 | 31 |
| 35 | - | - | 481 | 61 |
| 36 | - | - | 361 | 71 |
| 37 | - | - | 146 | 67 |
| 38 | - | - | 295 | 80 |
| 39 | - | - | 269 | 51 |

| | | | | |
|---|---|---|---|---|
| - : non testé nc : non calculable Eff: efficacité en % par rapport à la référence XCT0135908 | | | | |

Les composés selon l'invention présentent un taux d'induction allant jusqu'à 137 % (NURR1/RXRα) et 86 % (NURR1/RXRγ□□) et des EC₅₀ allant jusqu'à 11 nM (NURR1/RYRα) et 73 nM (NURR1/RXRγ□□).

Ces résultats *in vitro* montrent que les composés de l'invention sont capables de modifier les mécanismes de la maladie sur certains modèles cellulaires et de stopper le processus dégénératif en générant des agents neuroprotecteurs permettant de lutter contre la mort cellulaire des neurones dopaminergiques. Ils confirment donc l'intérêt de ces composés pour leur utilisation en tant que principes actifs de médicaments destinés à la prévention et/ou au traitement des maladies neurodégénératives, et plus particulièrement, de la maladie de Parkinson.

L'invention concerne également une composition pharmaceutique contenant, en tant que principe actif, au moins un composé de formule (I), ou l'un de ses sels pharmaceutiquement acceptables.

Selon un autre aspect, la présente demande vise à couvrir l'utilisation d'un composé de formule (I) ou d'une composition pharmaceutique contenant un tel composé, pour la prévention et/ou le traitement des maladies dans lesquelles le récepteur NURR-1 est impliqué, notamment les maladies neurodégénératives, et plus particulièrement la maladie de Parkinson.

Selon un autre aspect, la présente demande vise à couvrir une méthode de prévention et/ou de traitement des maladies dans lesquelles le récepteur NURR-1 est impliqué, notamment les maladies neurodégénératives, et plus particulièrement la maladie de Parkinson, qui consiste à administrer à un patient en ayant besoin une quantité thérapeutiquement efficace d'un composé de formule (I) ou d'une composition pharmaceutique contenant un tel composé.

Les compositions pharmaceutiques conformes à l'invention peuvent être préparées de façon classique, à l'aide d'excipients pharmaceutiquement acceptables afin d'obtenir des formes administrables par voie parentérale ou, de préférence, par voie orale, par exemple des comprimés ou des gélules.

Dans le cas de formes injectables, on utilisera avantageusement les composés de formule (I) sous forme de sels solubles dans un milieu aqueux. Comme indiqué précédemment, les sels sont préférentiellement formés entre un composé de formule (I) et une base non toxique pharmaceutiquement acceptable. La formulation peut être soit une solution du composé dans un milieu aqueux isotonique en présence d'excipients solubles, soit un lyophilisat du composé auquel le solvant de dilution est ajouté de façon extemporanée. Ces préparations pourront être injectées sous forme de perfusion ou en bolus en fonction des besoins du patient.

De façon pratique, en cas d'administration du composé par voie parentérale, la posologie quotidienne chez l'homme sera de préférence comprise entre 2 et 250 mg.

Les préparations administrables par voie orale seront de préférence présentées sous forme d'une gélule ou d'un comprimé contenant le composé de l'invention broyé finement ou mieux, micronisé, et mélangé avec des excipients connus de l'homme du métier, tels que par exemple du lactose, de l'amidon prégélatinisé et du stéarate de magnésium.

A titre d'exemple, on a granulé un mélange constitué de 500 g du composé de l'exemple 2 finement broyé, 500 g d'amidon prégélatinisé, 1250 g de lactose, 15 g de laurylsulfate de sodium et 235 g de polyvinylpyrrolidone. Ce mélange granulé a ensuite été additionné à 20 g de stéarate de magnésium et 80 g de cellulose microcristalline et le mélange obtenu a été réparti après broyage et tamisage dans des gélules de 260 mg. On a ainsi obtenu des gélules contenant chacune 50 mg de principe actif.

De façon pratique, en cas d'administration du composé par voie orale, la posologie quotidienne chez l'homme sera de préférence comprise entre 5 et 500 mg. On pourra à cet effet utiliser des doses unitaires comprenant de 5 à 250 mg de principe actif, de préférence des doses unitaires comprenant de 5 à 100 mg de principe actif.

## Revendications

1. Composé de formule (I) : 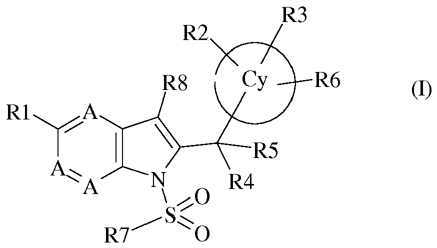 dans laquelle :
l'un des groupes A représente un atome d'azote et les autres groupes A représentent un atome de carbone ;
Cy représente un phényle ou un noyau hétéroaromatique à 5 ou 6 chaînons ;
R1 représente un atome d'hydrogène, un atome d'halogène, un groupe (C₁-C₄)alkyle éventuellement totalement ou partiellement halogéné, ou un groupe (C₁-C₄)alcoxy ;
R2 et R3 représentent chacun, indépendamment l'un de l'autre, un atome d'hydrogène, un atome d'halogène, un groupe (C₁-C₄)alkyle, un groupe hydroxy, ou un groupe (C₁-C₄)alcoxy ;
R4 et R5 représentent chacun, indépendamment l'un de l'autre, un atome d'hydrogène, un atome d'halogène, un groupe (C₁-C₄)alkyle, ou un groupe hydroxy;
ou R4 et R5 forment ensemble avec l'atome de carbone auquel ils sont rattachés un groupe éthylène (C=CH2) ou un groupe carbonyle (C=O) ;
R6 représente un groupe -COOR9 ou un groupe -SO₂NHR10, -CONHNHCOOR11, -CONR12R13 ou -CONHSO₂R14, ou un groupe 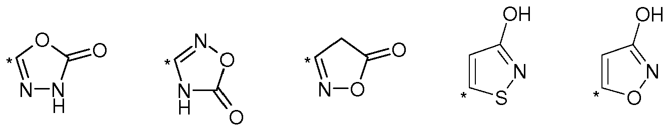 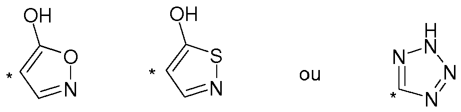
R7 représente un phényle éventuellement substitué par un groupe (C₁-C₄)alkyle, ou un noyau hétéroaromatique ayant de 6 à 10 chaînons éventuellement substitué par un groupe (C₁-C₄)alkyle ;
R8 représente un atome d'hydrogène, un groupe (C₁-C₄)alkyle ou un atome d'halogène ;
R9 représente un atome d'hydrogène ou un groupe (C₁-C₄)alkyle ;
R10, R11, R12, R13 et R14 représentent chacun indépendamment l'un de l'autre un atome d'hydrogène ou un groupe (C₁-C₄)alkyle ;
ou un sel pharmaceutiquement acceptable dudit composé de formule (I) ;
à l'exception des composés suivants :
l'acide 2-(1-phénylsulfonyl-1H-pyrrolo[2,3-b]pyridin-2-yl-carbonyl)-benzoïque ;
le N,N-diéthyl-4-[hydroxy-[1-(phénylsulfonyl)-1H-pyrrolo[2,3-b]pyridin-2-yl]méthyl]-2-méthoxy-3-pyridinecarboxamide ;
le N,N-diéthyl-2-méthoxy-4-[[1-(phénylsulfonyl)-1H-pyrrolo[2,3-b]pyridin-2-yl]carbonyl]-3-pyridinecarboxamide ;
le N,N-diéthyl-4-[1-hydroxy-1-[1-(phénylsulfonyl)-1H-pyrrolo[2,3-b]pyridin-2-yl]éthyl]-2-méthoxy-3-pyridinecarboxamide.

2. Composé selon la revendication 1, dans lequel R8 représente un atome d'hydrogène ;
ou un sel pharmaceutiquement acceptable dudit composé.

3. Composé selon la revendication 1, dans lequel :
Cy représente un phényle ou un noyau hétéroaromatique à 5 ou 6 chaînons ;
R1 représente un atome d'halogène ou un groupe (C₁-C₄)alkyle éventuellement totalement ou partiellement halogéné ;
R2 et R3 représentent chacun, indépendamment l'un de l'autre, un atome d'hydrogène ou un atome d'halogène;
R4 et R5 représentent chacun, indépendamment l'un de l'autre, un atome d'hydrogène, un atome d'halogène ou un groupe hydroxy ;
ou R4 et R5 forment ensemble avec l'atome de carbone auxquels ils sont rattachés un groupe carbonyle (C=O) ;
R6 représente un groupe -COOR9;
R7 représente un phényle éventuellement substitué par un groupe (C₁-C₄)alkyle;
R8 représente un atome d'hydrogène ;
R9 représente un atome d'hydrogène ou un groupe (C₁-C₄)alkyle ;
ou un sel pharmaceutiquement acceptable dudit composé.

4. Composé selon l'une des revendications 1 à 3, dans lequel Cy représente un phényle, thiényle, thiazolyle, furanyle ou pyridyle ;
ou un sel pharmaceutiquement acceptable dudit composé.

5. Composé selon l'une des revendications 1 à 4, dans lequel R2 et R3 représentent chacun un atome d'hydrogène ;
ou un sel pharmaceutiquement acceptable dudit composé.

6. Composé selon l'une des revendications 1 à 5, dans lequel R4 et R5 représentent chacun un atome d'hydrogène ;
ou un sel pharmaceutiquement acceptable dudit composé.

7. Composé selon la revendication 1, choisi parmi :
l'acide 5-[[1-[[3-(1,1-diméthyléthyl)phényl]sulfonyl]-5-(trifluorométhyl)-1H-pyrrolo[3,2-b]pyridin-2-yl]méthyl]-thiophène-2-carboxylique,
l'acide 3-[[1-[[3-(1,1-diméthyléthyl)phényl]sulfonyl]-5-(trifluorométhyl)-1H-pyrrolo[2,3-c]pyridin-2-yl]méthyl]-2-benzoïque,
l'acide 2-chloro-4-[[1-[[3-(1,1-diméthyléthyl)phényl]sulfonyl]-5-(trifluorométhyl)-1H-pyrrolo[2,3-c]pyridin-2-yl]méthyl]-benzoïque,
l'acide 5-[[1-[[3-(1,1-diméthyléthyl)phényl]sulfonyl]-5-(trifluorométhyl)-1H-pyrrolo[2,3-c]pyridin-2-yl]méthyl]-furane-3-carboxylique,
l'acide 5-[[1-[[3-(1,1-diméthyléthyl)phényl]sulfonyl]-5-(trifluorométhyl)-1H-pyrrolo[2,3-c]pyridin-2-yl]méthyl]-pyridine-3-carboxylique,
l'acide 4-[[1-[[3-(1,1-diméthyléthyl)phényl]sulfonyl]-5-(trifluorométhyl)-1H-pyrrolo[2,3-c]pyridin-2-yl]méthyl]-thiophène-2-carboxylique,
l'acide 5-[[1-[[3-(1,1-diméthyléthyl)phényl]sulfonyl]-5-(trifluorométhyl)-1H-pyrrolo[2,3-c]pyridin-2-yl]méthyl]-2-fluoro-benzoïque,
l'acide 2-[[1-[[3-(1,1-diméthyléthyl)phényl]sulfonyl]-5-(trifluorométhyl)-1H-pyrrolo[3,2-b]pyridin-2-yl]méthyl]-thiazole-4-carboxylique,
le 4-[1-(4-(1-méthyléthyl)phénylsulfonyl)-5-trifluorométhyl-1H-pyrrolo[2,3-b]pyridin-2-ylméthyl]-benzoate de méthyle,
l'acide 4-[[1-[[3-(1-méthyléthyl)phényl]sulfonyl]-5-(trifluorométhyl)-1H-pyrrolo[2,3-b]pyridin-2-yl]méthyl]-benzoïque,
le 4-[[1-[(3,4-dihydro-4-méthyl-2H-1,4-benzoxazin-6-yl)sulfonyl]-5-(trifluorométhyl)-1H-pyrrolo[2,3-c]pyridin-2-yl]méthyl]-benzoate de méthyle,
l'acide 4-[[1-[(3,4-dihydro-4-méthyl-2H-1,4-benzoxazin-6-yl)sulfonyl]-5-(trifluorométhyl)-1H-pyrrolo[2,3-c]pyridin-2-yl]méthyl]-benzoïque,
le 4-[1-(4-(1-méthyléthyl)phénylsulfonyl)-5-trifluorométhyl-1H-pyrrolo[2,3-c]pyridin-2-ylméthyl]-benzoate de méthyle,
l'acide 4-[[1-[[4-(1-méthyléthyl)phényl]sulfonyl]-5-(trifluorométhyl)-1H-pyrrolo[2,3-c]pyridin-2-yl]méthyl]-benzoïque,
le 4-[1-(4-(1-méthyléthyl)phénylsulfonyl)-5-trifluorométhyl-1H-pyrrolo[3,2-b]pyridin-2-ylméthyl]-benzoate de méthyle,
l'acide 4-[1-(4-(1-méthyléthyl)phénylsulfonyl)-5-trifluorométhyl-1H-pyrrolo[3,2-b]pyridin-2-ylméthyl] -benzoïque,
le 4-[[1-[(3,4-dihydro-4-méthyl-2H-1,4-benzoxazin-6-yl)sulfonyl]-5-(trifluorométhyl)-1H-pyrrolo[2,3-b]pyridin-2-yl]méthyl]-benzoate de méthyle,
l'acide 4-[[1-[(3,4-dihydro-4-méthyl-2H-1,4-benzoxazin-6-yl)sulfonyl]-5-(trifluorométhyl)-1H-pyrrolo[2,3-b]pyridin-2-yl]méthyl]-benzoïque,
le 4-[1-(4-(1-méthyléthyl)phénylsulfonyl)-5-chloro-1H-pyrrolo[2,3-c]pyridin-2-ylméthyl]-benzoate de méthyle,
l'acide 4-[[5-chloro-1-[[4-(1-méthyléthyl)phényl]sulfonyl]-1H-pyrrolo[2,3-c]pyridin-2-yl]méthyl]-benzoïque,
le 4-[1-(3-(1,1-diméthyléthyl)phénylsulfonyl)-5-trifluorométhyl-1H-pyrrolo[3,2-b]pyridin-2-ylméthyl]-benzoate de méthyle,
l'acide 4-[[1-[[3-(1,1-diméthyléthyl)phényl]sulfonyl]-5-(trifluorométhyl)-1H-pyrrolo[3,2-b]pyridin-2-yl]méthyl]-benzoïque,
le 4-{Hydroxy-[1-(3-(1-méthyléthyl)phénylsulfonyl)-5-trifluorométhyl-1H-pyrrolo[2,3-b]pyridin-2-yl]-méthyl}-benzoate de méthyle,
le 4-[1-(3-(1-méthyléthyl)phénylsulfonyl)-5-trifluorométhyl-1H-pyrrolo[2,3-b]pyridin-2-ylméthyl]-benzoate de méthyle,
l'acide 4-[[1-[[3-(1-méthyléthyl)phényl]sulfonyl]-5-(trifluorométhyl)-1H-pyrrolo[2,3-b]pyridin-2-yl]méthyl]-benzoïque,
le 4-{Hydroxy-[1-(3-(1,1-diméthyléthyl)phénylsulfonyl)-5-trifluorométhyl-1H-pyrrolo[2,3-b]pyridin-2-yl]-méthyl}-benzoate de méthyle,
le 4-[1-(3-(1,1-diméthyléthyl)phénylsulfonyl)-5-trifluorométhyl-1H-pyrrolo[2,3-b]pyridin-2-ylméthyl]-benzoate de méthyle,
l'acide 4-[[1-[[3-(1,1-diméthyléthyl)phényl]sulfonyl]-5-(trifluorométhyl)-1H-pyrrolo[2,3-b]pyridin-2-yl]méthyl]-benzoïque,
le 4-{Hydroxy-[5-chloro-1-(4-(1-méthyléthyl)phénylsulfonyl)-1H-pyrrolo[2,3-b]pyridin-2-yl]-méthyl}-benzoate de méthyle,
le 4-[5-Chloro-1-(4-(1-méthyléthyl)phénylsulfonyl)-1H-pyrrolo[2,3-b]pyridin-2-ylméthyl]-benzoate de méthyle,
l'acide 4-[[5-chloro-1-[[4-(1-méthyléthyl)phényl]sulfonyl]-1H-pyrrolo-[2,3-b]pyridin-2-yl]méthyl]-benzoïque,
l'acide 5-{hydroxy-[1-(3-(1,1-diméthyléthyl)phénylsulfonyl)-5-trifluorométhyl-1H-pyrrolo[3,2-b]pyridin-2-yl]-méthyl}-thiophène-2-carboxylique,
le 5-[[1-[[3-(1,1-diméthyléthyl)phényl]sulfonyl]-5-(trifluorométhyl)-1H-pyrrolo[3,2-b]pyridin-2-yl]-fluorométhyl]-N,N-diéthyl-2-thiophènecarboxamide,
l'acide 5-{1-(3-(1,1-diméthyléthyl)phénylsulfonyl)-5-trifluorométhyl-1H-pyrrolo[3,2-b]pyridine-2-carbonyl}-thiophène-2-carboxylique,
l'acide 4-[[5-chloro-1-[[4-(1-méthyléthyl)phényl]sulfonyl]-1H-pyrrolo-[2,3-b]pyridin-2-yl]méthyl]-benzoïque, sel de sodium,
l'acide 4-[[5-chloro-1-[[4-(1-méthyléthyl)phényl]sulfonyl]-1H-pyrrolo-[2,3-b]pyridin-2-yl]méthyl]-benzoïque, sel de pipérazine,
l'acide 4-[[1-[[3-(1,1-diméthyléthyl)phényl]sulfonyl]-3-méthyl-5-(trifluorométhyl)-1H-pyrrolo[3,2-b]pyridin-2-yl]méthyl]-benzoïque,
l'acide 5-[[1-[[4-(1,1-méthyléthyl)phényl]sulfonyl]-5-chloro-1H-pyrrolo[2,3-b]pyridin-2-yl]méthyl]-thiophène-2-carboxylique,
le N-{4-[1-(3-tert-butyl-benzènesulfonyl)-5-trifluorométhyl-1H-pyrrolo[3,2-b]pyridin-2-ylméthyl]-benzoyl}-méthanesulfonamide ;
ou les sels pharmaceutiquement acceptables de ces composés.

8. Composé de formule (I) : 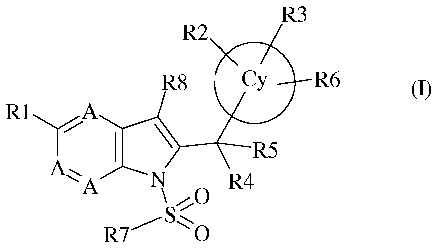 dans laquelle :
l'un des groupes A représente un atome d'azote et les autres groupes A représentent un atome de carbone ;
Cy représente un phényle ou un noyau hétéroaromatique à 5 ou 6 chaînons ;
R1 représente un atome d'hydrogène, un atome d'halogène, un groupe (C₁-C₄)alkyle éventuellement totalement ou partiellement halogéné, ou un groupe (C₁-C₄)alcoxy ;
R2 et R3 représentent chacun, indépendamment l'un de l'autre, un atome d'hydrogène, un atome d'halogène, un groupe (C₁-C₄)alkyle, un groupe hydroxy, ou un groupe (C₁-C₄)alcoxy ;
R4 et R5 représentent chacun, indépendamment l'un de l'autre, un atome d'hydrogène, un atome d'halogène, un groupe (C₁-C₄)alkyle, ou un groupe hydroxy;
ou R4 et R5 forment ensemble avec l'atome de carbone auquel ils sont rattachés un groupe éthylène (C=CH2) ou un groupe carbonyle (C=O) ;
R6 représente un groupe -COOR9 ou un groupe -SO₂NHR10, -CONHNHCOOR11, -CONR12R13 ou -CONHSO₂R14, ou un groupe 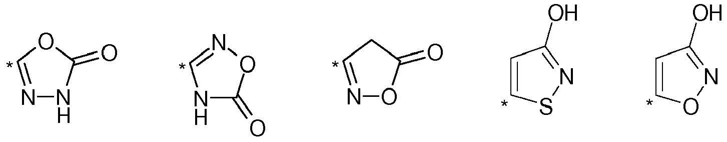 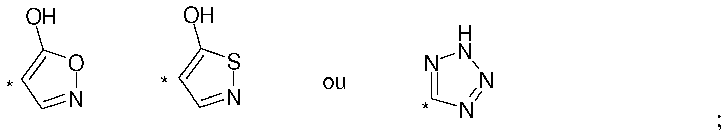
R7 représente un phényle éventuellement substitué par un groupe (C₁-C₄)alkyle, ou un noyau hétéroaromatique ayant de 6 à 10 chaînons éventuellement substitué par un groupe (C₁-C₄)alkyle ;
R8 représente un atome d'hydrogène, un groupe (C₁-C₄)alkyle ou un atome d'halogène ;
R9 représente un atome d'hydrogène ou un groupe (C₁-C₄)alkyle ;
R10, R11, R12, R13 et R14 représentent chacun indépendamment l'un de l'autre un atome d'hydrogène ou un groupe (C₁-C₄)alkyle
ou un sel pharmaceutiquement acceptable de celui-ci ;
pour son utilisation comme substance thérapeutiquement active.

9. Composé de formule (I) 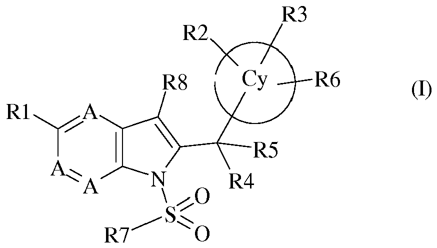 dans laquelle A, Cy, R1, R2, R3, R4, R5, R6, R7 et R8 sont tels que définis à la revendication 8, ou un sel pharmaceutiquement acceptable de celui-ci, pour son utilisation dans la prévention et/ou le traitement des maladies neurodégénératives.

10. Composé pour son utilisation selon la revendication 9, où la maladie neurodégénérative est la maladie de Parkinson.

11. Composé pour son utilisation selon la revendication 9 ou la revendication 10, qui est choisi parmi :
l'acide 5-[[1-[[3-(1,1-diméthyléthyl)phényl]sulfonyl]-5-(trifluorométhyl)-1H-pyrrolo[3,2-b]pyridin-2-yl]méthyl]-thiophène-2-carboxylique,
l'acide 3-[[1-[[3-(1,1-diméthyléthyl)phényl]sulfonyl]-5-(trifluorométhyl)-1H-pyrrolo[2,3-c]pyridin-2-yl]méthyl]-2-benzoïque,
l'acide 2-chloro-4-[[1-[[3-(1,1-diméthyléthyl)phényl]sulfonyl]-5-(trifluorométhyl)-1H-pyrrolo[2,3-c]pyridin-2-yl]méthyl]-benzoïque,
l'acide 5-[[1-[[3-(1,1-diméthyléthyl)phényl]sulfonyl]-5-(trifluorométhyl)-1H-pyrrolo[2,3-c]pyridin-2-yl]méthyl]-furane-3-carboxylique,
l'acide 5-[[1-[[3-(1,1-diméthyléthyl)phényl]sulfonyl]-5-(trifluorométhyl)-1H-pyrrolo[2,3-c]pyridin-2-yl]méthyl]-pyridine-3-carboxylique,
l'acide 4-[[1-[[3-(1,1-diméthyléthyl)phényl]sulfonyl]-5-(trifluorométhyl)-1H-pyrrolo[2,3-c]pyridin-2-yl]méthyl]-thiophène-2-carboxylique,
l'acide 5-[[1-[[3-(1,1-diméthyléthyl)phényl]sulfonyl]-5-(trifluorométhyl)-1H-pyrrolo[2,3-c]pyridin-2-yl]méthyl]-2-fluoro-benzoïque,
l'acide 2-[[1-[[3-(1,1-diméthyléthyl)phényl]sulfonyl]-5-(trifluorométhyl)-1H-pyrrolo[3,2-b]pyridin-2-yl]méthyl]-thiazole-4-carboxylique,
le 4-[1-(4-(1-méthyléthyl)phénylsulfonyl)-5-trifluorométhyl-1H-pyrrolo[2,3-b]pyridin-2-ylméthyl]-benzoate de méthyle,
l'acide 4-[[1-[[3-(1-méthyléthyl)phényl]sulfonyl]-5-(trifluorométhyl)-1H-pyrrolo[2,3-b]pyridin-2-yl]méthyl]-benzoïque,
le 4-[[1-[(3,4-dihydro-4-méthyl-2H-1,4-benzoxazin-6-yl)sulfonyl]-5-(trifluorométhyl)-1H-pyrrolo[2,3-c]pyridin-2-yl]méthyl]-benzoate de méthyle,
l'acide 4-[[1-[(3,4-dihydro-4-méthyl-2H-1,4-benzoxazin-6-yl)sulfonyl]-5-(trifluorométhyl)-1H-pyrrolo[2,3-c]pyridin-2-yl]méthyl]-benzoïque,
le 4-[1-(4-(1-méthyléthyl)phénylsulfonyl)-5-trifluorométhyl-1H-pyrrolo[2,3-c]pyridin-2-ylméthyl]-benzoate de méthyle,
l'acide 4-[[1-[[4-(1-méthyléthyl)phényl]sulfonyl]-5-(trifluorométhyl)-1H-pyrrolo[2,3-c]pyridin-2-yl]méthyl]-benzoïque,
le 4-[1-(4-(1-méthyléthyl)phénylsulfonyl)-5-trifluorométhyl-1H-pyrrolo[3,2-b]pyridin-2-ylméthyl]-benzoate de méthyle,
l'acide 4-[1-(4-(1-méthyléthyl)phénylsulfonyl)-5-trifluorométhyl-1H-pyrrolo[3,2-b]pyridin-2-ylméthyl]-benzoïque,
le 4-[[1-[(3,4-dihydro-4-méthyl-2H-1,4-benzoxazin-6-yl)sulfonyl]-5-(trifluorométhyl)-1H-pyrrolo[2,3-b]pyridin-2-yl]méthyl]-benzoate de méthyle,
l'acide 4-[[1-[(3,4-dihydro-4-méthyl-2H-1,4-benzoxazin-6-yl)sulfonyl]-5-(trifluorométhyl)-1H-pyrrolo[2,3-b]pyridin-2-yl]méthyl]-benzoïque,
le 4-[1-(4-(1-méthyléthyl)phénylsulfonyl)-5-chloro-1H-pyrrolo[2,3-c]pyridin-2-ylméthyl]-benzoate de méthyle,
l'acide 4-[[5-chloro-1-[[4-(1-méthyléthyl)phényl]sulfonyl]-1H-pyrrolo[2,3-c]pyridin-2-yl]méthyl]-benzoïque,
le 4-[1-(3-(1,1-diméthyléthyl)phénylsulfonyl)-5-trifluorométhyl-1H-pyrrolo[3,2-b]pyridin-2-ylméthyl]-benzoate de méthyle,
l'acide 4-[[1-[[3-(1,1-diméthyléthyl)phényl]sulfonyl]-5-(trifluorométhyl)-1H-pyrrolo[3,2-b]pyridin-2-yl]méthyl]-benzoïque,
le 4-{Hydroxy-[1-(3-(1-méthyléthyl)phénylsulfonyl)-5-trifluorométhyl-1H-pyrrolo[2,3-b]pyridin-2-yl]-méthyl}-benzoate de méthyle,
le 4-[1-(3-(1-méthyléthyl)phénylsulfonyl)-5-trifluorométhyl-1H-pyrrolo[2,3-b]pyridin-2-ylméthyl]-benzoate de méthyle,
l'acide 4-[[1-[[3-(1-méthyléthyl)phényl]sulfonyl]-5-(trifluorométhyl)-1H-pyrrolo[2,3-b]pyridin-2-yl]méthyl]-benzoïque,
le 4-{Hydroxy-[1-(3-(1,1-diméthyléthyl)phénylsulfonyl)-5-trifluorométhyl-1H-pyrrolo[2,3-b]pyridin-2-yl]-méthyl}-benzoate de méthyle,
le 4-[1-(3-(1,1-diméthyléthyl)phénylsulfonyl)-5-trifluorométhyl-1H-pyrrolo[2,3-b]pyridin-2-ylméthyl]-benzoate de méthyle,
l'acide 4-[[1-[[3-(1,1-diméthyléthyl)phényl]sulfonyl]-5-(trifluorométhyl)-1H-pyrrolo[2,3-b]pyridin-2-yl]méthyl]-benzoïque,
le 4-{Hydroxy-[5-chloro-1-(4-(1-méthyléthyl)phénylsulfonyl)-1H-pyrrolo[2,3-b]pyridin-2-yl]-méthyl}-benzoate de méthyle,
le 4-[5-Chloro-1-(4-(1-méthyléthyl)phénylsulfonyl)-1H-pyrrolo[2,3-b]pyridin-2-ylméthyl]-benzoate de méthyle,
l'acide 4-[[5-chloro-1-[[4-(1-méthyléthyl)phényl]sulfonyl]- 1H-pyrrolo-[2,3-b]pyridin-2-yl]méthyl]-benzoïque,
l'acide 5-{hydroxy-[1-(3-(1,1-diméthyléthyl)phénylsulfonyl)-5-trifluorométhyl-1H-pyrrolo[3,2-b]pyridin-2-yl]-méthyl}-thiophène-2-carboxylique,
le 5-[[1-[[3-(1,1-diméthyléthyl)phényl]sulfonyl]-5-(trifluorométhyl)-1H-pyrrolo[3,2-b]pyridin-2-yl]-fluorométhyl]-N,N-diéthyl-2-thiophènecarboxamide,
l'acide 5-{1-(3-(1,1-diméthyléthyl)phénylsulfonyl)-5-trifluorométhyl-1H-pyrrolo[3,2-b]pyridine-2-carbonyl}-thiophène-2-carboxylique,
l'acide 4-[[5-chloro-1-[[4-(1-méthyléthyl)phényl]sulfonyl]-1H-pyrrolo-[2,3-b]pyridin-2-yl]méthyl]-benzoïque, sel de sodium,
l'acide 4-[[5-chloro-1-[[4-(1-méthyléthyl)phényl]sulfonyl]-1H-pyrrolo-[2,3-b]pyridin-2-yl]méthyl]-benzoïque, sel de pipérazine,
l'acide 4-[1-[[3-(1,1-diméthyléthyl)phényl]sulfonyl]-3-méthyl-5-(trifluorométhyl)-1H-pyrrolo[3,2-b]pyridin-2-yl]méthyl]-benzoïque,
l'acide 5-[[1-[[4-(1,1-méthyléthyl)phényl]sulfonyl]-5-chloro-1H-pyrrolo[2,3-b]pyridin-2-yl]méthyl]-thiophène-2-carboxylique,
le N-{4-[1-(3-tert-butyl-benzènesulfonyl)-5-trifluorométhyl-1H-pyrrolo[3,2-b]pyridin-2-ylméthyl]-benzoyl}-méthanesulfonamide ;
ou les sels pharmaceutiquement acceptables de ces composés.

12. Composition pharmaceutique comprenant, en tant que substance active, un composé de formule (I) : 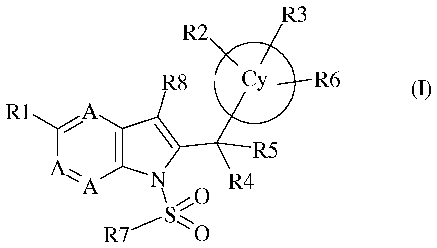 dans laquelle A, Cy, R1, R2, R3, R4, R5, R6, R7 et R8 sont tels que définis à la revendication 8, ou un sel pharmaceutiquement acceptable de celui-ci ;
et au moins un excipient pharmaceutiquement acceptable.

## Patentansprüche

1. Verbindung der Formel (I): 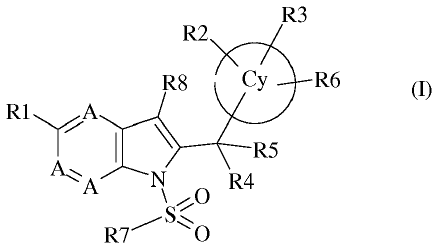 worin:
eine der A-Gruppen ein Stickstoffatom darstellt und die anderen A-Gruppen ein Kohlenstoffatom darstellen;
Cy ein Phenyl oder einen 5- oder 6-gliedrigen heteroaromatischen Kern darstellt;
R1 ein Wasserstoffatom, ein Halogenatom, eine (C₁-C₄)-Alkylgruppe, eventuell vollständig oder teilweise halogeniert, oder eine (C₁-C₄)-Alkoxygruppe darstellt;
R2 und R3 jeweils, unabhängig voneinander, ein Wasserstoffatom, ein Halogenatom, eine (C₁-C₄)-Alkylgruppe, eine Hydroxygruppe oder eine (C₁-C₄)-Alkoxygruppe darstellen;
R4 und R5 jeweils, unabhängig voneinander, ein Wasserstoffatom, ein Halogenatom, eine (C₁-C₄)-Alkylgruppe oder eine Hydroxygruppe darstellen;
oder R4 und R5 zusammen mit dem Kohlenstoffatom, an das sie gebunden sind, eine Ethylengruppe (C=CH2) oder eine Carbonylgruppe (C=O) bilden;
R6 eine -COOR9-Gruppe oder eine -SO₂NHR10-, -CONHNHCOOR11-, - CONR12R13- oder -CONHSO₂R14-Gruppe oder eine 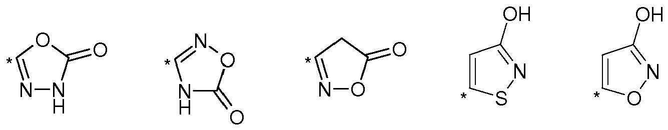 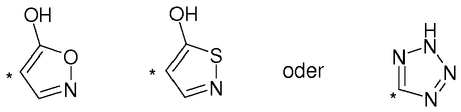
-Gruppe darstellt;
R7 ein Phenyl, eventuell substituiert durch eine (C₁-C₄)-Alkylgruppe, oder einen 6- bis 10-gliedrigen heteroaromatischen Kern, eventuell substituiert durch eine (C₁-C₄)-Alkylgruppe, darstellt;
R8 ein Wasserstoffatom, eine (C₁-C₄)-Alkylgruppe oder ein Halogenatom darstellt;
R9 ein Wasserstoffatom oder eine (C₁-C₄)-Alkylgruppe darstellt;
R10, R11, R12, R13 und R14 jeweils unabhängig voneinander ein Wasserstoffatom oder eine (C₁-C₄)-Alkylgruppe darstellen;
oder ein pharmazeutisch akzeptables Salz der Verbindung der Formel (I);
mit Ausnahme der folgenden Verbindungen:
2-((1-Phenylsulfonyl-1 H-pyrrolo[2,3-b]pyridin-2-yl-carbonyl)-benzoesäure;
N,N-Diethyl-4-[hydroxy-[1-(phenylsulfonyl)-1 H-pyrrolo[2,3-b]pyridin-2-yl]methyl]-2-methoxy-3-pyridincarboxamid;
N,N-Diethyl-2-methoxy-4-[[1-(phenylsulfonyl)-1 H-pyrrolo[2,3-b]pyridin-2-yl]carbonyl]-3-pyridincarboxamid;
N,N-Diethyl-4-[1-hydroxy-1-[1-(phenylsulfonyl)-1H-pyrrolo[2,3-b]pyridin-2-yl]ethyl]-2-methoxy-3-pyridincarboxamid.

2. Verbindung nach Anspruch 1, wobei R8 ein Wasserstoffatom darstellt;
oder ein pharmazeutisch akzeptables Salz der Verbindung.

3. Verbindung nach Anspruch 1, wobei:
Cy ein Phenyl oder einen 5- oder 6-gliedrigen heteroaromatischen Kern darstellt;
R1 ein Halogenatom oder eine (C₁-C₄)-Alkylgruppe, eventuell vollständig oder teilweise halogeniert, darstellt;
R2 und R3 jeweils, unabhängig voneinander, ein Wasserstoffatom oder ein Halogenatom darstellen;
R4 und R5 jeweils, unabhängig voneinander, ein Wasserstoffatom, ein Halogenatom oder eine Hydroxygruppe darstellen;
oder R4 und R5 zusammen mit dem Kohlenstoffatom, an das sie gebunden sind, eine Carbonylgruppe (C=O) bilden;
R6 eine -COOR9-Gruppe darstellt;
R7 ein Phenyl, eventuell substituiert durch eine (C₁-C₄)-Alkylgruppe darstellt;
R8 ein Wasserstoffatom darstellt;
R9 ein Wasserstoffatom oder eine (C₁-C₄)-Alkylgruppe darstellt;
oder ein pharmazeutisch akzeptables Salz der Verbindung.

4. Verbindung nach einem der Ansprüche 1 bis 3, wobei Cy ein Phenyl, Thienyl, Thiazolyl, Furanyl oder Pyridyl darstellt;
oder ein pharmazeutisch akzeptables Salz der Verbindung.

5. Verbindung nach einem der Ansprüche 1 bis 4, wobei R2 und R3 jeweils ein Wasserstoffatom darstellen;
oder ein pharmazeutisch akzeptables Salz der Verbindung.

6. Verbindung nach einem der Ansprüche 1 bis 5, wobei R4 und R5 jeweils ein Wasserstoffatom darstellen;
oder ein pharmazeutisch akzeptables Salz der Verbindung.

7. Verbindung nach Anspruch 1, die ausgewählt ist aus:
5-[[1-[[3-(1,1-Dimethylethyl)phenyl]sulfonyl]-5-(trifluoromethyl)-1 H-pyrrolo[3,2-b]pyridin-2-yl]methyl]-thiophen-2-carbonsäure,
3-[[1-[[3-(1,1-Dimethylethyl)phenyl]sulfonyl]-5-(trifluoromethyl)-1 H-pyrrolo[2,3-c]pyridin-2-yl]methyl]-2-benzoesäure,
2-Chloro-4-[[1-[[3-(1,1-dimethylethyl)phenyl]sulfonyl]-5-(trifluoromethyl)-1H-pyrrolo[2,3-c]pyridin-2-yl]methyl]-benzoesäure,
5-[[1-[[3-(1,1-Dimethylethyl)phenyl]sulfonyl]-5-(trifluoromethyl)-1 H-pyrrolo[2,3-c]pyridin-2-yl]methyl]-furan-3-carbonsäure,
5-[[1-[[3-(1,1-Dimethylethyl)phenyl]sulfonyl]-5-(trifluoromethyl)-1H-pyrrolo[2,3-c]pyridin-2-yl]methyl]-pyridin-3-carbonsäure,
4-[[1-[[3-(1,1-Dimethylethyl)phenyl]sulfonyl]-5-(trifluoromethyl)-1H-pyrrolo[2,3-c]pyridin-2-yl]methyl]-thiophen-2-carbonsäure,
5-[[1-[[3-(1,1-Dimethylethyl)phenyl]sulfonyl]-5-(trifluoromethyl)-1H-pyrrolo[2,3-c]pyridin-2-yl]methyl]-2-fluoro-benzoesäure,
2-[[1-[[3-(1,1-Dimethylethyl)phenyl]sulfonyl]-5-(trifluoromethyl)-1H-pyrrolo[3,2-b]pyridin-2-yl]methyl]-thiazol-4-carbonsäure,
4-[1-(4-(1-Methylethyl)phenylsulfonyl)-5-trifluoromethyl-1H-pyrrolo[2,3-b]pyridin-2-ylmethyl]-methylbenzoat,
4-[[1-[[3-(1-Methylethyl)phenyl]sulfonyl]-5-(trifluoromethyl)-1 H-pyrrolo[2,3-b]pyridin-2-yl]methyl]-benzoesäure,
4-[[1-[(3,4-Dihydro-4-methyl-2H-1,4-benzoxazin-6-yl)sulfonyl]-5-(trifluoromethyl)-1 H-pyrrolo[2,3-c]pyridin-2-yl]methyl]-methylbenzoat,
4-[[1-[(3,4-Dihydro-4-methyl-2H-1,4-benzoxazin-6-yl)sulfonyl]-5-(trifluoromethyl)-1 H-pyrrolo[2,3-c]pyridin-2-yl]methyl]-benzoesäure,
4-[1-(4-(1-Methylethyl)phenylsulfonyl)-5-trifluoromethyl-1H-pyrrolo[2,3-c]pyridin-2-ylmethyl]-methylbenzoat,
4-[[1-[[4-(1-Methylethyl)phenyl]sulfonyl]-5-(trifluoromethyl)-1 H-pyrrolo[2,3-c]pyridin-2-yl]methyl]-benzoesäure,
4-[1-(4-(1-Methylethyl)phenylsulfonyl)-5-trifluoromethyl-1H-pyrrolo[3,2-b]pyridin-2-ylmethyl]-methylbenzoat,
4-[1-(4-(1-Methylethyl)phenylsulfonyl)-5-trifluoromethyl-1H-pyrrolo[3,2-b]pyridin-2-ylmethyl]-benzoesäure,
4-[[1-[(3,4-Dihydro-4-methyl-2H-1,4-benzoxazin-6-yl)sulfonyl]-5-(trifluoromethyl)-1H-pyrrolo[2,3-b]pyridin-2-yl]methyl]-methylbenzoat,
4-[[1-[(3,4-Dihydro-4-methyl-2H-1,4-benzoxazin-6-yl)sulfonyl]-5-(trifluoromethyl)-1H-pyrrolo[2,3-b]pyridin-2-yl]methyl]-benzoesäure,
4-[1-(4-(1-Methylethyl)phenylsulfonyl)-5-chloro-1 H-pyrrolo[2,3-c]pyridin-2-ylmethyl]-methylbenzoat,
4-[[5-Chloro-1-[[4-(1-methylethyl)phenyl]sulfonyl]-1H-pyrrolo[2,3-c]pyridin-2-yl]methyl]-benzoesäure,
4-[1-(3-(1,1-Dimethylethyl)phenylsulfonyl)-5-trifluoromethyl-1H-pyrrolo[3,2-b]pyridin-2-ylmethyl]-methylbenzoat,
4-[[1-[[3-(1,1-Dimethylethyl)phenyl]sulfonyl]-5-(trifluoromethyl)-1H-pyrrolo[3,2-b]pyridin-2-yl]methyl]-benzoesäure,
4-{Hydroxy-[1-(3-(1-methylethyl)phenylsulfonyl)-5-trifluoromethyl-1H-pyrrolo[2,3-b]pyridin-2-yl]-methyl}-methylbenzoat,
4-[1-(3-(1-Methylethyl)phenylsulfonyl)-5-trifluoromethyl-1H-pyrrolo[2,3-b]pyridin-2-ylmethyl]-methylbenzoat,
4-[[1-[[3-(1-methylethyl)phenyl]sulfonyl]-5-(trifluoromethyl)-1 H-pyrrolo[2,3-b]pyridin-2-yl]methyl]-benzoesäure,
4-{Hydroxy-[1-(3-(1,1-dimethylethyl)phenylsulfonyl)-5-trifluoromethyl-1H-pyrrolo[2,3-b]pyridin-2-yl]-methyl}-methylbenzoat,
4-[1-(3-(1,1-Dimethylethyl)phenylsulfonyl)-5-trifluoromethyl-1H-pyrrolo[2,3-b]pyridin-2-ylmethyl]-methylbenzoat,
4-[[1-[[3-(1,1-Dimethylethyl)phenyl]sulfonyl]-5-(trifluoromethyl)-1H-pyrrolo[2,3-b]pyridin-2-yl]methyl]-benzoesäure,
4-{Hydroxy-[5-chloro-1-(4-(1-methylethyl)phenylsulfonyl)-1H-pyrrolo[2,3-b]pyridin-2-yl]-methyl}-methylbenzoat,
4-[5-Chloro-1-(4-(1-methylethyl)phenylsulfonyl)-1 H-pyrrolo[2,3-b]pyridin-2-ylmethyl]-methylbenzoat,
4-[[5-Chloro-1-[[4-(-methylethyl)phenyl]sulfonyl]-1H-pyrrolo-[2,3-b]pyridin-2-yl]methyl]-benzoesäure,
5-{Hydroxy-[1-(3-(1,1-dimethylethyl)phenylsulfonyl)-5-trifluoromethyl-1H-pyrrolo[3,2-b]pyridin-2-yl]-methyl}-thiophen-2-carbonsäure,
5-[[1-[[3-(1,1-Dimethylethyl)phenyl]sulfonyl]-5-(trifluoromethyl)-1H-pyrrolo[3,2-b]pyridin-2-yl]-fluoromethyl]-N,N-diethyl-2-thiophencarboxamid,
5-{1-(3-(1,1-Dimethylethyl)phenylsulfonyl)-5-trifluoromethyl-1H-pyrrolo[3,2-b]pyridin-2-carbonyl}-thiophen-2-carbonsäure,
4-[[5-Chloro-1-[[4-(-methylethyl)phenyl]sulfonyl]-1H-pyrrolo-[2,3-b]pyridin-2-yl]methyl]-benzoesäure, Natriumsalz,
4-[[5-Chloro-1-[[4-(-methylethyl)phenyl]sulfonyl]-1H-pyrrolo-[2,3-b]pyridin-2-yl]methyl]-benzoesäure, Piperazinsalz,
4-[[1-[[3-(1,1-Dimethylethyl)phenyl]sulfonyl]-3-methyl-5-(trifluoromethyl)-1H-pyrrolo[3,2-b]pyridin-2-yl]methyl]-benzoesäure,
5-[[1-[[4-(1,1-Methylethyl)phenyl]sulfonyl]-5-chloro-1H-pyrrolo[2,3-b]pyridin-2-yl]methyl]-thiophen-2-carbonsäure,
N-{4-[1-(3-tert-Butyl-benzolsulfonyl)-5-trifluoromethyl-1H-pyrrolo[3,2-b]pyridin-2-ylmethyl]-benzoyl}-methansulfonamid,
oder die pharmazeutisch akzeptablen Salze dieser Verbindungen.

8. Verbindung der Formel (I): 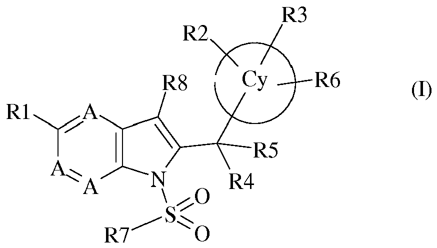 worin:
eine der A-Gruppen ein Stickstoffatom darstellt und die anderen A-Gruppen ein Kohlenstoffatom darstellen;
Cy ein Phenyl oder einen 5- oder 6-gliedrigen heteroaromatischen Kern darstellt;
R1 ein Wasserstoffatom, ein Halogenatom, eine (C₁-C₄)-Alkylgruppe, eventuell vollständig oder teilweise halogeniert, oder eine (C₁-C₄)-Alkoxygruppe darstellt;
R2 und R3 jeweils, unabhängig voneinander, ein Wasserstoffatom, ein Halogenatom, eine (C₁-C₄)-Alkylgruppe, eine Hydroxygruppe oder eine (C₁-C₄)-Alkoxygruppe darstellen;
R4 und R5 jeweils, unabhängig voneinander, ein Wasserstoffatom, ein Halogenatom, eine (C₁-C₄)-Alkylgruppe oder eine Hydroxygruppe darstellen;
oder R4 und R5 zusammen mit dem Kohlenstoffatom, an das sie gebunden sind, eine Ethylengruppe (C=CH2) oder eine Carbonylgruppe (C=O) bilden;
R6 eine -COOR9-Gruppe oder eine -SO₂NHR10-, -CONHNHCOOR11-, - CONR12R13- oder -CONHSO₂R14-Gruppe oder eine 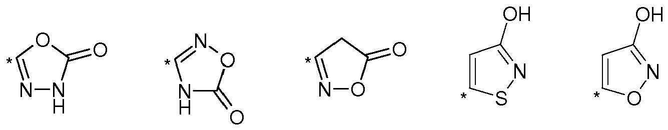 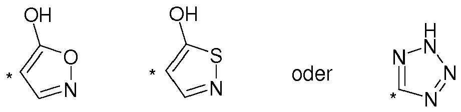
-Gruppe darstellt;
R7 ein Phenyl, eventuell substituiert durch eine (C₁-C₄)-Alkylgruppe, oder einen 6- bis 10-gliedrigen heteroaromatischen Kern, eventuell substituiert durch eine (C₁-C₄)-Alkylgruppe, darstellt;
R8 ein Wasserstoffatom, eine (C₁-C₄)-Alkylgruppe oder ein Halogenatom darstellt;
R9 ein Wasserstoffatom oder eine (C₁-C₄)-Alkylgruppe darstellt;
R10, R11, R12, R13 und R14 jeweils unabhängig voneinander ein Wasserstoffatom oder eine (C₁-C₄)-Alkylgruppe darstellen;
oder ein pharmazeutisch akzeptables Salz hiervon;
für ihre/seine Verwendung als therapeutisch aktive Substanz.

9. Verbindung der Formel (I) 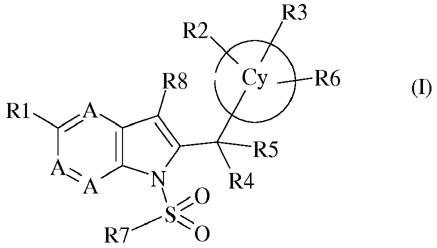 worin A, Cy, R1, R2, R3, R4, R5, R6, R7 und R8 so sind wie in Anspruch 8 definiert, oder ein pharmazeutisch akzeptables Salz hiervon, für ihre/seine Verwendung bei der Vorbeugung und/oder der Behandlung von neurodegenerativen Erkrankungen.

10. Verbindung für ihre Verwendung nach Anspruch 9, wobei die neurodegenerative Erkrankung die Parkinson-Krankheit ist.

11. Verbindung für ihre Verwendung nach Anspruch 9 oder Anspruch 10, die ausgewählt ist aus:
5-[[1-[[3-(1,1-Dimethylethyl)phenyl]sulfonyl]-5-(trifluoromethyl)-1H-pyrrolo[3,2-b]pyridin-2-yl]methyl]-thiophen-2-carbonsäure,
3-[[1-[[3-(1,1-Dimethylethyl)phenyl]sulfonyl]-5-(trifluoromethyl)-1H-pyrrolo[2,3-c]pyridin-2-yl]methyl]-2-benzoesäure,
2-Chloro-4-[[1-[[3-(1,1-dimethylethyl)phenyl]sulfonyl]-5-(trifluoromethyl)-1H-pyrrolo[2,3-c]pyridin-2-yl]methyl]-benzoesäure,
5-[[1-[[3-(1,1-Dimethylethyl)phenyl]sulfonyl]-5-(trifluoromethyl)-1H-pyrrolo[2,3-c]pyridin-2-yl]methyl]-furan-3-carbonsäure,
5-[[1-[[3-(1,1-Dimethylethyl)phenyl]sulfonyl]-5-(trifluoromethyl)-1H-pyrrolo[2,3-c]pyridin-2-yl]methyl]-pyridin-3-carbonsäure,
4-[[1-[[3-(1,1-Dimethylethyl)phenyl]sulfonyl]-5-(trifluoromethyl)-1H-pyrrolo[2,3-c]pyridin-2-yl]methyl]-thiophen-2-carbonsäure,
5-[[1-[[3-(1,1-Dimethylethyl)phenyl]sulfonyl]-5-(trifluoromethyl)-1 H-pyrrolo[2,3-c]pyridin-2-yl]methyl]-2-fluoro-benzoesäure,
2-[[1-[[3-(1,1-Dimethylethyl)phenyl]sulfonyl]-5-(trifluoromethyl)-1H-pyrrolo[3,2-b]pyridin-2-yl]methyl]-thiazol-4-carbonsäure,
4-[1-(4-(1-Methylethyl)phenylsulfonyl)-5-trifluoromethyl-1H-pyrrolo[2,3-b]pyridin-2-ylmethyl]-methylbenzoat,
4-[[1-[[3-(1-Methylethyl)phenyl]sulfonyl]-5-(trifluoromethyl)-1 H-pyrrolo[2,3-b]pyridin-2-yl]methyl]-benzoesäure,
4-[[1-[(3,4-Dihydro-4-methyl-2H-1,4-benzoxazin-6-yl)sulfonyl]-5-(trifluoromethyl)-1H-pyrrolo[2,3-c]pyridin-2-yl]methyl]-methylbenzoat,
4-[[1-[(3,4-Dihydro-4-methyl-2H-1,4-benzoxazin-6-yl)sulfonyl]-5-(trifluoromethyl)-1H-pyrrolo[2,3-c]pyridin-2-yl]methyl]-benzoesäure,
4-[1-(4-(1-Methylethyl)phenylsulfonyl)-5-trifluoromethyl-1H-pyrrolo[2,3-c]pyridin-2-ylmethyl]-methylbenzoat,
4-[[1-[[4-(1-Methylethyl)phenyl]sulfonyl]-5-(trifluoromethyl)-1 H-pyrrolo[2,3-c]pyridin-2-yl]methyl]-benzoesäure,
4-[1-(4-(1-Methylethyl)phenylsulfonyl)-5-trifluoromethyl-1H-pyrrolo[3,2-b]pyridin-2-ylmethyl]-methylbenzoat,
4-[1-(4-(1-Methylethyl)phenylsulfonyl)-5-trifluoromethyl-1H-pyrrolo[3,2-b]pyridin-2-ylmethyl]-benzoesäure,
4-[[1-[(3,4-Dihydro-4-methyl-2H-1,4-benzoxazin-6-yl)sulfonyl]-5-(trifluoromethyl)-1H-pyrrolo[2,3-b]pyridin-2-yl]methyl]-methylbenzoat,
4-[[1-[(3,4-Dihydro-4-methyl-2H-1,4-benzoxazin-6-yl)sulfonyl]-5-(trifluoromethyl)-1H-pyrrolo[2,3-b]pyridin-2-yl]methyl]-benzoesäure,
4-[1-(4-(1-Methylethyl)phenylsulfonyl)-5-chloro-1 H-pyrrolo[2,3-c]pyridin-2-ylmethyl]-methylbenzoat,
4-[[5-Chloro-1-[[4-(1-methylethyl)phenyl]sulfonyl]-1H-pyrrolo[2,3-c]pyridin-2-yl]methyl]-benzoesäure,
4-[1-(3-(1,1-Dimethylethyl)phenylsulfonyl)-5-trifluoromethyl-1H-pyrrolo[3,2-b]pyridin-2-ylmethyl]-methylbenzoat,
4-[[1-[[3-(1,1-Dimethylethyl)phenyl]sulfonyl]-5-(trifluoromethyl)-1H-pyrrolo[3,2-b]pyridin-2-yl]methyl]-benzoesäure,
4-{Hydroxy-[1-(3-(1-methylethyl)phenylsulfonyl)-5-trifluoromethyl-1H-pyrrolo[2,3-b]pyridin-2-yl]-methyl}-methylbenzoat,
4-[1-(3-(1-Methylethyl)phenylsulfonyl)-5-trifluoromethyl-1H-pyrrolo[2,3-b]pyridin-2-ylmethyl]-methylbenzoat,
4-[[1-[[3-(1-methylethyl)phenyl]sulfonyl]-5-(trifluoromethyl)-1 H-pyrrolo[2,3-b]pyridin-2-yl]methyl]-benzoesäure,
4-{Hydroxy-[1-(3-(1,1-dimethylethyl)phenylsulfonyl)-5-trifluoromethyl-1H-pyrrolo[2,3-b]pyridin-2-yl]-methyl}-methylbenzoat,
4-[1-(3-(1,1-Dimethylethyl)phenylsulfonyl)-5-trifluoromethyl-1H-pyrrolo[2,3-b]pyridin-2-ylmethyl]-methylbenzoat,
4-[[1-[[3-(1,1-Dimethylethyl)phenyl]sulfonyl]-5-(trifluoromethyl)-1H-pyrrolo[2,3-b]pyridin-2-yl]methyl]-benzoesäure,
4-{Hydroxy-[5-chloro-1-(4-(1-methylethyl)phenylsulfonyl)-1H-pyrrolo[2,3-b]pyridin-2-yl]-methyl}-methylbenzoat,
4-[5-Chloro-1-(4-(1-methylethyl)phenylsulfonyl)-1 H-pyrrolo[2,3-b]pyridin-2-ylmethyl]-methylbenzoat,
4-[[5-Chloro-1-[[4-(-methylethyl)phenyl]sulfonyl]-1H-pyrrolo-[2,3-b]pyridin-2-yl]methyl]-benzoesäure,
5-{Hydroxy-[1-(3-(1,1-dimethylethyl)phenylsulfonyl)-5-trifluoromethyl-1H-pyrrolo[3,2-b]pyridin-2-yl]-methyl}-thiophen-2-carbonsäure,
5-[[1-[[3-(1,1-Dimethylethyl)phenyl]sulfonyl]-5-(trifluoromethyl)-1H-pyrrolo[3,2-b]pyridin-2-yl]-fluoromethyl]-N,N-diethyl-2-thiophencarboxamid,
5-{1-(3-(1,1-Dimethylethyl)phenylsulfonyl)-5-trifluoromethyl-1H-pyrrolo[3,2-b]pyridin-2-carbonyl}-thiophen-2-carbonsäure,
4-[[5-Chloro-1-[[4-(1-methylethyl)phenyl]sulfonyl]-1H-pyrrolo-[2,3-b]pyridin-2-yl]methyl]-benzoesäure, Natriumsalz,
4-[[5-Chloro-1-[[4-(1-methylethyl)phenyl]sulfonyl]-1H-pyrrolo-[2,3-b]pyridin-2-yl]methyl]-benzoesäure, Piperazinsalz,
4-[[1-[[3-(1,1-Dimethylethyl)phenyl]sulfonyl]-3-methyl-5-(trifluoromethyl)-1H-pyrrolo[3,2-b]pyridin-2-yl]methyl]-benzoesäure,
5-[[1-[[4-(1,1-Methylethyl)phenyl]sulfonyl]-5-chloro-1H-pyrrolo[2,3-b]pyridin-2-yl]methyl]-thiophen-2-carbonsäure,
N-{4-[1-(3-tert-Butyl-benzolsulfonyl)-5-trifluoromethyl-1H-pyrrolo[3,2-b]pyridin-2-ylmethyl]-benzoyl}-methansulfonamid,
oder die pharmazeutisch akzeptablen Salze dieser Verbindungen.

12. Pharmazeutische Zusammensetzung, die als Wirkstoff eine Verbindung der Formel (I) umfasst: 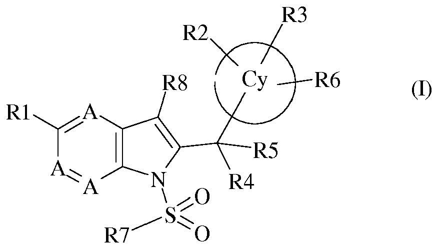 worin A, Cy, R1, R2, R3, R4, R5, R6, R7 und R8 so sind wie in Anspruch 8 definiert, oder ein pharmazeutisch akzeptables Salz hiervon;
und wenigstens einen pharmazeutisch akzeptablen Hilfsstoff.

## Claims

1. A compound of formula (I): 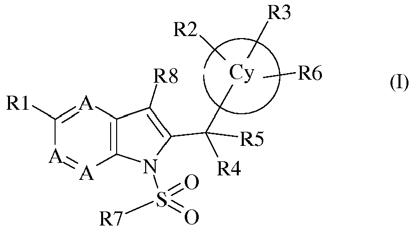 in which:
one of the groups A represents a nitrogen atom and the other groups A represent a carbon atom;
Cy represents a phenyl or a 5- or 6-membered heteroaromatic ring;
R1 represents a hydrogen atom, a halogen atom, a group (C₁-C₄)alkyl that is optionally totally or partially halogenated, or a group (C₁-C₄)alkoxy;
R2 and R3 represent, independently of each other, a hydrogen atom, a halogen atom, a group (C₁-C₄)alkyl, a hydroxyl group or a group (C₁-C₄)alkoxy;
R4 and R5 represent, independently of each other, a hydrogen atom, a halogen atom, a group (C₁-C₄)alkyl or a hydroxyl group;
or R4 and R5 form, together with the carbon atoms to which they are attached, an ethylene group (C=CH2) or a carbonyl group (C=O);
R6 represents a group -COOR9 or a group -SO₂NHR10, -CONHNH-COOR11, -CONR12R13 or -CONHSO₂R14, or a group 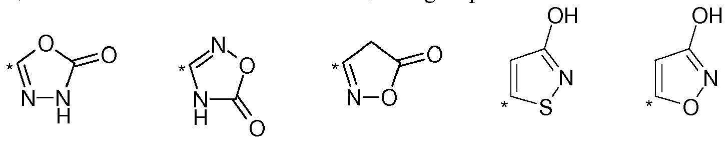 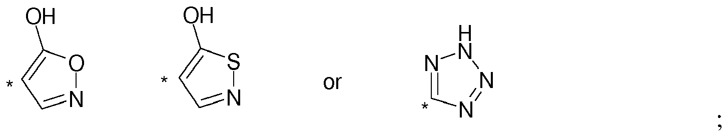
R7 represents a phenyl optionally substituted with a group (C₁-C₄)alkyl, or a 6-to 10-membered heteroaromatic ring optionally substituted with a group (C₁-C₄)alkyl;
R8 represents a hydrogen atom, a group (C₁-C₄)alkyl or a halogen atom;
R9 represents a hydrogen atom or a group (C₁-C₄)alkyl;
R10, R11, R12, R13 and R14 represent, independently of each other, a hydrogen atom or a group (C₁-C₄)alkyl;
or a pharmaceutically acceptable salt of the said compound of formula (I);
with the exception of the following compounds:
2-((1-phenylsulfonyl-1H-pyrrolo[2,3-b]pyridin-2-yl-carbonyl)benzoic acid;
N,N-diethyl-4-[hydroxy[1-(phenylsulfonyl)-1H-pyrrolo[2,3-b]pyridin-2-yl]methyl]-2-methoxy-3-pyridinecarboxamide;
N,N-diethyl-2-methoxy-4-[[1-(phenylsulfonyl)-1H-pyrrolo[2,3-b]pyridin-2-yl]carbonyl]-3-pyridinecarboxamide;
N,N-diethyl-4-[1-hydroxy-1-[1-(phenylsulfonyl)-1H-pyrrolo[2,3-b]pyridin-2-yl]ethyl]-2-methoxy-3-pyridinecarboxamide.

2. A compound according to claim 1, in which R8 represents a hydrogen atom;
or a pharmaceutically acceptable salt of the said compound.

3. A compound according to claim 1, in which:
Cy represents a phenyl or a 5- or 6-membered heteroaromatic ring;
R1 represents a halogen atom or a group (C₁-C₄)alkyl that is optionally totally or partially halogenated;
R2 and R3 represent, independently of each other, a hydrogen atom or a halogen atom;
R4 and R5 represent, independently of each other, a hydrogen atom, a halogen atom or a hydroxyl group;
or R4 and R5 form, together with the carbon atom to which they are attached, a carbonyl group (C=O);
R6 represents a group -COOR9;
R7 represents a phenyl optionally substituted with a group (C₁-C₄)alkyl;
R8 represents a hydrogen atom;
R9 represents a hydrogen atom or a group (C₁-C₄)alkyl;
or a pharmaceutically acceptable salt of the said compound.

4. A compound according to one of claims 1 to 3, in which Cy represents a phenyl, thienyl, thiazolyl, furyl or pyridyl;
or a pharmaceutically acceptable salt of the said compound.

5. A compound according to one of claims 1 to 4, in which R2 and R3 each represent a hydrogen atom;
or a pharmaceutically acceptable salt of the said compound.

6. A compound according to one of claims 1 to 5, in which R4 and R5 each represent a hydrogen atom;
or a pharmaceutically acceptable salt of the said compound.

7. A compound according to claim 1, chosen from:
5-[[1-[[3-(1,1-dimethylethyl)phenyl]sulfonyl]-5-(trifluoromethyl)-1H-pyrrolo[3,2-b]pyridin-2-yl]methyl]-thiophene-2-carboxylic acid,
3-[[1-[[3-(1,1-dimethylethyl)phenyl]sulfonyl]-5-(trifluoromethyl)-1H-pyrrolo[2,3-c]pyridin-2-yl]methyl]-2-benzoic acid,
2-chloro-4-[[1-[[3-(1,1-dimethylethyl)phenyl]sulfonyl]-5-(trifluoromethyl)-1H-pyrrolo[2,3-c]pyridin-2-yl]methyl]-benzoic acid,
5-[[1-[[3-(1,1-dimethylethyl)phenyl]sulfonyl]-5-(trifluoromethyl)-1H-pyrrolo[2,3-c]pyridin-2-yl]methyl]-furane-3-carboxylic acid,
5-[[1-[[3-(1,1-dimethylethyl)phenyl]sulfonyl]-5-(trifluoromethyl)-1H-pyrrolo[2,3-c]pyridin-2-yl]methyl]-pyridine-3-carboxylic acid,
4-[[1-[[3-(1,1-dimethylethyl)phenyl]sulfonyl]-5-(trifluoromethyl)-1H-pyrrolo[2,3-c]pyridin-2-yl]methyl]-thiophene-2-carboxylic acid,
5-[[1-[[3-(1,1-dimethylethyl)phenyl]sulfonyl]-5-(trifluoromethyl)-1H-pyrrolo[2,3-c]pyridin-2-yl]methyl]-2-fluoro-benzoic acid,
2-[[1-[[3-(1,1-dimethylethyl)phenyl]sulfonyl]-5-(trifluoromethyl)-1H-pyrrolo[3,2-b]pyridin-2-yl]methyl]-thiazole-4-carboxylic acid,
Methyl 4-[1-(4-(1-methylethyl)phenylsulfonyl)-5-trifluoromethyl-1H-pyrrolo[2,3-b]pyridin-2-ylmethyl]-benzoate,
4-[[1-[[3-(1-methylethyl)phenyl]sulfonyl]-5-(trifluoromethyl)-1H-pyrrolo[2,3-b]pyridin-2-yl]methyl]-benzoic acid,
Methyl 4-[[1-[(3,4-dihydro-4-methyl-2H-1,4-benzoxazin-6-yl)sulfonyl]-5-(trifluoromethyl)-1H-pyrrolo[2,3-c]pyridin-2-yl]methyl]-benzoate,
4-[[1-[(3,4-dihydro-4-methyl-2H-1,4-benzoxazin-6-yl)sulfonyl]-5-(trifluoromethyl)-1H-pyrrolo[2,3-c]pyridin-2-yl]methyl]-benzoic acid,
Methyl 4-[1-(4-(1-methylethyl)phenylsulfonyl)-5-trifluoromethyl-1H-pyrrolo[2,3-c]pyridin-2-ylmethyl]-benzoate,
4-[[1-[[4-(1-methylethyl)phenyl]sulfonyl]-5-(trifluoromethyl)-1H-pyrrolo[2,3-c]pyridin-2-yl]methyl]-benzoic acid,
Methyl 4-[1-(4-(1-methylethyl)phenylsulfonyl)-5-trifluoromethyl-1H-pyrrolo[3,2-b]pyridin-2-ylmethyl]-benzoate,
4-[1-(4-(1-methylethyl)phenylsulfonyl)-5-trifluoromethyl-1H-pyrrolo[3,2-b]pyridin-2-ylmethyl]-benzoic acid,
Methyl 4-[[1-[(3,4-dihydro-4-methyl-2H-1,4-benzoxazin-6-yl)sulfonyl]-5-(trifluoromethyl)-1H-pyrrolo[2,3-b]pyridin-2-yl]methyl]-benzoate,
4-[[1-[(3,4-dihydro-4-methyl-2H-1,4-benzoxazin-6-yl)sulfonyl]-5-(trifluoromethyl)-1H-pyrrolo[2,3-b]pyridin-2-yl]methyl]-benzoic acid,
Methyl 4-[1-(4-(1-methylethyl)phenylsulfonyl)-5-chloro-1H-pyrrolo[2,3-c]pyridin-2-ylmethyl]-benzoate,
4-[[5-chloro-1-[[4-(1-methylethyl)phenyl]sulfonyl]-1H-pyrrolo[2,3-c]pyridin-2-yl]methyl]-benzoic acid,
Methyl 4-[1-(3-(1,1-dimethylethyl)phenylsulfonyl)-5-trifluoromethyl-1H-pyrrolo[3,2-b]pyridin-2-ylmethyl]-benzoate,
4-[[1-[[3-(1,1-dimethylethyl)phenyl]sulfonyl]-5-(trifluoromethyl)-1H-pyrrolo[3,2-b]pyridin-2-yl]methyl]-benzoic acid,
Methyl 4-{Hydroxy-[1-(3-(1-methylethyl)phenylsulfonyl)-5-trifluoromethyl-1H-pyrrolo[2,3-b]pyridin-2-yl]-methyl}-benzoate,
Methyl 4-[1-(3-(1-methylethyl)phenylsulfonyl)-5-trifluoromethyl-1H-pyrrolo[2,3-b]pyridin-2-ylmethyl] -benzoate,
4-[[1-[[3-(1-methylethyl)phenyl]sulfonyl]-5-(trifluoromethyl)-1H-pyrrolo[2,3-b]pyridin-2-yl]methyl]-benzoic acid,
Methyl 4-{Hydroxy-[1-(3-(1,1-dimethylethyl)phenylsulfonyl)-5-trifluoromethyl-1H-pyrrolo[2,3-b]pyridin-2-yl]-methyl}-benzoate,
Methyl 4-[1-(3-(1,1-dimethylethyl)phenylsulfonyl)-5-trifluoromethyl-1H-pyrrolo[2,3-b]pyridin-2-ylmethyl]-benzoate,
4-[[1-[[3-(1,1-dimethylethyl)phenyl]sulfonyl]-5-(trifluoromethyl)-1H-pyrrolo[2,3-b]pyridin-2-yl]methyl]-benzoic acid,
Methyl 4-{Hydroxy-[5-chloro-1-(4-(1-methylethyl)phenylsulfonyl)-1H-pyrrolo[2,3-b]pyridin-2-yl]-methyl}-benzoate,
Methyl 4-[5-Chloro-1-(4-(1-methylethyl)phenylsulfonyl)-1H-pyrrolo[2,3-b]pyridin-2-ylmethyl] -benzoate,
4-[[5-chloro-1-[[4-(1-methylethyl)phenyl]sulfonyl]- 1H-pyrrolo-[2,3-b]pyridin-2-yl]methyl]-benzoic acid,
5-{hydroxy[1-(3-(1,1-dimethylethyl)phenylsulfonyl)-5-trifluoromethyl-1H-pyrrolo[3,2-b]pyridin-2-yl]methyl}thiophene-2-carboxylic acid,
5-[[1-[[3-(1,1-dimethylethyl)phenyl]sulfonyl]-5-(trifluoromethyl)-1H-pyrrolo[3,2-b]pyridin-2-yl]fluoromethyl]-N,N-diethyl-2-thiophenecarboxamide,
5-{1-(3-(1,1-dimethylethyl)phenylsulfonyl)-5-trifluoromethyl-1H-pyrrolo[3,2-b]pyridine-2-carbonyl}thiophene-2-carboxylic acid,
4-[[5-chloro-1-[[4-(1-methylethyl)phenyl]sulfonyl]-1H-pyrrolo[2,3-b]pyridin-2-yl]methyl]benzoic acid, sodium salt,
4-[[5-chloro-1-[[4-(1-methylethyl)phenyl]sulfonyl]-1H-pyrrolo[2,3-b]pyridin-2-yl]methyl]benzoic acid, piperazine salt,
4-[[1-[[3-(1,1-dimethylethyl)phenyl]sulfonyl]-3-methyl-5-(trifluoromethyl)-1H-pyrrolo[3,2-b]pyridin-2-yl]methyl]benzoic acid,
5-[[1-[[4-(1,1-methylethyl)phenyl]sulfonyl]-5-chloro-1H-pyrrolo[2,3-b]pyridin-2-yl]methyl]thiophene-2-carboxylic acid,
N-{4-[1-(3-tert-butylbenzenesulfonyl)-5-trifluoromethyl-1H-pyrrolo[3,2-b]pyridin-2-ylmethyl]benzoyl}methanesulfonamide;
or pharmaceutically acceptable salts of these compounds.

8. A compound of formula (I): 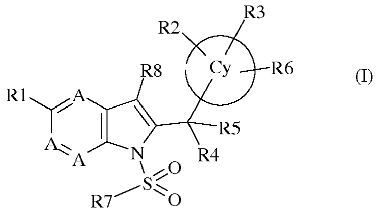 in which:
one of the groups A represents a nitrogen atom and the other groups A represent a carbon atom;
Cy represents a phenyl or a 5- or 6-membered heteroaromatic ring;
R1 represents a hydrogen atom, a halogen atom, a group (C₁-C₄)alkyl that is optionally totally or partially halogenated, or a group (C₁-C₄)alkoxy;
R2 and R3 represent, independently of each other, a hydrogen atom, a halogen atom, a group (C₁-C₄)alkyl, a hydroxyl group or a group (C₁-C₄)alkoxy;
R4 and R5 represent, independently of each other, a hydrogen atom, a halogen atom, a group (C₁-C₄)alkyl or a hydroxyl group;
or R4 and R5 form, together with the carbon atoms to which they are attached, an ethylene group (C=CH2) or a carbonyl group (C=O);
R6 represents a group -COOR9 or a group -SO₂NHR10, -CONHNH-COOR11, -CONR12R13 or -CONHSO₂R14, or a group 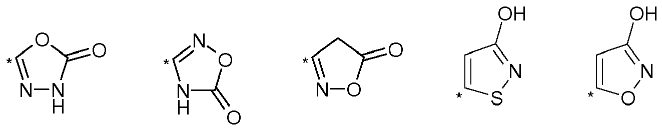 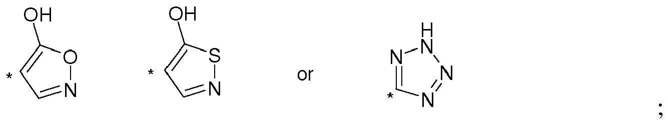
R7 represents a phenyl optionally substituted with a group (C₁-C₄)alkyl, or a 6-to 10-membered heteroaromatic ring optionally substituted with a group (C₁-C₄)alkyl;
R8 represents a hydrogen atom, a group (C₁-C₄)alkyl or a halogen atom;
R9 represents a hydrogen atom or a group (C₁-C₄)alkyl;
R10, R11, R12, R13 and R14 represent, independently of each other, a hydrogen atom or a group (C₁-C₄)alkyl;
or a pharmaceutically acceptable salt thereof;
for its use as a therapeutically active substance.

9. A compound of formula (I) 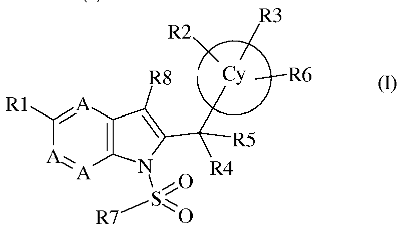 in which A, Cy, R1, R2, R3, R4, R5, R6, R7 and R8 are as defined in claim 8, or a pharmaceutically acceptable salt thereof, for its use in the prevention and/or treatment of neurodegenerative diseases.

10. A compound for the use according to claim 9, where the neurodegenerative disease is Parkinson's disease.

11. A compound for the use according to claim 9 or claim 10, which is chosen from:
5-[[1-[[3-(1,1-dimethylethyl)phenyl]sulfonyl]-5-(trifluoromethyl)-1H-pyrrolo[3,2-b]pyridin-2-yl]methyl]-thiophene-2-carboxylic acid,
3-[[1-[[3-(1,1-dimethylethyl)phenyl]sulfonyl]-5-(trifluoromethyl)-1H-pyrrolo[2,3-c]pyridin-2-yl]methyl]-2-benzoic acid,
2-chloro-4-[[1-[[3-(1,1-dimethylethyl)phenyl]sulfonyl]-5-(trifluoromethyl)-1H-pyrrolo[2,3-c]pyridin-2-yl]methyl]-benzoic acid,
5-[[1-[[3-(1,1-dimethylethyl)phenyl]sulfonyl]-5-(trifluoromethyl)-1H-pyrrolo[2,3-c]pyridin-2-yl]methyl]-furane-3-carboxylic acid,
5-[[1-[[3-(1,1-dimethylethyl)phenyl]sulfonyl]-5-(trifluoromethyl)-1H-pyrrolo[2,3-c]pyridin-2-yl]methyl]-pyridine-3-carboxylic acid,
4-[[1-[[3-(1,1-dimethylethyl)phenyl]sulfonyl]-5-(trifluoromethyl)-1H-pyrrolo[2,3-c]pyridin-2-yl]methyl]-thiophene-2-carboxylic acid,
5-[[1-[[3-(1,1-dimethylethyl)phenyl]sulfonyl]-5-(trifluoromethyl)-1H-pyrrolo[2,3-c]pyridin-2-yl]methyl]-2-fluoro-benzoic acid,
2-[[1-[[3-(1,1-dimethylethyl)phenyl]sulfonyl]-5-(trifluoromethyl)-1H-pyrrolo[3,2-b]pyridin-2-yl]methyl]-thiazole-4-carboxylic acid,
Methyl 4-[1-(4-(1-methylethyl)phenylsulfonyl)-5-trifluoromethyl-1H-pyrrolo[2,3-b]pyridin-2-ylmethyl]-benzoate,
4-[[1-[[3-(1-methylethyl)phenyl]sulfonyl]-5-(trifluoromethyl)-1H-pyrrolo[2,3-b]pyridin-2-yl]methyl]-benzoic acid,
Methyl 4-[[1-[(3,4-dihydro-4-methyl-2H-1,4-benzoxazin-6-yl)sulfonyl]-5-(trifluoromethyl)-1H-pyrrolo[2,3-c]pyridin-2-yl]methyl]-benzoate,
4-[[1-[(3,4-dihydro-4-methyl-2H-1,4-benzoxazin-6-yl)sulfonyl]-5-(trifluoromethyl)-1H-pyrrolo[2,3-c]pyridin-2-yl]methyl]-benzoic acid,
Methyl 4-[1-(4-(1-methylethyl)phenylsulfonyl)-5-trifluoromethyl-1H-pyrrolo[2,3-c]pyridin-2-ylmethyl]-benzoate,
4-[[1-[[4-(1-methylethyl)phenyl]sulfonyl]-5-(trifluoromethyl)-1H-pyrrolo[2,3-c]pyridin-2-yl]methyl]-benzoic acid,
Methyl 4-[1-(4-(1-methylethyl)phenylsulfonyl)-5-trifluoromethyl-1H-pyrrolo[3,2-b]pyridin-2-ylmethyl] -benzoate,
4-[1-(4-(1-methylethyl)phenylsulfonyl)-5-trifluoromethyl-1H-pyrrolo[3,2-b]pyridin-2-ylmethyl]-benzoic acid,
Methyl 4-[[1-[(3,4-dihydro-4-methyl-2H-1,4-benzoxazin-6-yl)sulfonyl]-5-(trifluoromethyl)-1H-pyrrolo[2,3-b]pyridin-2-yl]methyl]-benzoate,
4-[[1-[(3,4-dihydro-4-methyl-2H-1,4-benzoxazin-6-yl)sulfonyl]-5-(trifluoromethyl)-1H-pyrrolo[2,3-b]pyridin-2-yl]methyl]-benzoic acid,
Methyl 4-[1-(4-(1-methylethyl)phenylsulfonyl)-5-chloro-1H-pyrrolo[2,3-c]pyridin-2-ylmethyl]-benzoate,
4-[[5-chloro-1-[[4-(1-methylethyl)phenyl]sulfonyl]-1H-pyrrolo[2,3-c]pyridin-2-yl]methyl]-benzoic acid,
Methyl 4- [1-(3-(1,1-dimethylethyl)phenylsulfonyl)-5-trifluoromethyl-1H-pyrrolo[3,2-b]pyridin-2-ylmethyl]-benzoate,
4-[[1-[[3-(1,1-dimethylethyl)phenyl]sulfonyl]-5-(trifluoromethyl)-1H-pyrrolo[3,2-b]pyridin-2-yl]methyl]-benzoic acid,
Methyl 4-{Hydroxy-[1-(3-(1-methylethyl)phenylsulfonyl)-5-trifluoromethyl-1H-pyrrolo[2,3-b]pyridin-2-yl]-methyl}-benzoate,
Methyl 4-[1-(3-(1-methylethyl)phenylsulfonyl)-5-trifluoromethyl-1H-pyrrolo[2,3-b]pyridin-2-ylmethyl] -benzoate,
4-[[1-[[3-1-methylethyl)phenyl]sulfonyl]-5-(trifluoromethyl)-1H-pyrrolo[2,3-b]pyridin-2-yl]methyl]-benzoic acid,
Methyl 4-{Hydroxy-[1-(3-(1,1-dimethylethyl)phenylsulfonyl)-5-trifluoromethyl-1H-pyrrolo[2,3-b]pyridin-2-yl]-methyl}-benzoate,
Methyl 4-[1-(3-(1,1-dimethylethyl)phenylsulfonyl)-5-trifluoromethyl-1H-pyrrolo[2,3-b]pyridin-2-ylmethyl]-benzoate,
4-[[1-[[3-(1,1-dimethylethyl)phenyl]sulfonyl]-5-(trifluoromethyl)-1H-pyrrolo[2,3-b]pyridin-2-yl]methyl]-benzoic acid,
Methyl 4-{Hydroxy-[5-chloro-1-(4-(1-methylethyl)phenylsulfonyl)-1H-pyrrolo[2,3-b]pyridin-2-yl]-methyl}-benzoate,
Methyl 4-[5-Chloro-1-(4-(1-methylethyl)phenylsulfonyl)-1H-pyrrolo[2,3-b]pyridin-2-ylmethyl] -benzoate,
4-[[5-chloro-1-[[4-(1-methylethyl)phenyl]sulfonyl]- 1H-pyrrolo-[2,3-b]pyridin-2-yl]methyl]-benzoic acid,
5-{hydroxy[1-(3-(1,1-dimethylethyl)phenylsulfonyl)-5-trifluoromethyl-1H-pyrrolo[3,2-b]pyridin-2-yl]methyl}thiophene-2-carboxylic acid,
5-[[1-[[3-(1,1-dimethylethyl)phenyl]sulfonyl]-5-(trifluoromethyl)-1H-pyrrolo[3,2-b]pyridin-2-yl]fluoromethyl]-N,N-diethyl-2-thiophenecarboxamide,
5-{1-(3-(1,1-dimethylethyl)phenylsulfonyl)-5-trifluoromethyl-1H-pyrrolo[3,2-b]pyridine-2-carbonyl}thiophene-2-carboxylic acid,
4-[[5-chloro-1-[[[4-(1-methylethyl)phenyl]sulfonyl]-1H-pyrrolo[2,3-b]pyridin-2-yl]methyl]benzoic acid, sodium salt,
4-[[5-chloro-1-[[4-(1-methylethyl)phenyl]sulfonyl]-1H-pyrrolo[2,3-b]pyridin-2-yl]methyl]benzoic acid, piperazine salt,
4-[[1-[[3-(1,1-dimethylethyl)phenyl]sulfonyl]-3-methyl-5-(trifluoromethyl)-1H-pyrrolo[3,2-b]pyridin-2-yl]methyl]benzoic acid,
5-[[1-[[4-(1,1-methylethyl)phenyl]sulfonyl]-5-chloro-1H-pyrrolo[2,3-b]pyridin-2-yl]methyl]thiophene-2-carboxylic acid,
N-{4-[1-(3-tert-butylbenzenesulfonyl)-5-trifluoromethyl-1H-pyrrolo[3,2-b]pyridin-2-ylmethyl]benzoyl}methanesulfonamide;
or pharmaceutically acceptable salts of these compounds.

12. A pharmaceutical composition comprising, as active substance, a compound of formula (I): 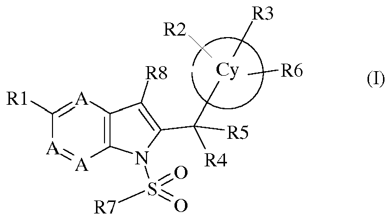 in which A, Cy, R1, R2, R3, R4, R5, R6, R7 and R8 are as defined in claim 8, or a pharmaceutically acceptable salt thereof;
and at least one pharmaceutically acceptable excipient.
